(19) Europäisches Patentamt
European Patent Office
Office européen des brevets

(11) Numéro de publication : **0 235 111 B1**

(12) # FASCICULE DE BREVET EUROPEEN

(45) Date de publication du fascicule du brevet : **10.04.91 Bulletin 91/15**

(21) Numéro de dépôt : **87870019.4**

(22) Date de dépôt : **10.02.87**

(51) Int. Cl.$^5$ : **C07D 471/04,** A61K 31/44, // (C07D471/04, 221:00, 209:00)

(54) **Dérivés d'indolizine, leur procédé de préparation ainsi que les compositions les contenant.**

Le dossier contient des informations techniques présentées postérieurement au dépôt de la demande et ne figurant pas dans le présent fascicule.

(30) Priorité : **14.02.86 FR 8602045**

(43) Date de publication de la demande : **02.09.87 Bulletin 87/36**

(45) Mention de la délivrance du brevet : **10.04.91 Bulletin 91/15**

(84) Etats contractants désignés : **AT CH DE ES FR GB GR IT LI LU NL SE**

(56) Documents cités :
**FR-A- 2 341 578**
**US-A- 3 947 470**
**US-A- 4 117 128**
**Burger 3rd edition**

(73) Titulaire : **SANOFI S.A.**
**40, Avenue George V**
**F-75008 Paris (FR)**

(72) Inventeur : **Gubin, Jean**
**Avenue de Mutsaard 70 Bte 36**
**B-1020 Bruxelles (BE)**
Inventeur : **Descamps, Marcel**
**Place des Carmes 8/301**
**B-1300 Wavre (BE)**
Inventeur : **Chatelain, Pierre**
**Avenue du Haras 111**
**B-1150 Bruxelles (BE)**
Inventeur : **Nisato, Dino**
**Impasse de l'Olivette 4**
**F-34680 Saint Georges d'orques (FR)**

(74) Mandataire : **Moncheny, Michel et al**
**c/o Cabinet Lavoix 2 Place d'Estienne d'Orves**
**F-75441 Paris Cedex 09 (FR)**

EP 0 235 111 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention se rapporte, d'une manière générale, à de nouveaux dérivés hétérocycliques et plus particulièrement à de nouveaux dérivés d'indolizine ainsi qu'à leur procédé de préparation.

Les dérivés d'indolizine de l'invention peuvent être représentés par la formule générale :

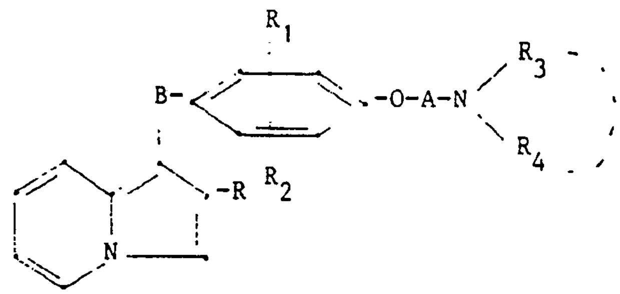

I

dans laquelle

B représente un groupement –S–, ou $-SO_2-$

R représente l'hydrogène, un radical alkyle ayant de 1 à 8 atomes de carbone, linéaire ou ramifié, un radical cycloalkyle comportant au maximum 6 atomes de carbone ou un groupement phényl non substitué ou substitué par un ou plusieurs substituants, identiques ou différents, sélectionnés parmi des atomes d'halogène, par exemple fluor, chlore, brome et parmi des groupements alkyles inférieurs ayant de 1 à 4 atomes de carbone, alcoxy inférieurs ayant de 1 à 4 atomes de carbone ou nitro,

$R_1$ et $R_2$, qui sont identiques ou différents, représentent chacun l'hydrogène, le radical méthyle ou éthyle ou un halogène, tel que le chlore, le brome ou l'iode,

A représente un radical alkylène, linéaire ou ramifié, ayant de 2 à 5 atomes de carbone ou le radical hydroxy-2 propylène,

$R_3$ représente un radical alkyle ayant de 1 à 8 atomes de carbone, linéaire ou ramifié, ou un radical de formule :

$$-Alk-R_5$$

dans laquelle Alk représente une liaison simple ou un radical alkylène, linéaire ou ramifié, ayant de 1 à 5 atomes de carbone et $R_5$ représente un radical pyridyle, phényle, méthylènedioxy-2,3 phényle, méthylènedioxy-3,4 phényle ou un groupement phényle substitué par un ou plusieurs substituants identiques ou différents sélectionnés parmi des atomes d'halogène, des groupements alkyles inférieurs ayant de 1 à 4 atomes de carbone ou des groupements alcoxy inférieurs, ayant de 1 à 4 atomes de carbone.

$R_4$ représente l'hydrogène ou un radical alkyle, ayant de 1 à 8 atomes de carbone

ou $R_3$ et $R_4$ lorsqu'ils sont pris ensemble, représentent un radical tétraméthylène-1,4 ; pentaméthylène-1,5; oxo-3 pentaméthylène-1,5 ; aza-3 pentaméthylène-1,5 ; méthylaza-3 pentaméthylène-1,5 ; phényl-aza-3 pentaméthylène-1,5 ou –CH = CH-N = CH– de sorte que $R_3$ et $R_4$ pris avec l'atome d'azote auquel ils sont attachés, peuvent représenter notamment un radical pyrrolidinyle, pipéridinyle, morpholinyle, pipérazinyle, méthyl-4 pipérazinyle, phényl-4 pipérazinyle ou 1H-imidazolyle.

Dans le présent contexte, aussi bien dans la description que dans les revendications, les termes repris ci-dessus comportent la signification suivante :

"alkyle" désigne les restes d'hydrocarbures aliphatiques saturés ayant jusqu'à 8 atomes de carbone tels que méthyle, éthyle, n-propyle, isopropyle, n-butyle, isobutyle, tertiobutyle, n-pentyle, néopentyle, n-hexyle, n-heptyle ou n-octyle,

"alkyle inférieur" désigne les restes d'hydrocarbures aliphatiques saturés ayant jusqu'à 4 atomes de carbone tels que méthyle, éthyle, n-propyle, isopropyle, n-butyle, isobutyle, tertiobutyle ou méthyl-1 propyle,

"alcoxy inférieur" désigne un groupement hydroxy substitué par un groupement alkyle inférieur tel que défini précédemment.

Ainsi, en tenant compte des valeurs données ci-dessus :

R peut représenter notamment un radical méthyle, éthyle, n-propyle, isopropyle, n-butyle, isobutyle, tertiobutyle, méthyl-1 propyle, n-pentyle, néopentyle, phényle, mono-fluoro-, mono-chloro- ou mono-bromo-phényle, di-fluoro-, di-chloro- ou di-bromo-phényle, mono-méthyl- ou di-méthyl-phényle, mono-méthoxy- ou di-méthoxy-phényle ou un radical méthylphényle substitué par un atome d'halogène,

A peut représenter notamment une chaîne éthylène-1,2 ; propylène-1,3 ; méthyl-2 propylène-1,3 ; tétraméthylène-1,4 ou pentaméthylène-1,5.

$R_3$ peut représenter notamment un radical méthyle, éthyle, n-propyle, isopropyle, n-butyle, isobutyle, tertiobutyle, méthyl-1 propyle, n-pentyle, n-hexyle, n-heptyle, n-octyle, phényle, benzyle, phénéthyle, méthoxyphényle, diméthoxyphénéthyle par exemple diméthoxy-3,4 phénéthyle, diméthylphénéthyle, diméthoxybenzyle, pyridyléthyle ou un radical phénéthyle substitué dans la partie aromatique par des radicaux méthyle et méthoxy,

$R_4$ peut représenter notamment un radical méthyle, éthyle, n-propyle, isopropyle, n-butyle, isobutyle, tertiobutyle, n-pentyle, néopentyle, n-hexyle, n-heptyle ou n-octyle,

$R_3$ et $R_4$ pris ensemble sont tels que définis précédemment.

L'invention se rapporte également aux sels pharmaceutiquement acceptables des composés de formule I formés à partir d'un acide organique ou inorganique.

Comme exemples de sels organiques de ce genre, on peut citer les oxalate, maléate, fumarate, méthanesulfonate, benzoate, ascorbate, pamoate, succinate, hexamate, bisméthylènesalicylate, éthanedisulfonate, acétate, propionate, tartrate, salicylate, citrate, gluconate, lactate, malate, cinnamate, mandélate, citraconate, aspartate, palmitate, stéarate, itaconate, glycolate, p-aminobenzoate, glutamate, benzènesulfonate et théophylline acétate ainsi que les sels formés à partir d'un acide aminé tel que le sel de lysine ou d'histidine.

Comme exemples de sels inorganiques de ce genre, on peut citer les chlorhydrate, bromhydrate, sulfate, sulfamate, phosphate et nitrate.

Les composés de formule I peuvent exister dans certains cas sous la forme d'isomères optiques notamment en raison du carbone asymétrique présent lorsque A représente une chaîne hydroxy-2 propylène.

L'invention se rapporte à la fois à l'ensemble des isomères des composés de formule I, isomères considérés sous forme dextrogyre ou lévogyre ou, sous forme de mélange, par exemple sous forme de mélange racémique.

On a trouvé que les dérivés d'indolizine de l'invention possèdent de remarquables propriétés pharmacologiques notamment des propriétés inhibitrices de la translocation calcique ainsi que des propriétés bradycardisantes, hypotensives et antiadrénergiques.

De ce point de vue, les composés préférés de l'invention sont ceux dans lesquels B représente un groupement $-SO_2-$.

Ces propriétés sont capables de rendre les composés en question très utiles dans le traitement de certains syndromes pathologiques du système cardiovasculaire en particulier dans le traitement de l'angine de poitrine, de l'hypertension, de l'arythmie, de l'insuffisance circulatoire cérébrale.

Dans le domaine antitumoral, les composés de l'invention pourront être utiles comme potentialisateurs d'anticancéreux.

En conséquence, l'invention se rapporte également à des compositions pharmaceutiques ou vétérinaires contenant, comme principe actif, au moins un dérivé d'indolizine de formule I ou un sel pharmaceutiquement acceptable de ce dérivé, en association avec un véhicule pharmaceutique ou un excipient approprié.

Selon la voie d'administration choisie, la posologie journalière pour un être humain pesant 60 kg se situera entre 2 et 500 mg de principe actif.

Les composés de l'invention peuvent être obtenus comme suit :

I. – Les composés de formule I dans laquelle B représente un groupement –S– ou $-SO_2-$ et A représente un radical alkylène ayant de 2 à 5 atomes de carbone peuvent être préparés, selon l'invention, en condensant, en présence d'un accepteur d'acide et dans un solvant polaire tel qu'un alcool par exemple le butanol, une cétone telle que la méthyl-éthyl-cétone, ou un solvant non polaire tel qu'un hydrocarbure aromatique, par exemple le benzène, le toluène ou un xylène, un dérivé d'(alcoxy-4 benzènesulfonyl)-1 indolizine de formule générale :

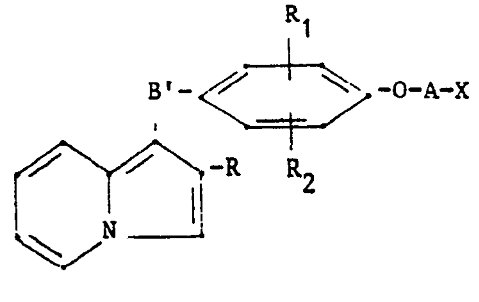

II

dans laquelle B′ représente un groupement –S– ou $-SO_2-$, R, $R_1$ et $R_2$ ont la même signification que précédemment, A représente un radical alkylène tel que défini dans la formule I et X représente un atome d'halogène,

de préférence le brome, ou un groupement alkylsulfonyloxy ayant de 1 à 4 atomes de carbone tel que par exemple méthanesulfonyloxy ou arylsulfonyloxy ayant de 6 à 10 atomes de carbone tel que benzènesulfonyloxy ou p-toluènesulfonyloxy avec une amine de formule générale :

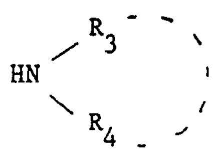

III

dans laquelle $R_3$ et $R_4$ ont la même signification que précédemment pour former le dérivé souhaité d'indolizine de formule I sous forme de base libre.

Généralement, la condensation en question est effectuée à une température comprise entre la température ambiante et la température de reflux du milieu, l'accepteur d'acide pouvant être par exemple un carbonate ou hydroxyde de métal alcalin ou un excès d'amine de formule III.

Les composés de formule II en question peuvent être obtenus :

a) Lorsque X est un halogène, par condensation d'un dérivé d'indolizine de formule générale :

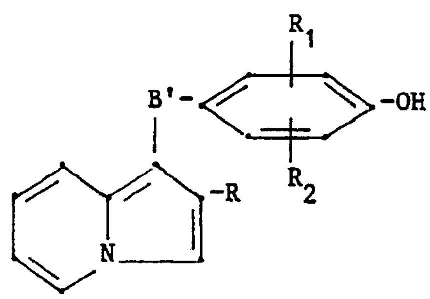

IV

dans laquelle B′, R, $R_1$ et $R_2$ ont la même signification que précédemment, avec un dihalogénoalcane de formule générale :

Hal-A-Hal V

dans laquelle A représente un radical alkylène tel que défini dans la formule I et Hal représente un atome d'halogène de préférence le brome et ce, au reflux, dans un solvant tel que la méthyl-éthyl-cétone ou le N,N-diméthylformamide et en présence d'un agent basique tel qu'un carbonate de métal alcalin par exemple le carbonate de potassium, un hydrure de métal alcalin tel que l'hydrure de sodium, un hydroxyde de métal alcalin par exemple l'hydroxyde de sodium ou de potassium ou un alcoolate de métal alcalin par exemple le méthylate ou l'éthylate de sodium,

b) lorsque X représente un groupement alkylsulfonyloxy ayant de 1 à 4 atomes de carbone ou arylsulfonyloxy ayant de 6 à 10 atomes de carbone par condensation d'un halogénure de formule générale :

Hal-W

dans laquelle W représente un radical alkylsulfonyle ayant de 1 à 4 atomes de carbone, par exemple méthanesulfonyle ou arylsulfonyle ayant de 6 à 10 atomes de carbone, par exemple benzènesulfonyle ou p-toluènesulfonyle, dans un solvant accepteur d'acide, par exemple la pyridine, avec un dérivé d'indolizine de formule générale :

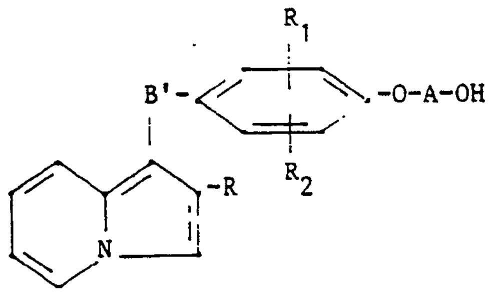

VI

dans laquelle B′, R, $R_1$ et $R_2$ ont la même signification que précédemment et A représente un radical alkylène tel que défini dans la formule I.

Quant aux composés de formule VI, ceux-ci peuvent être préparés en condensant dans un solvant approprié tel que le N,N-diméthylformamide et en présence d'un agent basique tel qu'un carbonate de métal alcalin par exemple le carbonate de potassium, un hydroxyde de métal alcalin tel que l'hydroxyde de sodium ou de potassium, un hydrure de métal alcalin tel que l'hydrure de sodium ou un alcoolate de métal alcalin par exemple le méthylate ou l'éthylate de sodium, un dérivé d'indolizine de formule IV ci-dessus, avec un alcool halogéné de formule générale :

Hal-A-OH VII

dans laquelle A représente un radical alkylène tel que défini dans la formule I et Hal a la même signification que précédemment.

Les composés de formule IV sont obtenus par hydrolyse au reflux et en milieu basique des composés de formule générale :

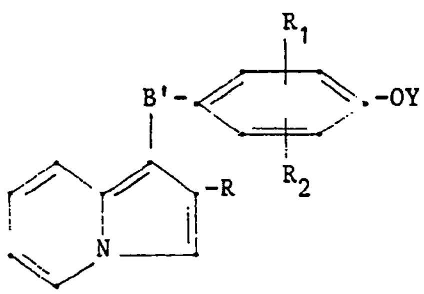

VIII

dans laquelle B′, R, $R_1$ et $R_2$ ont la même signification que précédemment et Y représente un radical alkylsulfonyle ayant de 1 à 4 atomes de carbone par exemple méthanesulfonyle, arylsulfonyle ayant de 6 à 10 atomes de carbone, par exemple benzènesulfonyle ou p-toluènesulfonyle ou alcanoyle ayant de 1 à 4 atomes de carbone par exemple acétyle.

Quant aux composés de formule VIII, ceux-ci peuvent être préparés par cyclisation au reflux d'un picolylsulfone ou sulfure de formule générale :

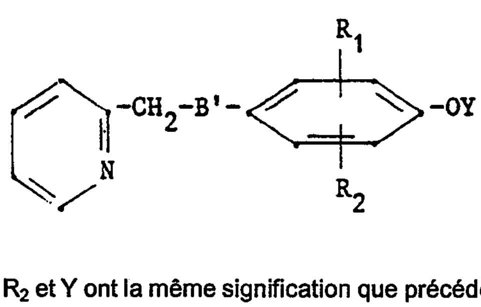

IX

dans laquelle B′, $R_1$, $R_2$ et Y ont la même signification que précédemment, avec une α-halogénocétone de formule générale :

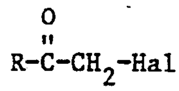

X

dans laquelle R et Hal ont la même signitication que précédemment et ce, dans un solvant tel que la méthyléthylcétone et en présence d'un agent basique tel qu'un carbone de métal alcalin par exemple le carbonate de potassium ou un bicarbonate de métal alcalin tel que le bicarbonate de sodium.

Quant aux picolylsulfones de formule IX, celles-ci sont obtenues par réaction d'une chlorométhyl-pyridine de formule générale :

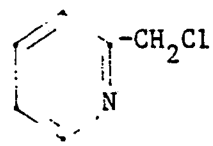

XI

avec un dérivé alkylsulfonyloxy-4 ayant de 1 à 4 atomes de carbone ou arylsulfonyloxy-4 ayant de 6 à 10 atomes de carbone benzènesulfinate de métal alcalin, par exemple le p-toluènesulfonyloxy-4 benzènesulfinate de sodium selon le procédé décrit dans J. Chem. Soc., 1965, p. 3090. Les dérivés de formule XI sont des produits connus ou pouvant être préparés par des méthodes connues.

Selon une variante, on peut obtenir les composés de formule IV dans laquelle B' représente un groupement –S– directement à partir d'un picolyl sulfure de formule générale :

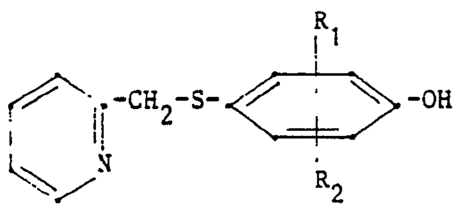

XII

dans laquelle $R_1$ et $R_2$ ont la même signification que précédemment, par réaction avec une α-halogénocétone de formule X et cyclisation du sel de pyridinium ainsi obtenu et ce, au reflux, dans un solvant tel que l'eau et en présence d'un agent basique tel qu'un carbonate de métal alcalin par exemple le carbonate de potassium ou un bicarbonate de métal alcalin par exemple le bicarbonate de sodium.

Les composés de formule XII sont des produits connus ayant été publiés dans J. Med. Chem. 26, 218 (1983) ou des produits qui peuvent être préparés par des procédés connus et les composés de formule IX dans laquelle B' représente un groupement –S– peuvent être obtenus à partir des composés de formule XII par réaction avec un halogénure d'alkylsulfonyle ayant de 1 à 4 atomes de carbone ou d'arylsulfonyle ayant de 6 à 10 atomes de carbone.

Suivant une méthode alternative, on peut également obtenir les composés de formule I dans laquelle B représente un groupement –S– ou $-SO_2-$ et A représente un radical alkylène ayant de 2 à 5 atomes de carbone de préférence ceux dans lesquels A représente un radical propylène, en faisant réagir, en présence d'un agent basique tel qu'un carbonate de métal alcalin par exemple le carbonate de potassium, un hydroxyde de métal alcalin tel que l'hydroxyde de sodium ou de potassium, un hydrure de métal alcalin tel que l'hydrure de sodium ou un alcoolate de métal alcalin par exemple le méthylate ou l'éthylate de sodium, une (hydroxy-4 benzènesulfonyl)-1 indolizine de formule IV ci-dessus, avec un composé de formule générale :

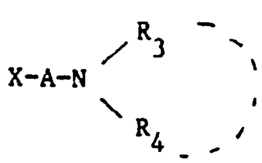

XIII

dans laquelle X représente un atome d'halogène, de préférence le chlore ou un radical alkylsulfonyle ayant de

1 à 4 atomes de carbone ou arylsufonyle ayant de 6 à 10 atomes de carbone tels que par exemple un radical benzènesulfonyle ou p-toluènesulfonyle, A représente un radical alkylène ayant de 2 à 5 atomes de carbone et $R_3$ et $R_4$ ont la même signification que précédemment, la réaction ayant lieu au reflux et dans un solvant polaire tel que la méthyl-éthyl-cétone ou le diméthylsulfoxyde pour former le dérivé désiré d'indolizine de formule I sous forme de base libre.

Les composés de formule XIII sont des produits connus ou pouvant être préparés par des méthodes connues.

II. – Les composés de formule I dans laquelle B représente un groupement –S– ou –$SO_2$– et A représente une chaîne hydroxy-2 propylène peuvent être obtenus en faisant réagir au reflux un dérivé d'indolizine de formule IV avec une épihalohydrine telle que l'épichlorhydrine ou l'épibromhydrine sous forme dextrogyre, lévogyre ou sous forme de mélange de ces isomères par exemple sous forme racémique et en présence d'un agent basique tel qu'un carbonate de métal alcalin par exemple le carbonate de potassium, un hydroxyde de métal alcalin par exemple l'hydroxyde de sodium ou de potassium, un hydrure de métal alcalin tel que l'hydrure de sodium ou un alcoolate de métal alcalin par exemple le méthylate ou l'éthylate de sodium et dans un solvant polaire tel que la méthyl-éthylcétone pour donner les dérivés oxyranylméthoxy de formule générale :

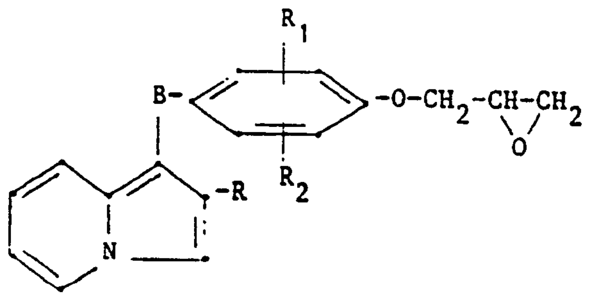

XIV

dans laquelle B, R, $R_1$ et $R_2$ ont la même signification que précédemment.

Les dérivés oxyranylméthoxy de formule XIV sont alors traités au reflux avec une amine de formule III et ce, dans un solvant polaire tel que la méthyl-éthyl-cétone ou dans un excès d'amine de formule III pour donner le dérivé désiré d'indolizine de formule I sous forme de base libre.

Dans certains cas, des produits secondaires peuvent se former parallèlement aux composés de formule XIV ci-dessus, en l'occurence des dérivés [(halogéno-3 hydroxy-2 propoxy)-4 benzènesulfonyl]-1 indolizine. Par réaction avec l'amine de formule III, ces dérivés donneront naissance néanmoins aux composés désirés de formule I dans laquelle A représente une chaîne hydroxy-2 propylène.

Les composés de formules II, IV, VI, VIII et XIV sont des composés intermédiaires nouveaux. A ce titre, ils constituent un autre objet de l'invention.

Globalement, ces composés de formules II, IV, VI, VIII et XIV peuvent être représentés par la formule générale :

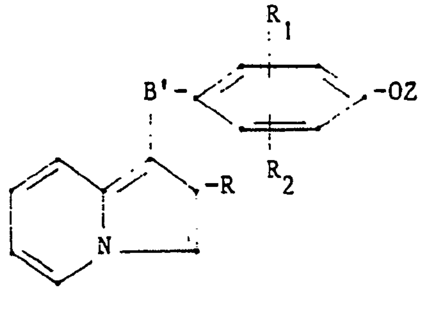

XV

dans laquelle B', R, $R_1$ et $R_2$ ont la même signification que précédemment et Z représente l'hydrogène, un radical oxyranylméthyle, alkylsulfonyle ayant de 1 à 4 atomes de carbone, arylsulfonyle ayant de 6 à 10 atomes de carbone ou un radical de formule :

$$-A-Z_1$$

dans laquelle A représente un radical alkylène linéaire ou ramifié, ayant de 2 à 5 atomes de carbone et $Z_1$ repré-

sente un atome d'halogène, le radical hydroxy ou un radical alkylsulfonyloxy ayant de 1 à 4 atomes de carbone ou arylsulfonyloxy ayant de 6 à 10 atomes de carbone.

Les composés de formule I ainsi obtenus sous forme de base libre peuvent ensuite être transformés en sels pharmaceutiquement acceptables par réaction avec un acide organique ou inorganique approprié par exemple l'acide oxalique, maléique, fumarique, méthanesulfonique, benzoïque, ascorbique, pamoïque, succinique, hexamique, bisméthylènesalicylique, éthanedisulfonique, acétique, propionique, tartrique, salicylique, citrique, gluconique, lactique, malique, cinnamique, mandélique, citraconique, aspartique, plamitique, stéarique, itaconique, glycolique, p-aminobenzoïque, glutamique, benzènesulfonique, théophylinne acétique, ou avec la lysine ou l'histidine.

On connaît déjà dérivés de benzofuranne et de benzothiophène doués de propriétés sur le système cardiovasculaire, ces dérivés étant caractérisés par une chaîne alkylaminobenzènesulfonyle (brevet US n° 3.947.470)".

De même, on a déjà décrit des dérivés d'indolizine substitués en position 1 par une chaîne alkyloxybenzoyle elle-même substituée par un groupement aminé et présentés comme ayant des effets pharmacologiques dans le domaine cardiovasculaire.

A cet effet, on peut citer le brevet français No. 2.341.578 et Eur. J. Med. Chem. 1977, 12, No. 4pp. 345-350 lesquels décrivent spécifiquement des éthyl -2, n-propyl-2 ou n-butyl-2 [(di-n-propyl- ou di-n-butylamino-3 propoxy)-4 benzoyl]-1 indolizine éventuellement diméthylés sur le radical benzoyle.

Ces composés connus ont révélé des activités antiadrénergiques nulles ou faibles, en tout état de cause trop faibles pour être d'un apport quelconque à la thérapeutique.

On a découvert de manière surprenante, dans le cadre de la présente invention, qu'en remplaçant le groupement carbonyle au niveau de la chaîne alkyloxybenzoyle des composés connus par un groupement sulfonyle, on obtient des composés présentant des activités antiadrénergiques α et β beaucoup plus importantes que celles des composés de l'art antérieur.

D'autre part, on a constaté que l'activité inhibitrice calcique des composés de l'invention est au moins égale sinon supérieure à celle observée lors de tests pratiqués avec les composés connus. Au contraire des composés connus, on a ainsi pu mettre en évidence pour les composés de la présente invention un spectre pharmacologique faisant apparaître les composantes anticalcique et antiadrénergique α et β avec un équilibre d'intensité thérapeutiquement valable par exemple pour le traitement de l'angor.

On connaît, par ailleurs, de US-A-3 947 470 des dérivés de benzofuranne et de benzothiophène substitués présentant une activité vasodilatatrice coronarienne.

En outre, on connaît de US-A-4 117 128 des dérivés sulphonyl-benzofuranne et benzothiophènes actifs dans le traitement de l'angine de poitrine, notamment par leur effet vasodilatateur coronarien.

Comme il a été rapporté en détail par R. CHARLIER dans "Bruxelles Medical", N° 9, septembre 1969, pages 543-560, il est admis qu'une médication antiangineuse doit être capable notamment d'antagoniser les réactions cardiovasculaires de type adrénergique. A cet effet, on a proposé des agents aptes à bloquer les récepteurs α.

Cependant l'application clinique de tels composés au traitement de l'angor resta sans succès très probablement par le fait que les antagonistes des récepteurs α n'induisent qu'une neutralisation très partielle du système adrénergique, l'activité des récepteurs β étant respectée.

Or, les manifestations hémodynamiques les plus indésirables qui surviennent chez l'angineux de poitrine au cours de ses accès douloureux sont avant tout cardiaques et relèvent de ce fait des récepteurs β.

Parallèlement, on a proposé des médications antagonistes des récepteurs adrénergiques β. Ces composés, d'un intérêt clinique réel, diminuent les crises d'angor en réduisant le travail cardiaque par ralentissement de la fréquence du rythme. Cependant, il n'y a pas de chute des résistances artérielles périphériques qui s'élèvent au contraire par libération du tonus α.

Ces médications modifient toutefois certains paramètres hémodynamiques dans un sens qui, sur le plan fondamental, constitue une contrepartie défavorable à l'angineux de poitrine en particulier et au cardiaque en général.

Si l'on considère l'aspect antiadrénergique des β-bloquants, il devient évident que seules la tachycardie et l'augmentation de la puissance et de la vélocité de la contraction cardiaque sont susceptibles d'être neutralisées, l'hypertension artérielle relevant d'une stimulation des récepteurs α sur lesquels les antagonistes β n'ont pas d'action.

Or, si les perturbations cardiovasculaires entraînées par la stimulation des récepteurs β sont les plus préjudiciables à l'angineux, il n'en reste pas moins que l'hypertension artérielle joue également un rôle qui n'est pas négligeable.

Au surplus, le blocage des récepteurs β comporte un risque privant l'insuffisant cardiaque d'un mécanisme compensateur qu'il met normalement en jeu pour limiter son insuffisance circulatoire.

Ce mécanisme réflexe dont la composante principale emprunte la voie du système β-adrénergique aboutit notamment à une augmentation de la puissance et de la vélocité de la contraction cardiaque. Par conséquent, si ce système est bloqué, l'insuffisant cardiaque voit s'aggraver sa défaillance fonctionnelle. Il est donc logique de considérer que l'emploi d'un β-bloquant dont l'action est pure et complète comportera toujours un risque cardiaque.

Il paraît donc souhaitable de ne pas rechercher des propriétés antagonistes α ou β complètes, étant donné les effets secondaires qu'elles peuvent entraîner en clinique. Il semble plus logique de viser à amortir plutôt qu'a supprimer les perturbations cardiovasculaires qui caractérisent l'hyperstimulation du système adrénergique dans sa totalité.

Les composés de l'invention répondent à cet objectif puisqu'ils présentent des propriétés antiadrénergiques de type α et β incomplètes. Ils peuvent donc être considérés, non comme des β-bloquants mais comme des adrénofreinateurs c'est-à-dire des antagonistes partiels des réactions adrénergiques α et β potentiellement dépourvues des désavantages énumérés ci-dessus pour les β-bloquants.

En outre, la composante inhibitrice calcique mise en évidence chez les composés de l'invention complétera d'une manière remarquable leur spectre pharmacologique cardiovasculaire.

On sait, en effet, que la translocation des ions calcium est une des composantes essentielles du potentiel d'action au niveau des cellules cardiaques et que, à ce titre, elle joue un rôle primordial dans la conduction électrique ainsi que dans ses troubles éventuels (arythmie). En outre, il est connu que les ions calcium sont impliqués dans le couplage excitation-contraction lequel contrôle, au niveau de la musculature lisse le degré de vasoconstriction et, par la même occasion, joue un rôle critique dans la crise d'angine de poitrine.

Les composés antagonistes du calcium agissent au niveau de la membrane cellulaire en empêchant sélectivement le calcium d'intervenir dans le processus de contraction au sein de la cellule artérielle.

Or, il apparaît de plus en plus évident, à l'heure actuelle, que les résultats cliniques apportés par l'association d'inhibiteurs calciques et d'inhibiteurs β-adrénergiques sont meilleurs que lorsque chaque inhibiteur est utilisé isolément (J.A.M.A. 1982, 247, pages 1911-1917).

Il semble, en outre, qu'il n'existe, à l'heure actuelle, aucun β-bloquant exerçant en plus une action inhibitrice appréciable au niveau de la translocation calcique.

De ce point de vue, les composés de l'invention présentant à la fois une composante anticalcique et une composante antiadrénergique α et β seront d'un intérêt primordial car susceptibles d'applications thérapeutiques plus étendues qu'un β-bloquant isolé ou qu'un inhibiteur calcique isolé. A titre d'exemple, on citera :

- l'éthyl-2{{{N-Méthyl N-(diméthoxy-3,4 β-phénéthyl)amino]-3 propyl}oxy-4 benzènesulfonyl}}-1 indolizine (Ex. 28)
- l'isopropyl-2[(di-n-butylamino-3 propyl)-oxy-4 benzènesulfonyl]-1 indolizine (Ex. 1)
- l'éthyl-2 [(di-n-butylamino-3 propyl)oxy-4 benzènesulfonyl]-1 indolizine (Ex. 8) et
- l'isopropyle-2{{{[N-méthyl N-(diméthoxy-3,4 β-phénéthyl)amino]-3 propyl}oxy-4 benzènesulfonyl}}-1 indolizine (Ex. 30)

qui présentent une composante antiadrénergique α et β doublée d'un effet économisateur d'oxygène pouvant apporter un effet thérapeutique chez l'homme dans le syndrome d'angine d'effort, lequel peut par ailleurs être traité par les β-bloquants classiques. Cependant l'intérêt majeur de ces composés résidera dans le fait qu'ils pourront, grâce à leur composante anticalcique, être utilisés dans le traitement de l'angine au repos, syndrome provoqué par l'apparition d'un spasme au niveau des coronaires lequel est combattu actuellement par des composés tels que le diltiazem, le vérapamil ou la nifépidine.

Au surplus, des composés de l'invention se sont également montrés capables de provoquer une augmentation du débit coronaire de manière importante.

Les résultats de tests pharmacologiques effectués en vue de déterminer les propriétés cardiovasculaires des composés de l'invention sont répertoriés ci-dessous.

I. Propriétés inhibitrices calciques

Les propriétés inhibitrices de la translocation calcique au niveau membranaire présentées par les composés de l'invention ont été mises en évidence par mesure de leur action antagoniste vis-à-vis de la réponse contractile à la dépolarisation provoquée par le potassium sur l'aorte isolée de rat. Il est bien établi que la dépolarisation de la membrane d'un muscle lisse par le potassium rend cette dernière perméable au calcium extracellulaire et provoque la contraction musculaire.

Dès lors, la mesure de l'inhibition de la réponse contractile à la dépolarisation par le potassium ou la mesure d'un relâchement de la contraction tonique à la dépolarisation potassique peut constituer une évaluation de la puissance d'un composé en tant qu'inhibiteur de la perméabilité membranaire aux ions $Ca^{++}$.

La technique utilisée est la suivante :

Sur des rats mâles Wistar pesant environ 300 g, on prélève l'aorte et on la coupe en bandelettes d'environ 40 mm de longueur et 3 mm de largeur.

On place ces fragments dans une cuve à organe isolé de 25 ml contenant une solution de Krebs-bicarbonate modifiée (NaCl 112 mM ; KCl 5 mM ; $NaHCO_3$ 25 mM ; $KH_2PO_4$ 1 mM ; $MgSO_4$ 1,2 mM ; $CaCl_2$ 2,5 mM ; glucose 11,5 mM ; eau distillée jusqu'à 1000 ml) parcourue par un courant de carbogène et maintenue à 37°C. On relie la préparation à un microcapteur de force et on enregistre la réponse contractile après amplification sur un enregistreur.

On applique une tension de 2 g à la préparation. On maintient celle-ci durant 60 minutes dans la solution de Krebs-bicarbonate modifiée puis on provoque les contractions en remplaçant la solution de Krebs-bicarbonate par une solution de Krebs-potassique (NaCl 17 mM ; KCl 100 mM ; $NaHCO_3$ 25 mM ; $KH_2PO_4$ 1 mM ; $MgSO_4$ 1,2 mM ; $CaCl_2$ 2,5 mM ; glucose 11,5 mM ; eau distillée jusqu'à 1000 ml). Lorsque la réponse contractile de la préparation est devenue reproductible, on introduit, dans le bain, une quantité donnée d'un composé de l'invention. Soixante minutes plus tard un nouveau spasme est provoqué par la dépolarisation potassique.

Les résultats obtenus sur l'aorte éprouvée sont alors exprimés en % de l'effet contracturant maximal avant l'incubation avec la substance à tester.

A titre d'exemples, les résultats suivants ont été obtenus, les composés de formule I étant sous la forme de base, de chlorhydrate ou d'oxalate.

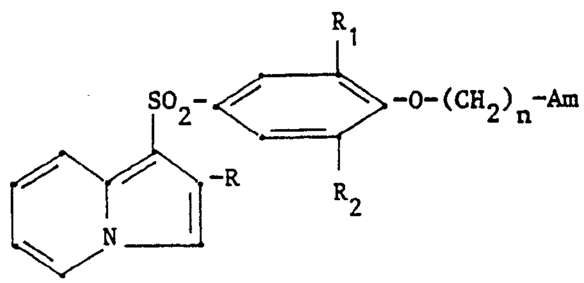

a) A la dose de $10^{-6}$M

| Composés | R | $R_1$ | $R_2$ | n | Am | % de l'effet contractant maximal |
|---|---|---|---|---|---|---|
| Ex. 12 | $n-C_4H_9$ | H | H | 3 | $-N(n-C_3H_7)_2$ | 36,3 |
| Ex. 13 | $n-C_4H_9$ | H | H | 3 | $-N(n-C_4H_9)_2$ | 39,8 |
| Ex. 29 | $n-C_4H_9$ | H | H | 3 | $-NH-C(CH_3)_3$ | 30,7 |
| Ex. 18 | - 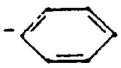 | H | H | 3 | $-N(n-C_4H_9)_2$ | 55,6 |
| Ex. 19 | - 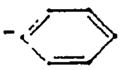 | $CH_3$ | H | 3 | $-N(n-C_4H_9)_2$ | 77,2 |
| Ex. 24 | - 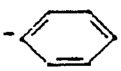 | H | H | 3 | $-NH-C(CH_3)_3$ | 62,1 |
| Ex. 25 | - 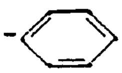 | H | $CH_3$ | 3 | $-NH-C(CH_3)_3$ | 67,7 |
| Ex. 21 | $-C_2H_5$ | H | H | 3 | $-NH(n-C_4H_9)$ | 8,3 |
| Ex. 20 | $-C_2H_5$ | H | H | 3 | $-N(CH_3)(n-C_4H_9)$ | 6,8 |
| Ex. 10 | $-C_2H_5$ | $CH_3$ | $CH_3$ | 3 | $-N(n-C_4H_9)_2$ | 2,9 |
| Ex. 4 | $-CH_3$ | H | H | 3 | $-N(C_2H_5)_2$ | 77,2 |
| Ex. 5 | $-CH_3$ | H | H | 3 | $-N(n-C_3H_7)_2$ | 48,9 |
| Ex. 6 | $-CH_3$ | H | H | 3 | $-N(n-C_4H_9)_2$ | 13,9 |
| Ex. 7 | $-C_2H_5$ | H | H | 3 | $-N(n-C_3H_7)_2$ | 8,3 |
| Ex. 9 | $-C_2H_5$ | $CH_3$ | H | 3 | $-N(n-C_4H_9)_2$ | 17,4 |
| Ex. 3 | $-C_2H_5$ | H | H | 3 | $-NH-C(CH_3)_3$ | 30,7 |
| Ex. 2 | $-C_2H_5$ | H | H | 3 | - 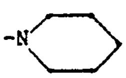 | 22,6 |
| Ex. 11 | $-n-C_3H_7$ | H | H | 3 | $-N(n-C_4H_9)_2$ | 8,8 |
| Ex. 14 | $-CH(CH_3)_2$ | H | H | 3 | $-N(CH_3)_2$ | 32,6 |
| Ex. 15 | $-CH(CH_3)_2$ | H | H | 3 | $-N(C_2H_5)_2$ | 18,4 |
| Ex. 17 | $-CH(CH_3)_2$ | H | H | 3 | $-N(n-C_3H_7)_2$ | 7,4 |
| Ex. 1 | $-CH(CH_3)_2$ | H | H | 3 | $-N(n-C_4H_9)_2$ | 2,0 |
| Ex. 31 | $-CH(CH_3)_2$ | H | H | 3 | $-NH-CH_2-CH_2-$ 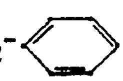 | 16,8 |
| Ex. 32 | $-CH(CH_3)_2$ | H | H | 3 | $-NH-CH_2-$ 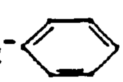 | 3,6 |

| | | | | | | |
|---|---|---|---|---|---|---|
| Ex. 33 | $-CH(CH_3)_2$ | H | H | 3 | 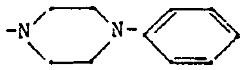 | 24,6 |
| Ex. 34 | $-CH(CH_3)_2$ | H | H | 3 | $-NH-CH_2-CH_2-$ 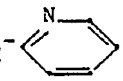 | 3,3 |
| Ex. 35 | $-CH(CH_3)_2$ | H | H | 3 | 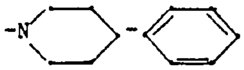 | 8,3 |
| Ex. 36 | $-CH(CH_3)_2$ | H | H | 3 | $-N(n-C_8H_{17})_2$ | 83,3 |
| Ex. 44 | $-CH(CH_3)_2$ | H | H | 3 | -NH- 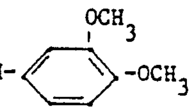 | 29,8 |
| Ex. 51 | 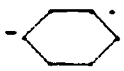 | H | H | 3 | $-N(n-C_4H_9)_2$ | 58,9 |

b) A la dose de $10^{-7}$M

| | | | | | | |
|---|---|---|---|---|---|---|
| Ex. 21 | $-C_2H_5$ | H | H | 3 | $-NH(n-C_4H_9)$ | 74,7 |
| Ex. 20 | $-C_2H_5$ | H | H | 3 | $-N(CH_3)(n-C_4H_9)$ | 60,0 |
| Ex. 10 | $-C_2H_5$ | $CH_3$ | $CH_3$ | 3 | $-N(n-C_4H_9)_2$ | 49,0 |
| Ex. 22 | $-C_2H_5$ | H | H | 2 | $-N(n-C_4H_9)_2$ | 37,0 |
| Ex. 23 | $-C_2H_5$ | H | H | 4 | $-N(n-C_4H_9)_2$ | 24,1 |
| Ex. 26 | $-C_2H_5$ | H | H | 3 | $-N(n-C_5H_{11})_2$ | 42,9 |
| Ex. 6 | $-CH_3$ | H | H | 3 | $-N(n-C_4H_9)_2$ | 69,1 |
| Ex. 7 | $-C_2H_5$ | H | H | 3 | $-N(n-C_3H_7)_2$ | 60,0 |
| Ex. 8 | $-C_2H_5$ | H | H | 3 | $-N(n-C_4H_9)_2$ | 30,9 |
| Ex. 9 | $-C_2H_5$ | $CH_3$ | H | 3 | $-N(n-C_4H_9)_2$ | 57,2 |
| Ex. 2 | $-C_2H_5$ | H | H | 3 | 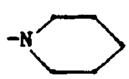 | 79,8 |
| Ex. 11 | $-n-C_3H_7$ | H | H | 3 | $-N(n-C_4H_9)_2$ | 37,7 |
| Ex. 15 | $-CH(CH_3)_2$ | H | H | 3 | $-N(C_2H_5)_2$ | 71,2 |
| Ex. 17 | $-CH(CH_3)_2$ | H | H | 3 | $-N(n-C_3H_7)_2$ | 50,4 |
| Ex. 1 | $-CH(CH_3)_2$ | H | H | 3 | $-N(n-C_4H_9)_2$ | 18,9 |
| Ex. 16 | $-C(CH_3)_3$ | H | H | 3 | $-N(n-C_4H_9)_2$ | 35,8 |

| | | | | | | |
|---|---|---|---|---|---|---|
| Ex. 27 | $-C_2H_5$ | H | H | 3 | 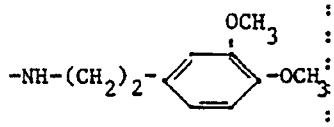 | 7,7 |
| Ex. 28 | $-C_2H_5$ | H | H | 3 | 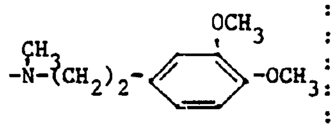 | 14,2 |
| Ex. 30 | $-CH(CH_3)_2$ | H | H | 3 | 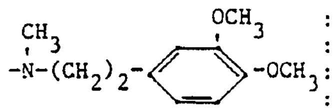 | 9,4 |
| Ex. 31 | $-CH(CH_3)_2$ | H | H | 3 | 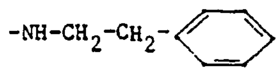 | 31,8 |
| Ex. 32 | $-CH(CH_3)_2$ | H | H | 3 | 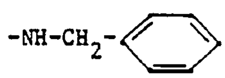 | 19,4 |
| Ex. 33 | $-CH(CH_3)_2$ | H | H | 3 | 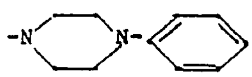 | 57,6 |
| Ex. 34 | $-CH(CH_3)_2$ | H | H | 3 | 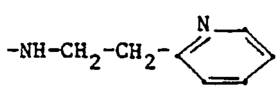 | 28,1 |
| Ex. 35 | $-CH(CH_3)_2$ | H | H | 3 | 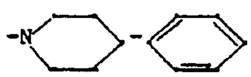 | 25,0 |
| Ex. 36 | $-CH(CH_3)_2$ | H | H | 3 | $-N(n-C_8H_{17})_2$ | 93,7 |
| Ex. 38 | $-C_2H_5$ | H | H | 3 | 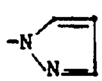 | 85,9 |
| Ex. 37 | $-CH(CH_3)_2$ | H | H | 3 | $-N(n-C_5H_{11})_2$ | 48,7 |
| Ex. 39 | $-CH(CH_3)_2$ | H | H | 4 | $-N(n-C_4H_9)_2$ | 17,9 |
| Ex. 40 | $-C_2H_5$ | H | H | 5 | $-N(n-C_4H_9)_2$ | 49,7 |
| Ex. 41 | $-CH(CH_3)_2$ | H | H | 3 | 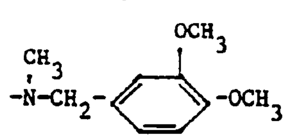 | 5,3 |

| | | | | | | |
|---|---|---|---|---|---|---|
| Ex. 46 | $-CH(CH_3)_2$ | H | H | 3 | 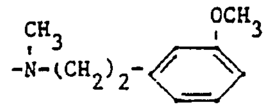 | 4,3 |
| Ex. 47 | $-CH(CH_3)_2$ | H | H | 3 | 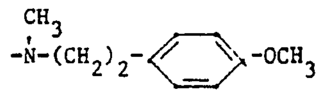 | 17,9 |
| Ex. 44 | $-CH(CH_3)_2$ | H | H | 3 | 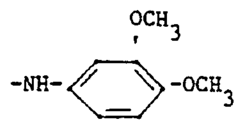 | 78,9 |
| Ex. 43 | $-CH(CH_3)_2$ | H | H | 3 | 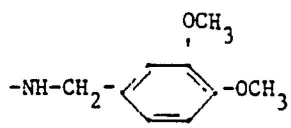 | 9,7 |
| Ex. 42 | $-CH(CH_3)_2$ | H | H | 4 | 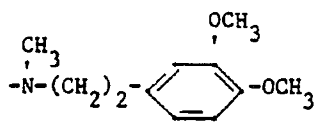 | 21,1 |
| Ex. 45 | $-CH(CH_3)_2$ | H | H | 3 | 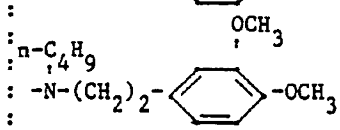 | 18,7 |
| Ex. 51 | 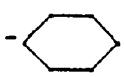 | H | H | 3 | $-N(n-C_4H_9)_2$ | 80,4 |

c) <u>A la dose de $10^{-8}$M</u>

| | | | | | | |
|---|---|---|---|---|---|---|
| Ex. 10 | $-C_2H_5$ | $CH_3$ | $CH_3$ | 3 | $-N(n-C_4H_9)_2$ | 82,6 |
| Ex. 22 | $-C_2H_5$ | H | H | 2 | $-N(n-C_4H_9)_2$ | 90,2 |
| Ex. 23 | $-C_2H_5$ | H | H | 4 | $-N(n-C_4H_9)_2$ | 77,8 |
| Ex. 26 | $-C_2H_5$ | H | H | 3 | $-N(n-C_5H_{11})_2$ | 82,9 |
| Ex. 1 | $-CH(CH_3)_2$ | H | H | 3 | $-N(n-C_4H_9)_2$ | 61,0 |
| Ex. 16 | $-C(CH_3)_3$ | H | H | 3 | $-N(n-C_4H_9)_2$ | 78,7 |

| | | | | | | |
|---|---|---|---|---|---|---|
| Ex. 27 | $-C_2H_5$ | H | H | 3 | 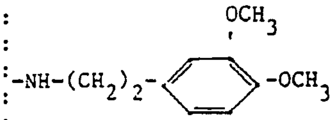 | 58,9 |
| Ex. 28 | $-C_2H_5$ | H | H | 3 | 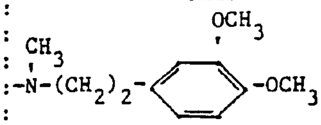 | 60,2 |
| Ex. 30 | $-CH(CH_3)_2$ | H | H | 3 | 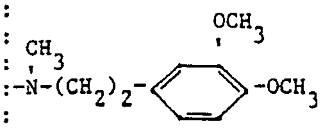 | 40,1 |
| Ex. 31 | $-CH(CH_3)_2$ | H | H | 3 | 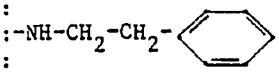 | 73,9 |
| Ex. 32 | $-CH(CH_3)_2$ | H | H | 3 | 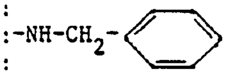 | 65,9 |
| Ex. 33 | $-CH(CH_3)_2$ | H | H | 3 | 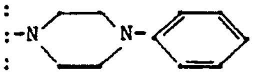 | 87,0 |
| Ex. 34 | $-CH(CH_3)_2$ | H | H | 3 | 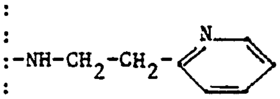 | 82,6 |
| Ex. 35 | $-CH(CH_3)_2$ | H | H | 3 | 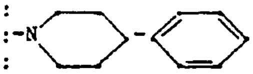 | 65,6 |
| Ex. 37 | $-CH(CH_3)_2$ | H | H | 3 | $-N(n-C_5H_{11})_2$ | 77,8 |
| Ex. 39 | $-CH(CH_3)_2$ | H | H | 4 | $-N(n-C_4H_9)_2$ | 62,9 |
| Ex. 40 | $-C_2H_5$ | H | H | 5 | $-N(n-C_4H_9)_2$ | 87,9 |
| Ex. 41 | $-CH(CH_3)_2$ | H | H | 3 | 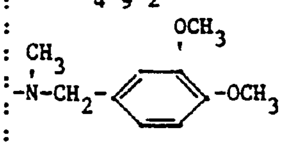 | 26,1 |

| | | | | | | |
|---|---|---|---|---|---|---|
| Ex. 46 | $-CH(CH_3)_2$ | H | H | 3 | 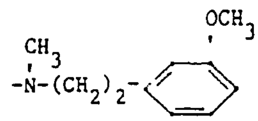 | 49,5 |
| Ex. 47 | $-CH(CH_3)_2$ | H | H | 3 | 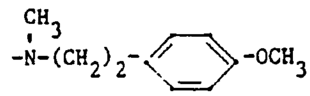 | 69,6 |
| Ex. 44 | $-CH(CH_3)_2$ | H | H | 3 | 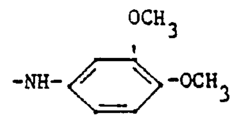 | 89,5 |
| Ex. 43 | $-CH(CH_3)_2$ | H | H | 3 | 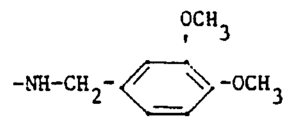 | 58,4 |
| Ex. 42 | $-CH(CH_3)_2$ | H | H | 4 | 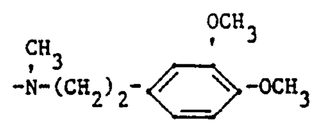 | 53,9 |
| Ex. 45 | $-CH(CH_3)_2$ | H | H | 3 | 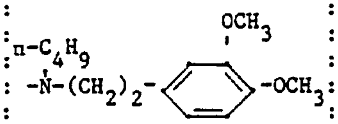 | 62,3 |

d) <u>A la dose de $10^{-9}$M</u>

| | | | | | | |
|---|---|---|---|---|---|---|
| Ex. 27 | $-C_2H_5$ | H | H | 3 | 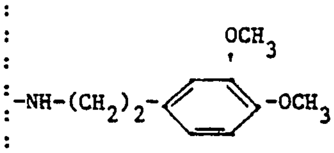 | 81,6 |
| Ex. 28 | $-C_2H_5$ | H | H | 3 | 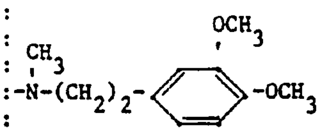 | 85,4 |
| Ex. 30 | $-CH(CH_3)_2$ | H | H | 3 | 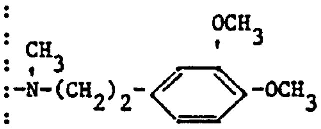 | 81,7 |
| Ex. 41 | $-CH(CH_3)_2$ | H | H | 3 | 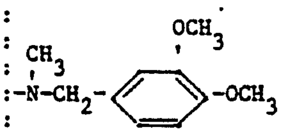 | 70,9 |

| | | | | | | |
|---|---|---|---|---|---|---|
| Ex. 46 | $-CH(CH_3)_2$ | H | H | 3 | 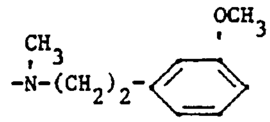 | 78,1 |
| Ex. 47 | $-CH(CH_3)_2$ | H | H | 3 | 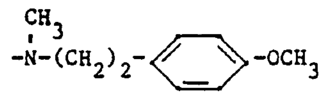 | 86,5 |
| Ex. 43 | $-CH(CH_3)_2$ | H | H | 3 | 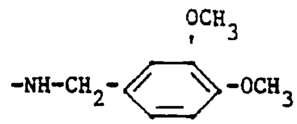 | 88,7 |
| Ex. 42 | $-CH(CH_3)_2$ | H | H | 4 | 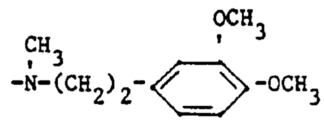 | 78,8 |
| Ex. 45 | $-CH(CH_3)_2$ | H | H | 3 | 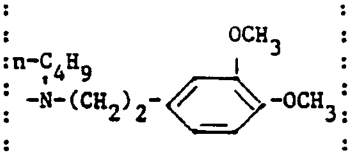 | 80,7 |

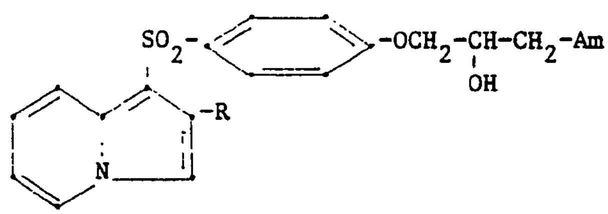

| Composé | R | Am | % de l'effet contracturant maximal | | | |
|---|---|---|---|---|---|---|
| | | | $10^{-6}M$ | $10^{-7}M$ | $10^{-8}M$ | $10^{-9}M$ |
| Ex.49 | $-CH(CH_3)_2$ | $-N-(n-C_4H_9)_2$ | 5,4 | 12,8 | 67,6 | 80,2 |
| Ex.50 | $-CH(CH_3)_2$ | 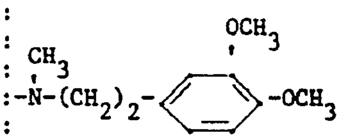 | 4,6 | 6,6 | 44 | 78 |

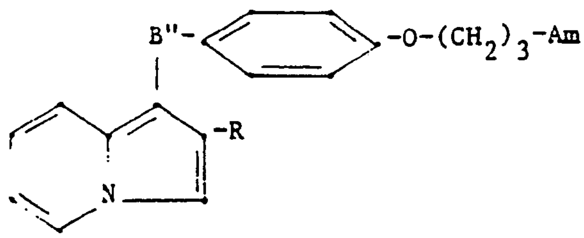

| Composé | B'' | R | Am | % de l'effet contracturant maximal | | | |
|---|---|---|---|---|---|---|---|
| | | | | $10^{-5}M$ | $10^{-6}M$ | $10^{-7}M$ | $10^{-8}M$ |
| Ex. 52 | -S- | $-CH(CH_3)_2$ | $-N-(n-C_4H_9)_2$ | 69,2 | 81,2 | - | - |
| Ex. 53 | -S- | $-CH(CH_3)_2$ | $CH_3$ $-N-(CH_2)_2-$ 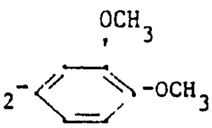 | 59,8 | 75,4 | - | - |

A titre de comparaison, on a obtenu les résultats suivants avec des composés connus :

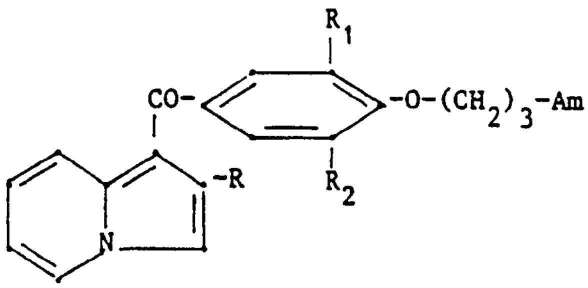

| Composé | R | $R_1$ | $R_2$ | Am | % de l'effet contracturant maximal | |
|---|---|---|---|---|---|---|
| | | | | | $10^{-6}$ M | $10^{-7}$ M |
| Composé A | $n-C_4H_9$ | H | H | $-N(n-C_4H_9)_2$ | 25,0 | 74,4 |
| Composé B | $-C_2H_5$ | $CH_3$ | $CH_3$ | $-N(n-C_4H_9)_2$ | 19,3 | 64,7 |
| Composé C | $-C_2H_5$ | H | H | $-N(n-C_3H_7)_2$ | 37,9 | 89,1 |

En établissant un rapport d'activité entre les composés de l'invention et les composés antérieurs correspondants, on obtient les résultats suivants :

| Composés | Rapport d'activité |
|---|---|
| Composé (Ex. 7) / Composé C | 6,0 |
| Composé (Ex. 13) / Composé A | 2,0 |
| Composé (Ex. 10) / Composé B | 2,2 |

Ces résultats montrent la supériorité des composés de l'invention sur les composés antérieurs correspondants.

II. Propriétés antiadrénergiques

Le but de ce test est de déterminer la capacité des composés de l'invention de réduire l'augmentation de la pression sanguine induite par l'épinéphrine (effet anti-α) et l'accélération de la fréquence cardiaque induite par l'isoprénaline (effet anti-β) chez le chien préalablement anesthésié au pentobarbital et atropiné.

On détermine d'abord pour chaque chien la dose d'éphinéphrine (entre 3 et 10 µg/kg) qui provoque une augmentation reproductible de la pression artérielle d'environ $133 \cdot 10^2$Pa et la dose d'isoprénaline (1 à 2 µg/kg qui provoque une augmentation reproductible de la fréquence cardiaque d'environ 70 battements/minute. On injecte alternativement toutes les dix minutes la dose d'épinéphrine et d'isoprénaline ainsi déterminée et après obtention de deux réponses de référence successives, on administre une quantité du composé à étudier par voie intraveineuse.

– Effet anti-α

On enregistre le pourcentage de réduction de l'hypertension provoquée par le composé à étudier comparativement à l'hypertension de référence obtenue précédemment (environ 100 mm Hg ($1,33\ 10^4$ Pa).

– Effet anti-β

On enregistre le pourcentage de réduction de l'accélération de la fréquence cardiaque provoquée par le composé à étudier comparativement à la tachycardie de référence mesurée précédemment (environ 70 battements).

Dans les deux cas, on a exprimé les résultats de la réduction de la pression artérielle ou de la fréquence cardiaque comme suit :

+ pour une réduction < 50%
++ pour une réduction ≧50%
+++ pour une réduction sub-totale (réduction presque complète)

On a enregistré les résultats suivants :

| Composés | Dose (mg/kg) | Effet anti-α | Effet anti-β |
|---|---|---|---|
| Ex. 7 | 5 | +++ | +++ |
| Ex. 8 | 0,5 | ++ | + |
| Ex. 9 | 1 | ++ | ++ |
| Ex. 26 | 0,5 | +++ | ++ |
| Ex. 20 | 5 | +++ | +++ |
| Ex. 21 | 5 | +++ | +++ |
| Ex. 2 | 5 | +++ | ++ |
| Ex. 27 | 1 | +++ | ++ |
| Ex. 28 | 0,1 | +++ | ++ |
| Ex. 23 | 1 | +++ | +++ |
| Ex. 11 | 1 | ++ | ++ |
| Ex. 12 | 5 | ++ | +++ |
| Ex. 13 | 5 | ++ | ++ |
| Ex. 29 | 5 | +++ | ++ |
| Ex. 14 | 2,5 | +++ | +++ |
| Ex. 15 | 2,5 | +++ | +++ |
| Ex. 17 | 1,3 | +++ | ++ |
| Ex 30 | 0,1 | +++ | +++ |
| Ex. 1 | 0,5 | ++ | ++ |
| Ex. 42 | 0,1 | +++ | +++ |
| Ex. 43 | 0,2 | +++ | +++ |
| Ex. 45 | 0,1 | ++ | ++ |
| Ex. 46 | 0,1 | +++ | +++ |
| Ex. 47 | 0,3 | ++ | + |
| Ex. 49 | 1 | +++ | ++ |
| Ex. 50 | 0,1 | +++ | +++ |
| Ex. 51 | 5,2 | +++ | ++ |
| Ex. 52 | 5,4 | +++ | ++ |
| Ex. 53 | 6,1 | +++ | + |
| Ex. 10 | 2,5 | +++ | + |

A titre de comparaison, les composés connus présentent les effets antiadrenergiques suivants :

| Composés | Dose (mg/kg) | Effet anti-α | Effet anti-β |
|---|---|---|---|
| Composé A | 10 | + | 0 |
| Composé B | 10 | + | + |
| Composé C | 10 | + | ++ |
| Composé D * | 10 | - | 0 |

* : éthyl-2[(di-n-butylamino-3 propyl)oxy-4 benzoyl]-1 indolizine

Ces résultats prouvent que les composés de l'invention présentent une activité antiadrénergique α et β beaucoup plus importante que celle des composés de l'art antérieur.

III. Toxicité

On a determiné, par voie intraveineuse chez la souris, la toxicité aiguë des composés de l'invention selon la méthode de LITCHFIELD et WILCOXON (J. Pharm. Exp. Therap. 1946, 96, 99).

On a obtenu les résultats suivants exprimés sous forme de $DL_{50}$ comparativement à un dérivé de benzoylindolizine en l'occurrence l'éthyl-2 [(di-n-butylamino-3 propyl)oxy-4 benzoyl]-3 indolizine ou butoprozine.

| Composés | $DL_{50}$ (mg/kg) |
|---|---|
| Ex. 6 | 31 |
| Ex. 11 | 28 |
| Ex. 7 | 26 |
| Ex. 18 | 35 |
| Ex. 19 | 60 |
| Ex. 13 | 31 |
| Ex. 9 | 55 |
| Ex. 28 | 32 |
| Ex. 30 | 140 |
| Butoprozine | 23 |

Ces résultats montrent que les composés de l'invention se comparent favorablement à la butoprozine en ce qui concerne la toxicité.

Les compositions thérapeutiques selon l'invention peuvent être présentées sous toute forme convenant à l'administration en thérapie humaine ou vétérinaire. Pour ce qui concerne l'unité d'administration, celle-ci peut prendre la forme, par exemple, d'un comprimé, d'une dragée, d'une capsule, d'une gélule, d'une poudre, d'une suspension ou d'un sirop pour l'administration orale, d'un suppositoire pour l'administration rectale ou d'une solution ou suspension pour l'administration parentérale.

Les compositions thérapeutiques de l'invention pourront comprendre, par unité d'administration, par exemple de 50 à 500 mg en poids d'ingrédient actif pour l'administration orale, de 50 à 200 mg d'ingrédient actif pour l'administration rectale et de 50 à 150 mg d'ingrédient actif pour l'administration parentérale.

Suivant la voie d'administration choisie, les compositions thérapeutiques ou vétérinaires de l'invention seront préparées en associant au moins un des composés de formule I ou un sel d'addition non toxique de ce composé avec un excipient approprié, ce dernier pouvant être constitué par exemple d'au moins un ingrédient

sélectionné parmi les substances suivantes : lactose, amidons, talc, stéarate de magnésium, polyvinylpyrrolidone, acide alginique, silice colloïdale, eau distillée, alcool benzylique ou agents édulcorants.

Les Exemples, non limitatifs suivants, illustrent l'invention :

EXEMPLE 1

Préparation de l'oxalate d'isopropyl-2 [(di-n-butylamino-3 propyl)oxy-4 benzènesulfonyl]-1 indolizine (SR 33513 A)

a) Isopropyl-2 (tosyloxy-4 benzènesulfonyl)-1 indolizine

On porte au reflux durant 22 heures, un mélange de 0,05 mole de tosyloxy-4 phényl β-picolylsulfone, 0,15 mole de bromo-1 méthyl-3 butan-2 one et 0,05 mole de carbonate de potassium dans 100 ml de méthyléthylcétone. Après ce laps de temps, on ramène le milieu réactionnel à la température ambiante puis on le filtre. On évapore soigneusement le filtrat sous vide afin de chasser la bromocétone en excès. On reprend le résidu pâteux dans de l'éther de pétrole, on le triture et on le filtre. On élimine ainsi les dernières traces de bromocétone.

On reprend le cake obtenu dans un mélange de 200 ml d'acétone/eau 70/30, on acidifie par quelques gouttes d'acide chlorhydrique puis on porte quelques minutes à ébullition. Après refroidissement et filtration, on isole un solide blanc que l'on peut recristalliser dans un mélange acétone/eau.

De cette manière, on obtient l'isopropyl-2 (tosyloxy-4 benzènesulfonyl)-1 indolizine avec un rendement de 70%.

P.F. : 180-183°C

A partir des produits de départ appropriés et en utilisant le procédé décrit ci-dessus, on a préparé les composés suivants :

Méthyl-2 (tosyloxy-4 benzènesulfonyl)-1 indolizine
P.F. : 169°C (acétone)
Ethyl-2 (tosyloxy-4 benzènesulfonyl)-1 indolizine
P.F. : 190°C (acétone)
n-Propyl-2 (tosyloxy-4 benzènesulfonyl)-1 indolizine
P.F. : 189°C (acétone)
Ethyl-2 (méthyl-3 tosyloxy-4 benzènesulfonyl)-1 indolizine
P.F. : 164°C (méthanol/chloroforme)
n-Butyl-2 (tosyloxy-4 benzènesulfonyl)-1 indolizine
P.F. : 145°C (acétone)
Phényl-2 (tosyloxy-4 benzènesulfonyl)-1 indolizine
P.F. : 168°C (dichloréthane)
Ethyl-2 (diméthyl-3,5 tosyloxy-4 benzènesulfonyl)-1 indolizine
P.F. : 161°C (acétone)
Cyclohexyl-2 (tosyloxy-4 benzènesulfonyl)-1 indolizine
P.F. : 173-175°C (acétone/eau)
tertio-Butyl-2 (tosyloxy-4 benzènesulfonyl)-1 indolizine
Huileux.

b) Isopropyl-2 (hydroxy-4 benzènesulfonyl)-1 indolizine

On verse 0,034 mole d'isopropyl-2 (tosyloxy-4 benzènesulfonyl)-1 indolizine dans un mélange de 80 ml d'eau contenant 0,34 mole d'hydroxyde de sodium et 80 ml d'éthanol puis on porte le mélange réactionnel au reflux pendant 24 heures.

Après refroidissement, on dilue la solution avec 300 ml d'eau puis on extrait à l'éther éthylique. Après acidification de la phase aqueuse, on observe la formation d'un précipité que l'on essore et sèche.

De cette manière, on obtient l'isopropyl-2 (hydroxy-4 benzènesulfonyl)-1 indolizine avec un rendement de 90%.

P.F. : 179-180°C (isopropanol/eau 3/1).

A partir des produits de départ appropriés et en utilisant le procédé décrit ci-dessus, on a préparé les composés suivants :

Méthyl-2 (hydroxy-4 benzènesulfonyl)-1 indolizine
P.F. : 177°C (méthanol/eau)

Ethyl-2 (hydroxy-4 benzènesulfonyl)-1 indolizine
P.F. : 204°C (acétate d'éthyle)
n-Propyl-2 (hydroxy-4 benzènesulfonyl)-1 indolizine
P.F. : 225°C (isopropanol)
Ethyl-2 (méthyl-3 hydroxy-4 benzènesulfonyl)-1 indolizine
P.F. : 214°C (isopropanol)
n-Butyl-2 (hydroxy-4 benzènesulfonyl)-1 indolizine
P.F. : 190°C (isopropanol)
Phényl-2 (hydroxy-4 benzènesulfonyl)-1 indolizine
P.F. : 234°C (méthanol)
Ethyl-2 (diméthyl-3,5 hydroxy-4 benzènesulfonyl)-1 indolizine
P.F. : 183°C (isopropanol)
tertio-Butyl-2 (hydroxy-4 benzènesulfonyl)-1 indolizine
P.F. : 169°C (chloroforme/éther de pétrole)
Cyclohexyl-2 (hydroxy-4 benzènesulfonyl)-1 indolizine
P.F. : 217°C (isopropanol/éther de pétrole)

c) Oxalate d'isopropyl-2 [(di-n-butylamino-3 propyl)oxy-4 benzènesulfonyl]-1 indolizine.

A 0,012 mole d'isopropyl-2 (hydroxy-4 benzènesulfonyl)-1 indolizine dans 100 ml de méthyléthylcétone, on ajoute 0,015 mole de chloro-1 di-n-butylamino-3 propane et 0,018 mole de carbonate de potassium finement broyé. On porte le mélange au reflux pendant 24 heures puis on le ramène à la température ambiante. On filtre les sels inorganiques et on évapore le filtrat sous le vide de la trompe à eau. On obtient une huile que l'on purifie par chromatographie sur colonne sèche d'alumine.

Le composé désiré, sous forme basique, ainsi purifié peut être isolé à l'état cristallin. On forme l'oxalate du produit obtenu par addition d'une quantité stoechiométrique d'acide oxalique à une solution de la base dissoute dans l'acétone.

De cette manière, on obtient l'oxalate d'isopropyl-2 [(di-n-butylamino-3 propyl)oxy-4 benzènesulfonyl]-1 indolizine avec un rendement de 26%.
P.F. : 133°C (isopropanol)

De la même manière que précédemment, on a préparé le composé suivant : Cyclohexyl-2 [(di-n-butylamino-3 propyl)oxy-4 benzènesulfonyl)-1 indolizine
P.F. : 130-131°C (methanol) (SR 33641) (Exemple 51)

## EXEMPLE 2

Préparation du chlorhydrate d'éthyl-2 [(pipéridino-3 propyl)oxy-4 benzènesulfonyl]-1 indolizine (SR 33528 A)

a) Ethyl-2 [(bromo-3 propyl)oxy-4 benzènesulfonyl]-1 indolizine

On dissout 0,01 mole d'éthyl-2 (hydroxy-4 benzènesulfonyl)-1 indolizine dans 50 ml de méthyléthylcétone. On ajoute 0,02 mole de carbonate de potassium et on porte le mélange au reflux pendant une heure.

On ajoute ensuite 0,04 mole de dibromo-1,3 propane et on poursuit le reflux durant 24 heures. Après réaction, on élimine les sels par filtration et on évapore la solution à sec. On purifie le résidu par chromatographie sur colonne de silice (solvant d'élution : dichloroéthane).

De cette manière, on obtient l'éthyl-2 [(bromo-3 propyl)oxy-4 benzènesulfonyl]-1 indolizine avec un rendement de 70%.
P.F. : 136°C (acétone)

A partir des produits de départ appropriés et en utilisant le procédé décrit ci-dessus, on a obtenu les composés suivants :
n-Butyl-2 [(bromo-3 propyl)oxy-4 benzènesulfonyl]-1 indolizine
P.F. : 119°C (acétone)
Isopropyl-2 [(bromo-3 propyl)oxy-4 benzènesulfonyl]-1 indolizine
P.F. : 131°C (acétone)
Phényl-2 [(bromo-3 propyl)oxy-4 benzènesulfonyl]-1 indolizine
P.F. : 199°C (dichloroéthane)
Ethyl-2 [(bromo-2 éthyl)oxy-4 benzènesulfonyl]-1 indolizine
Huileux

Ethyl-2 [(bromo-4 butyl)oxy-4 benzènesulfonyl]-1 indolizine
P.F. : 111°C (cyclohexane)
Isopropyl-2 [(bromo-4 butyl)oxy-4 benzènesulfonyl]-1 indolizine
P.F. : 111°C (acétate d'éthyle/éther de pétrole)
Phényl-2 [(bromo-3 propyl)oxy-4 méthyl-3 benzènesulfonyl]-1 indolizine
Huileux.

b) Chlorhydrate d'éthyl-2 [(pipéridino-3 propyl)oxy-4 benzènesulfonyl]-1 indolizine.

On met en solution dans 25 ml de butanol, 0,005 mole d'éthyl-2 [(bromo-3 propyl)oxy-4 benzènesulfonyl]-1 indolizine. On y ajoute 0,01 mole de carbonate de potassium et 0,01 mole de pipéridine puis on chauffe le mélange réactionnel au bain-marie pendant 20 heures.

Après ce laps de temps, on évapore à sec sous vide et on obtient ainsi une huile que l'on reprend dans l'éther éthylique. On élimine un insoluble formé de sels et on évapore à sec la solution éthérée. On purifie le résidu par chromatographie sur colonne sèche de silice en utilisant comme solvant un mélange chloroforme/méthanol 8/2 et on dissout l'huile pure obtenue, dans un mélange d'acétone et d'éther éthylique.

On forme alors le chlorhydrate par addition d'une solution d'acide chlorhydrique dans l'éther éthylique.

De cette manière, on obtient le chlorhydrate d'éthyl-2 [(pipéridino-3 propyl)oxy-4 benzènesulfonyl]-1 indolizine.
Rendement : 54%
P.F. : 183°C (acétone)

EXEMPLE 3

Préparation du chlorhydrate d'éthyl-2 [(tertiobutylamino-3 propyl)oxy-4 benzènesulfonyl]-1 indolizine (SR 33511 A)

On chauffe au bain-marie à 100°C et pendant 48 heures, un mélange de 0,007 mole d'éthyl-2 [(bromo-3 propyl)oxy-4 benzènesulfonyl]-1 indolizine et de 0,07 mole de tertiobutylamine dans 50 ml de toluène.

Après réaction, on évapore soigneusement à sec et sous vide et on reprend le résidu dans une solution aqueuse d'hydroxyde de sodium. On extrait avec du dichlorométhane et on évapore la phase organique à sec. On obtient un résidu huileux que l'on purifie par chromatographie sur colonne sèche de silice en utilisant comme solvant un mélange dichlorométhane/ammoniac/méthanol.

On reprend dans l'acétate d'éthyle le composé désiré purifié sous forme basique et on forme le chlorhydrate en y ajoutant, goutte à goutte, de l'acide chlorhydrique en solution dans l'éther éthylique.

De cette manière, on obtient le chlorhydrate d'éthyl-2 [(tertiobutylamino-3 propyl)oxy-4 benzènesulfonyl]-1 indolizine.
Rendement : 68%
P.F. : 229-231°C (acétate d'éthyle-méthanol)

A partir des produits de départ appropriés et en utilisant les procédés décrits dans les Exemples précédents, on a préparé les composés suivants :
Oxalate de méthyl-2 [(diéthylamino-3 propyl)oxy-4 benzènesulfonyl]-1 indolizine (SR 33520 A) (Exemple 4)
P.F. : 153°C (dichloroéthane/méthanol)
Méthyl-2 [(di-n-propylamino-3 propyl)oxy-4 benzènesulfonyl]-1 indolizine (SR 33518) (Exemple 5)
P.F. : 107-108°C (méthanol)
Oxalate de méthyl-2 [(di-n-butylamino-3 propyl)oxy-4 benzènesulfonyl]-1 indolizine (SR 33133 A) (Exemple 6)
P.F. : 131°C (acétate d'éthyle)
Chlorhydrate d'éthyl-2 [(di-n-propylamino-3 propyl)oxy-4 benzènesulfonyl]-1 indolizine (SR 33305 A) (Exemple 7)
P.F. : 192°C (acétone)
Chlorhydrate d'éthyl-2 [(di-n-butylamino-3 propyl)oxy-4 benzènesulfonyl]-1 indolizine (SR 33306 A) (Exemple 8)
P.F. : 153°C (acétone)
Chlorhydrate d'éthyl-2 [(di-n-butylamino-3 propyl)oxy-4 méthyl-3 benzènesulfonyl]-1 indolizine (SR 33508 A) (Exemple 9)
P.F. : 200-203°C (méthyléthylcétone/méthanol)
Chlorhydrate d'éthyl-2 [(di-n-butylamino-3 propyl)oxy-4 diméthyl-3,5 benzènesulfonyl]-1 indolizine (SR 33538 A) (Exemple 10)

P.F. : 136-137°C (acétate d'éthyle/méthanol)
Oxalate de n-propyl-2 [(di-n-butylamino-3 propyl)oxy-4 benzènesulfonyl]-1 indolizine (SR 33220 A) (Exemple 11)
P.F. : 111°C (isopropanol)
Oxalate de n-butyl-2 [(di-n-propylamino-3 propyl)oxy-4 benzènesulfonyl]-1 indolizine (SR 33507 A) (Exemple 12)
P.F. : 110-113°C (isopropanol)
Oxalate de n-butyl-2 [(di-n-butylamino-3 propyl)oxy-4 benzènesulfonyl]-1 indolizine (SR 33504 A) (Exemple 13)
P.F. : 85-87°C (acétate d'éthyle)
Isopropyl-2 [(diméthylamino-3 propyl)oxy-4 benzènesulfonyl]-1 indolizine (SR 33517) (Exemple 14)
P.F. : 90-92°C (diisopropyléther/diéthyléther)
Isopropyl-2 [(diéthylamino-3 propyl)oxy-4 benzènesulfonyl]-1 indolizine (SR 33516) (Exemple 15)
P.F. : 90-92°C (diisopropyléther)
tertio-Butyl-2 [(di-n-butylamino-3 propyl)oxy-4 benzènesulfonyl]-1 indolizine (SR 33541) (Exemple 16)
P.F. : 90-92°C (hexane)
Oxalate d'isopropyl-2 [(di-n-propylamino-3 propyl)oxy-4 benzènesulfonyl]-1 indolizine (SR 33512 A) (Exemple 17)
P.F. : 164-165°C (méthyléthylcétone/méthanol)
Chlorhydrate de phényl-2 [(di-n-butylamino-3 propyl)oxy-4 benzènesulfonyl]-1 indolizine (SR 33369 A) (Exemple 18)
P.F. : 158°C (acétone)
Chlorhydrate de phényl-2 [(di-n-butylamino-3 propyl)oxy-4 méthyl-3 benzènesulfonyl]-1 indolizine (SR 33486 A) (Exemple 19)
P.F. : 194°C (méthanol)
Oxalate d'éthyl-2 [(N-méthyl N-butylamino-3 propyl)oxy-4 benzènesulfonyl]-1 indolizine (SR 33533 A) (Exemple 20)
P.F. : 163°C (acétone)
Oxalate d'éthyl-2 [(n-butylamino-3 propyl)oxy-4 benzènesulfonyl]-1 indolizine (SR 33534 A) (Exemple 21)
P.F. : 141°C (acétone)
Chlorhydrate d'éthyl-2 [(di-n-butylamino-2 éthyl)oxy-4 benzènesulfonyl]-1 indolizine (SR 33547 A) (Exemple 22)
P.F. : 153°C (acétate d'éthyle)
Hémioxalate d'éthyl-2 [(di-n-butylamino-4 butyl)oxy-4 benzènesulfonyl]-1 indolizine (SR 33548 A) (Exemple 23)
P.F. : 150°C (acétate d'éthyle)
Chlorhydrate de phényl-2 [(tertiobutylamino-3 propyl)oxy-4 benzènesulfonyl]-1 indolizine (SR 33370 A) (Exemple 24)
P.F. : 228°C (acétone)
Chlorhydrate de phényl-2 [(tertiobutylamino-3 propyl)oxy-4 méthyl-3 benzènesulfonyl]-1 indolizine (SR 33485 A) (Exemple 25)
P.F. : 181°C (méthanol)
Chlorhydrate d'éthyl-2 [(di-n-pentylamino-3 propyl)oxy-4 benzènesulfonyl]-1 indolizine (SR 33550 A) (Exemple 26)
P.F. : 132-133°C (acétate d'éthyle/méthanol)
Oxalate d'éthyl-2 {[(diméthoxy-3,4 β-phénéthylamino)-3 propyl]oxy-4 benzènesulfonyl}-1 indolizine (SR 33544 A) (Exemple 27)
P.F. : 179-181°C (méthanol)
Ethyl-2 {{{[N-méthyl N-(diméthoxy-3,4 β-phénéthyl)amino]-3 propyl}oxy-4benzènesulfonyl}}-1 indolizine (SR 33549) (Exemple 28)
P.F. : 78-80°C (diisopropyl éther)
Oxalate de n-butyl-2 [(tertiobutylamino-3 propyl)oxy-4 benzènesulfonyl]-1 indolizine (SR 33503 A) (Exemple 29)
P.F. : 207-208°C (méthanol)
Isopropyl-2 {{{[N-méthyl N-(diméthoxy-3,4 β-phénéthyl)amino]-3 propyl} oxy-4 benzènesulfonyl}}-1 indolizine (SR 33557) (Exemple 30)
P.F. : 82-83°C (diisopropyléther/dichlorométhane)
Chlorhydrate d'isopropyl-2 [(β-phénéthylamino-3 propyl)oxy-4 benzènesulfonyl]-1 indolizine (SR 33577 A) (Exemple 31)

P.F. : 209-210°C (acétate d'éthyle/méthanol)
Chlorhydrate d'isopropyl-2 [(benzylamino-3 propyl)oxy-4 benzènesulfonyl]-1 indolizine (SR 33578 A) (Exemple 32)
P.F. : 193-195°C (acétate d'éthyle/méthanol)
Isopropyl-2 [(N-phénylpipérazino-3 propyl)oxy-4 benzènesulfonyl]-1 indolizine (SR 33579) (Exemple 33)
P.F. : 135-136°C (méthanol/dichlorométhane)
Dioxalate d'isopropyl-2 {[(pyridyl-2 éthylamino)-3 propyl]oxy-4 benzènesulfonyl}-1 indolizine (SR 33582 A) (Exemple 34)
P.F. : 154-156°C (méthanol)
Isopropyl-2 {[(phényl-3 pipéridino)-3 propyl]oxy-4 benzènesulfonyl}-1 indolizine (SR 33583) (Exemple 35)
P.F. : 79-80°C (méthanol)
Isopropyl-2 [(di-n-octylamino-3 propyl)oxy-4 benzènesulfonyl]-1 indolizine (SR 33584) (Exemple 36)
P.F. : < 50°C (pâteux)
Chlorhydrate d'isopropyl-2 [(di-n-pentylamino-3 propyl)oxy-4 benzènesulfonyl]-1 indolizine (SR 33603 A) (Exemple 37)
P.F. : 138°C (méthyléthylcétone/éther éthylique 2 :1)
Ethyl-2 {[(imidazolyl-1)-3 propyl]oxy-4 benzènesulfonyl}-1 indolizine (SR 33590) (Exemple 38)
P.F. : 130-131°C (acétate d'éthyle/méthanol/éther éthylique)
Isopropyl-2 [(di-n-butylamino-4 butyl)oxy-4 benzènesulfonyl]-1 indolizine (SR 33606) (Exemple 39)
P.F. : 96°C (n-hexane)
Ethyl-2 [(di-n-butylamino-5 pentyl)oxy-4 benzènesulfonyl]-1 indolizine (SR 33607) (Exemple 40)
P.F. : 89-90°C (n-hexane)
Isopropyl-2 {{{[N-méthyl N-(diméthoxy-3,4 benzyl)amino]-3 propyl}oxy-4 benzènesulfonyl}}-1 indolizine (SR 33611) (Exemple 41)
P.F. : 96-100°C (diisopropyléther/dichlorométhane)
Isopropyl-2 {{{[N-méthyl N-(diméthoxy-3,4 β-phénéthyl)amino]-4 butyl}oxy-4 benzènesulfonyl}}-1 indolizine (SR 33620) (Exemple 42)
P.F. : 84-86°C (hexane)
Isopropyl-2 {{{[(diméthoxy-3,4 benzyl)amino]-3 propyl}oxy-4 benzènesulfonyl}}-1 indolizine (SR 33621) (Exemple 43)
P.F. : 109-111°C (éther diisopropylique/dichloroéthane)
Chlorhydrate d'isopropyl-2 {[(diméthoxy-3,4 anilino)-3 propyl]oxy-4 benzènesulfonyl}-1 indolizine (SR 33624 A) (Exemple 44)
P.F. : 200-203°C (chlorure de méthylène)
Isopropyl-2 {{{[N-n-butyl N-(diméthoxy-3,4 β-phénéthyl)amino]-3 propyl} oxy-4 benzènesulfonyl}}-1 indolizine (SR 33629 A) (Exemple 45)
P.F. : 108-110°C (acétate d'éthyle/méthanol)
Oxalate acide d'isopropyl-2 {{{[N-méthyl N-(méthoxy-3 β-phénéthyl)amino]-3 propyl}oxy-4 benzènesulfonyl}}-1 indolizine (SR 33632 A) (Exemple 46)
P.F. : 111-113°C (acétate d'éthyle/méthanol)
Oxalate acide d'isopropyl-2 {{{[N-méthyl N-(méthoxy-4 β-phénéthyl)amino]-3 propyl}-4 benzènesulfonyl}}-1 indolizine (SR 33638) (Exemple 47)
P.F. : 140-144°C (acétate d'éthyle/méthanol)
Isopropyl-2 {[(diphénylméthyl-4 pipérazino)-3 propyl]oxy-4 benzènesulfonyl}-1 indolizine (SR 33663 A) (Exemple 48)
P.F. : 170°C (méthanol/dichlorométhane)

EXEMPLE 49

Préparation du chlorhydrate d'isopropyl-2 [(di-n-butylamino-3 hydroxy-2 propyl)oxy-4 benzènesulfonyl]-1 indolizine (SR 33644 A)

a) Isopropyl-2 [(époxy-2,3 propyl)oxy-4 benzènesulfonyl]-1 indolizine

Sous agitation, on chauffe à 90°C pendant 20 heures, un mélange de 0,02 mole d'isopropyl-2 (hydroxy-4 benzènesulphonyl)-1 indolizine, 0,02 mole de carbonate de potassium et 40 ml d'épichlorhydrine. Après cette période, on élimine sous vide l'épichlorhydrine en excès et on reprend le résidu dans du toluène. On lave la solution avec une solution diluée d'hydroxyde de sodium puis avec de l'eau. On évapore à sec sous vide la

phase organique pour obtenir une huile que l'on purifie par passage sur colonne de silice (éluant : dichlorométhane/acétate d'éthyle 95/5). Le produit désiré cristallise lentement.

De cette manière, on obtient l'isopropyl-2 [(époxy-2,3 propyl)oxy-4 benzènesulfonyl]-1 indolizine avec un rendement de 68%.

P.F. : 110-111°C (méthanol)

b) Chlorhydrate d'isopropyl-2 [(di-n-butylamino-3 hydroxy-2 propyl)oxy-4 benzènesulfonyl]-1 indolizine.

On porte au reflux durant une heure, une solution de 0,0027 mole d'isopropyl-2 [(époxy-2,3 propyl)oxy-4 benzènesulfonyl]-1 indolizine et 0,015 mole de di-n-butylamine dans 10 ml de méthanol. On ramène la solution à température ambiante et on élimine sous vide la di-n-butylamine en excès ainsi que le solvant. On reprend dans l'éther éthylique anhydre le résidu obtenu et on forme le chlorhydrate du composé désiré par ajout de chlorure d'hydrogène dans l'éther éthylique.

De cette manière, on obtient le chlorhydrate d'isopropyl-2[(di-n-butylamino-3 hydroxy-2 propyl)oxy-4 benzènesulfonyl]-1 indolizine que l'on recristallise dans un mélange acétone/éther éthylique.

Rendement : 68,9%

P.F. : 155-156°C.

EXEMPLE 50

Préparation du chlorhydrate d'isopropyl-2{{{[N-méthyl N-(diméthoxy-3,4 β-phénéthylamino]-3 hydroxy-2 propyl}oxy-4 benzènesulfonyl}}-1 indolizine (SR 33656 A)

A une solution de 0,075 mole d'isopropyl-2[(époxy-2,3 propyl)oxy-4 benzènesulfonyl]-1 indolizine dissous dans 25 ml de méthanol, on ajoute 0,01 mole de chlorhydrate de N-méthyl diméthoxy-3,4 β-phénéthylamine et 0,011 mole de triéthylamine. On porte le mélange au reflux pendant 5 heures. Après refroidissement, on évapore à sec sous vide le milieu réactionnel et on reprend le résidu huileux dans du dichlorométhane et de l'eau légèrement alcaline. On lave la phase organique, sèche et évapore sous vide. On obtient ainsi un composé brut que l'on purifie par chromatographie sur colonne de silice désactivée à la diéthylamine (éluant: dichlorométhane). On dissout le produit purifié dans l'éther éthylique anhydre et on forme le chlorhydrate du composé désiré par ajout de chlorure d'hydrogène dans l'éther éthylique. Le chlorhydrate en question précipite.

De cette manière, on obtient le chlorhydrate d'isopropyl-2 {{{[N-méthyl N-(diméthoxy-3,4 β-phénéthyl)amino]-3 hydroxy-2 propyl}oxy-4 benzènesulfonyl}}-1 indolizine.

P.F. : 110°C

EXEMPLE 52

Préparation de l'oxalate de [(di-n-butylamino-3 propyl)oxy-4 phénylthio]-1 isopropyl-2 indolizine (SR 33650 A)

a) Bromure de (méthyl-3 oxo-2 butyl)-1 {[(hydroxy-4 phényl)thio]méthyl}-2 pyridinium.

On chauffe à ébullition durant 24 heures, un mélange de 0,02 mole d' {[(hydroxy-4 phényl)thio]méthyl}-2 pyridine et de 0,03 mole de bromométhyl-isopropylcétone dans 160 ml d'acétone. Après cette période, on ramène le milieu réactionnel à la température ambiante et on observe un précipité qui augmente par addition d'éther éthylique sec. On filtre ce précipité, lave à l'éther éthylique sec et sèche sous vide.

De cette manière, on obtient le bromure de (méthyl-3 oxo-2 butyl)-1 {[(hydroxy-4 phényl)thio]méthyl}-2 pyridinium que l'on utilise sous forme brute.

Rendement : 65%

P.F. : 175°C

b) [(Hydroxy-4 phényl)thio]-1 isopropyl-2 indolizine

On dissout dans l'eau le bromure de pyridinium obtenu à l'étape précédente et on ajoute, à cette solution, un excès de bicarbonate de sodium. On chauffe le mélange pendant 25 minutes à 90°C puis on le ramène à température ambiante. On obtient ainsi une huile que l'on lave par décantation. On dissout cette huile dans du méthanol et on filtre puis évapore à sec la solution méthanolique. On purifie alors par chromatographie sur colonne de silice le produit brut obtenu (éluant : dichlorométhane/hexane 1/1).

De cette manière, on obtient l'[(hydroxy-4 phényl)thio]-1 isopropyl-2 indolizine avec un rendement de 90%. P.F. : 100°C.

c) Oxalate de [(di-n-butylamino-3 propyl)oxy-4 phénylthio]-1 isopropyl-2 indolizine.

A une solution de 0,01 mole d'[(hydroxy-4 phényl)thio]-1 isopropyl-2 indolizine dans 80 ml de diméthylsulfoxyde, on ajoute 5 g de carbonate de potassium anhydre et 0,015 mole de chloro-1 di-n-butylaminopropane. On maintient le milieu réactionnel sous agitation durant 24 heures ensuite on le lave avec 500 ml d'eau. On extrait la solution avec de l'éther éthylique et on lave la phase organique avec de l'eau. Après sèchage sur sulfate de sodium, on filtre et évapore à sec pour obtenir le produit désiré sous forme brute. On dissout alors ce produit brut dans l'éther éthylique sec et on ajoute une solution d'acide oxalique dans l'éther éthylique.

De cette manière, on obtient l'oxalate de [(di-n-butylamino-3 propyl) oxy-4 phénylthio]-1 isopropyl-2 indolizine avec un rendement de 65%. P.F. : 118°C (éthanol/éther diisopropylique).

En utilisant la même méthode que précédemment mais au départ du produit approprié, on a obtenu le composé suivant :
Oxalate de {{{[N-méthyl N-(diméthoxy-3,4 β-phénéthyl)amino]-3 propyl} oxy-4 phénylthio}}-1 isopropyl-2 indolizine (SR 33651)(Exemple 53). P.F. : 110°C.

EXEMPLE 55

Selon des techniques pharmaceutiques connues, on a préparé une capsule contenant les ingrédients suivants :

| Ingrédient | mg |
|---|---|
| Composé de l'invention | 100,0 |
| Amidons | 99,5 |
| Silice colloïdale | 0,5 |
| | 200,0 |

## Revendications

### Revendications pour les états contractants : CH, DE, FR, GB, IT, LI, LU, NL, SE

1. Dérivés d'indolizine correspondant à la formule générale :

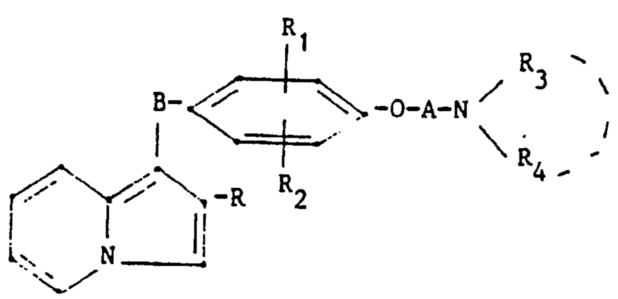

I

dans laquelle

B représente un groupement –S– ou $-SO_2-$

R représente l'hydrogène, un radical alkyle ayant de 1 à 8 atomes de carbone, linéaire ou ramifié, un radical

cycloalkyle comportant au maximum 6 atomes de carbone ou un groupement phényle non substitué ou substitué par un ou plusieurs substituants, identiques ou différents, sélectionnés parmi des atomes d'halogène et parmi des groupements alkyles inférieurs ayant de 1 à 4 atomes de carbone, alcoxy inférieurs ayant de 1 à 4 atomes de carbone ou nitro,

$R_1$ et $R_2$, qui sont identiques ou différents, représentent chacun l'hydrogène, le radical méthyle ou éthyle ou un halogène,

A représente un radical alkylène, linéaire ou ramifié, ayant de 2 à 5 atomes de carbone ou le radical hydroxy-2 propylène,

$R_3$ représente un radical alkyle, ayant de 1 à 8 atomes de carbone, linéaire ou ramifié, ou un radical de formule :

$$-Alk-R_5$$

dans laquelle Alk représente une liaison simple ou un radical alkylène, linéaire ou ramifié, ayant de 1 à 5 atomes de carbone et $R_5$ représente un radical pyridyle, phényle, méthylènedioxy-2,3 phényle, méthylènedioxy-3,4 phényle ou un groupement phényle substitué par un ou plusieurs substitants identiques ou différents sélectionnés parmi les atomes d'halogène, des groupements alkyles inférieurs ayant de 1 à 4 atomes de carbone ou des groupements alcoxy inférieurs, ayant de 1 à 4 atomes de carbone

$R_4$ représente l'hydrogène ou un radical alkyle, ayant de 1 à 8 atomes de carbone

ou $R_3$ et $R_4$, lorsqu'ils sont pris ensemble, représent un radical tétraméthylène-1,4 ; pentaméthylène-1,5 ; oxo-3 pentaméthylène-1,5 ; aza-3 pentaméthylène-1,5 ; méthylaza-3 pentaméthylène-1,5 ; phényl-aza-3 pentaméthylène-1,5 ou $-CH = CH-N = CH-$ de sorte que $R_3$ et $R_4$ pris avec l'atome d'azote auquel ils sont attachés, peuvent représenter notamment un radical pyrrolidinyle, pipéridinyle, morpholinyle, pipérazinyle, méthyl-4 pipérazinyle, phényl-4 pipérazinyle ou 1H-imidazolyle ainsi que les sels pharmaceutiquement acceptables de ces dérivés.

2. Dérivés d'indolizine correspondant à la formule générale :

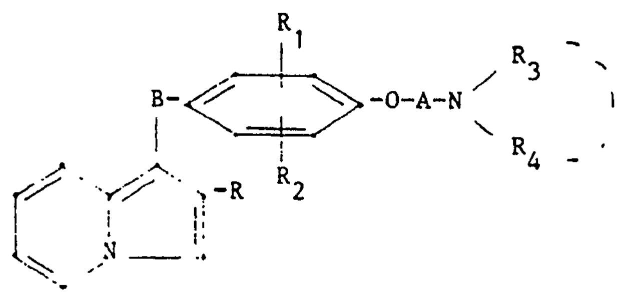

dans laquelle :

B représente un groupement –S– ou $-SO_2-$,

R représente l'hydrogène, un radical méthyle, éthyle, n-propyle, isopropyle, n-butyle, isobutyle, tertiobutyle, méthyl-1 propyle, n-pentyle, néopentyle, phényle, mono-fluoro-, mono-chloro- ou mono-bromo-phényle, di-fluoro-, di-chloro- ou di-bromo-phényle, mono-méthyl ou di-méthyl-phényle, mono-méthoxy- ou di-méthoxy-phényle, nitro-phényle ou méthyl-phényle substitué par un atome d'halogène,

$R_1$ et $R_2$, qui sont identiques ou différents, représentent chacun l'hydrogène, le chlore, le brome ou l'iode ou le radical méthyle,

A représente un radical éthylène-1,2 ; propylène-1,3 ; méthyl-2 propylène-1,3 ; tétraméthylène-1,4 ; pentaméthylène-1,5 ou hydroxy-2 propylène,

$R_3$ représente un radical méthyle, éthyle, n-propyle, isopropyle, n-butyle, isobutyle, tertiobutyle, méthyl-1 propyle, n-pentyle, n-hexyle, n-heptyle, n-octyle, phényle, benzyle, phénéthyle, méthoxyphénéthyle, diméthoxyphénéthyle, diméthylphénéthyle, diméthoxybenzyle, pyridyléthyle ou un radical phénéthyle substitué dans la partie aromatique par des radicaux méthyle et méthoxy,

$R_4$ représente l'hydrogène ou un radical méthyle, éthyle, n-propyle, isopropyle, n-butyle, isobutyle, tertiobutyle, n-pentyle, néopentyle, n-hexyle, n-heptyle ou n-octyle,

$R_3$ et $R_4$, pris ensemble représentent un radical tétraméthylène-1,4 ; pentaméthylène-1,5 ; oxa-3 pentaméthylène-1,5 ; aza-3 pentaméthylène-1,5 ; méthylaza-3 pentaméthylène-1,5 ; phénylaza-3 pentaméthylène-1,5 ou $-CH = CH-N = CH-$, ainsi que les sels pharmaceutiquement acceptables de ces dérivés.

3. Dérivés d'indolizine selon l'une des Revendications 1 ou 2, caractérisés en ce que B représente un grou-

pement $-SO_2-$.

4. Dérivés d'indolizine selon l'une des Revendications 1 ou 2, caractérisés en ce que $R_3$ représente un radical diméthoxy-3, phényle, diméthoxy-3,4 benzyle ou diméthoxy-3,4 phénéthyle.

5. Isopropyl-2 {{{[N-méthyl N-(diméthoxy-3,4 β-phénéthyl)amino]-3 propyl} oxy-4 benzènesulfonyl}}-1 indolizine et ses sels pharmaceutiquement acceptables.

6. Isopropyl-2[(di-n-butylamino-3 propyl)oxy-4 benzènesulfonyl]-1 indolizine et ses sels pharmaceutiquement acceptables.

7. Ethyl-2 {{{[N-méthyl N-(diméthoxy-3,4 β-phénéthyl)amino]-3 propyl} oxy-4 benzènesulfonyl}}-1 indolizine et ses sels pharmaceutiquement acceptables.

8. Ethyl-2 [(di-n-butylamino-3 propyl)oxy-4 benzènesulfonyl]-1 indolizine et ses sels pharmaceutiquement acceptables.

9. Dérivés d'indolizine selon une des Revendications 1 à 8, caractérisés en ce que le sel pharmaceutiquement acceptable est le chlorhydrate.

10. Procédé de préparation de dérivés d'indolizine selon la Revendication 1 dans laquelle A représente un radical alkylène ayant de 2 à 5 atomes de carbone et B représente un groupement $-S-$ ou $-SO_2-$, caractérisé en ce que l'on condense, en présence d'un accepteur d'acide, dans un solvant polaire ou non polaire et à une température comprise entre la température ambiante et la température de reflux, un dérivé d'(alcoxy-4 benzènesulfonyl)-1 indolizine de formule générale :

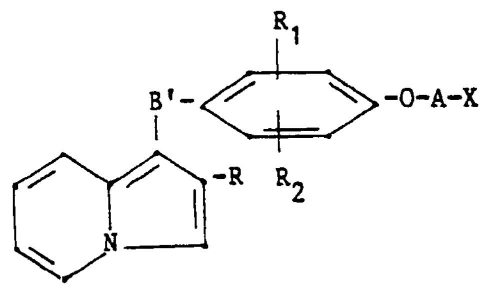

II

dans laquelle B′ représente un groupement $-S-$ ou $-SO_2-$, R, $R_1$ et $R_2$ ont la même signification que dans la Revendication 1, A a la même signification que précédemment et X représente un atome d'halogène, ou un groupement alkylsulfonyloxy ayant de 1 à 4 atomes de carbone ou arylsulfonyloxy ayant de 6 à 10 atomes de carbone, avec une amine de formule générale :

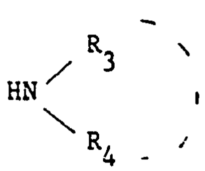

III

dans laquelle $R_3$ et $R_4$ ont la même signification que dans la Revendication 1 pour former le dérivé désiré d'indolizine que l'on fait réagir, si on le désire, avec un acide organique ou inorganique pour former un sel pharmaceutiquement acceptable de ce dérivé.

11. Procédé de préparation de dérivés d'indolizine selon la Revendication 1 dans laquelle A représente un radical alkylène ayant de 2 à 5 atomes de carbone et B représente un groupement $-S-$ ou $-SO_2-$, caractérisé en ce que l'on fait réagir, en présence d'un agent basique, une (hydroxy-4 benzènesulfonyl)-1 indolizine de formule générale :

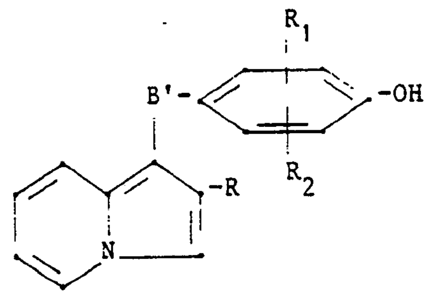

IV

dans laquelle B′ représente un groupement –S– ou –$SO_2$– et R, $R_1$ et $R_2$ ont la même signification que dans la Revendication 1 avec un composé de formule générale :

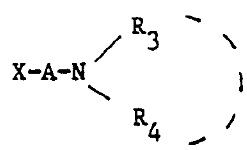

dans laquelle X représente un atome d'halogène ou un groupement alkylsulfonyle ayant de 1 à 4 atomes de carbone ou arylsulfonyle ayant de 6 à 10 atomes de carbone, A représente un radical alkylène ayant de 2 à 5 atomes de carbone et $R_3$ et $R_4$ ont la même valeur que dans la Revendication 1, la réaction ayant lieu au reflux et dans un solvant approprié pour obtenir le dérivé désiré d'indolizine que l'on peut, si on le désire, faire réagir avec un acide organique ou inorganique approprié pour former un sel pharmaceutiquement acceptable.

12. Procédé de préparation de dérivés d'indolizine selon la Revendication 1 dans laquelle A représente le radical hydroxy-2 propylène et B représente un groupement –S– ou –$SO_2$–, caractérisé en ce que l'on traite au reflux un dérivé oxyranylméthoxy de formule générale :

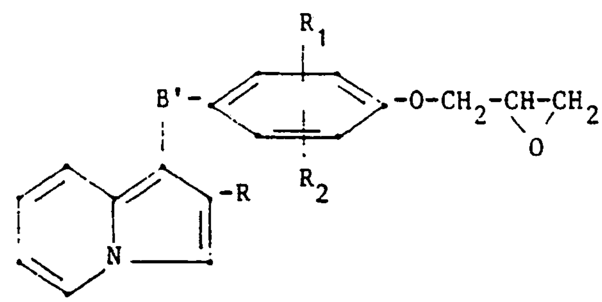

XIV

dans laquelle B′ représente un groupement –S– ou –$SO_2$– et R, $R_1$ et $R_2$ ont la même signification que dans la Revendication 1 avec une amine de formule générale :

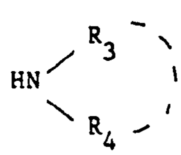

III

dans laquelle $R_3$ et $R_4$ ont la même signification que dans la Revendication 1 et ce, dans un solvant polaire ou dans un excès de ladite amine pour donner le dérivé désiré d'indolizine que l'on fait réagir, si on le désire, avec un acide organique ou inorganique pour former un sel pharmaceutiquement acceptable de ce dérivé.

13. Compositions pharmaceutiques ou vétérinaires contenant, comme principe actif, au moins un dérivé d'indolizine selon l'une des Revendications 1, 2 ou 9, en association avec un véhicule pharmaceutique ou un excipient approprié.

14. Compositions pharmaceutiques ou vétérinaires contenant, comme principe actif, au moins un dérivé d'indolizine selon l'une des Revendications 3 à 8, en association avec un véhicule pharmaceutique ou un excipient approprié.

15. Compositions pharmaceutiques ou vétérinaires selon l'une des Revendications 13 ou 14 pour le traitement de syndromes pathologiques du système cardiovasculaire contenant de 50 mg à 500 mg de principe actif.

16. Dérivés d'indolizine de formule générale :

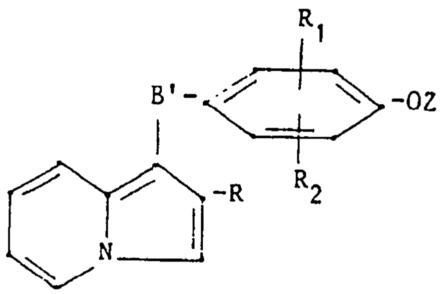

XV

dans laquelle

B' représente un groupement $-S-$ ou $-SO_2-$, R, $R_1$ et $R_2$ sont tels que définis à la Revendication 1

Z représente l'hydrogène, un radical oxyranylméthyle, alkylsulfonyle ayant de 1 à 4 atomes de carbone, arylsulfonyle ayant de 6 à 10 atomes de carbone ou un radical de formule :

$$-A-Z_1$$

dans laquelle A est tel que défini à la Revendication 1 et $Z_1$ représente un atome d'halogène le radical hydroxy ou un radical alkylsulfonyloxy ayant de 1 à 4 atomes de carbon ou arylsulfonyloxy ayant de 6 à 10 atomes de carbone.

17. Dérivés d'indolizine selon la Revendication 16, caractérisés en ce que Z représente un radical méthanesulfonyloxy, benzènesulfonyloxy, p-toluène sulfonyloxy et $Z_1$ représente un atome de brome, un radical méthanesulfonyloxy, benzènesulfonyloxy ou p-toluènesulfonyloxy.

18. Ethyl-2 [(bromo-3 propyl)oxy-4 benzènesulfonyl]-1 indolizine.

19. Isopropyl-2 [(bromo-3 propyl)oxy-4 benzènesulfonyl]-1 indolizine.

20. Ethyl-2 (tosyloxy-4 benzènesulfonyl)-1 indolizine.

21. Isopropyl-2 (tosyloxy-4 benzènesulfonyl)-1 indolizine.

22. Ethyl-2 (hydroxy-4 benzènesulfonyl)-1 indolizine.

23. Isopropyl-2 (hydroxy-4 benzènesulfonyl)-1 indolizine.

24. Procédé de préparation de dérivés d'indolizine selon la Revendication 16, caractérisé en ce que :

a) l'on cyclise au reflux, dans un solvant et en présence d'un agent basique, une picolylsulfone ou un picolylsulfure de formule générale :

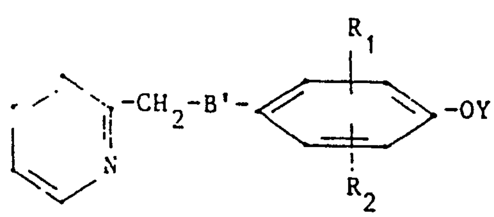

dans laquelle B', $R_1$ et $R_2$ ont la même signification que dans la Revendication 16 et Y représente un radical alkylsulfonyle ayant de 1 à 4 atomes de carbone ou un radical arylsulfonyle ayant de 6 à 10 atomes de carbone, avec une α-halogénocétone de formule générale :

$$R-\overset{O}{\overset{\|}{C}}-CH_2-Hal$$

dans laquelle R a la même signification que dans la Revendication 16 et Hal représente un atome d'halogène, pour obtenir les composés de formule XV dans laquelle Z représente un radical alkylsulfonyle ayant

de 1 à 4 atomes de carbone ou arylsulfonyle, ayant de 6 à 10 atomes de carbone.

b) l'on fait réagir un picolylsulfure de formule générale :

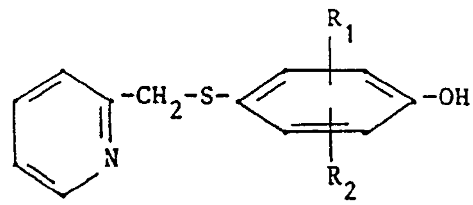

dans laquelle $R_1$ et $R_2$ ont la même signification que dans la Revendication 16, avec une α-halogénocétone de formule générale :

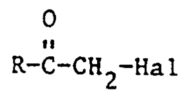

dans laquelle R et Hal ont la même signification que précédemment, pour obtenir un sel de pyridinium que l'on cyclise au reflux, dans un solvant et en présence d'un agent basique pour obtenir les composés de formule XV dans laquelle Z représente l'hydrogène et B' représente un groupement –S–,

c) l'on hydrolyse, au reflux et en milieu basique, un dérivé d'indolizine de formule XV dans laquelle Z représente un radical alkylsulfonyle ayant de 1 à 4 atomes de carbone ou arylsufonyle ayant de 6 à 10 atomes de carbone pour obtenir les composés de formules XV dans laquelle Z représente l'hydrogène,

d) l'on condense, au reflux, dans un solvant et en présence d'un agent basique, un dérivé d'indolizine de formule XV dans laquelle Z représente l'hydrogène, avec un dihalogénoalcane de formule générale :

Hal-A-Hal

dans laquelle A a la même signification que dans la Revendication 16 et Hal a la même signification que précédemment, pour obtenir les composés de formule XV dans laquelle Z représente un radical de formule $-A-Z_1$ dans laquelle $Z_1$ représente un atome d'halogène,

e) l'on condense dans un solvant et en présence d'un agent basique, un dérivé d'indolizine de formule XV dans laquelle Z représente l'hydrogène, avec un alcool halogéné de formule générale :

Hal-A-OH

dans laquelle A et Hal ont la même signification que précédemment pour obtenir les composés de formule XV dans laquelle Z représente un radical de formule $-A-Z_1$ dans laquelle $Z_1$ représente le groupement hydroxy,

f) l'on condense dans un solvant et en présence d'un accepteur d'acide, un dérivé d'indolizine de formule XV dans laquelle Z représente un radical de formule $-A-Z_1$ dans laquelle $Z_1$ représente le groupement hydroxy, avec un halogénure de formule générale :

Hal-Y

dans laquelle Hal et Y ont la même signification que précédemment pour obtenir un dérivé d'indolizine de formule XV dans laquelle Z représente un radical de formule $-A-Z_1$ dans laquelle $Z_1$ représente un groupement alkylsulfonyloxy ayant de 1 à 4 atomes de carbone ou arylsulfonyloxy, ayant de 6 à 10 atome de carbone,

g) l'on fait réagir au reflux, dans un solvant polaire et en présence d'un agent basique, un dérivé d'indolizine de formule XV dans laquelle Z représente l'hydrogène avec une épihalohydrine, pour obtenir les composés de formule XV dans laquelle Z représente un radical oxyranylméthyle.

25. Procédé selon l'une des Revendications 11 ou 24, caractérisé en ce que l'agent basique est un carbonate de métal alcalin, un hydroxyde de métal alcalin, un hydrure de métal alcalin ou un alcoolate de métal alcalin.

26. Utilisation des dérivés d'indolizine selon la Revendication 16 pour la préparation des dérivés d'indolizine selon l'une des Revendications 1 à 8.

**Revendications pour les états contractants : AT, ES, GR**

1. Procédé de préparation de dérivés d'indolizine correspondant à la formule générale :

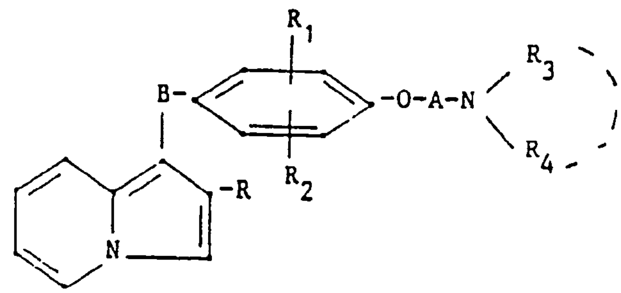

I

dans laquelle

A représente un radical alkylène ayant de 2 à 5 atomes de carbone,

B représente un groupement –S– ou $-SO_2-$,

R représente l'hydrogène, un radical alkyle ayant de 1 à 8 atomes de carbone, linéaire ou ramifié, un radical cycloalkyle comportant au maximum 6 atomes de carbone ou un groupement phényle non substitué ou substitué par un ou plusieurs substituants, identiques ou différents, sélectionnés parmi des atomes d'halogène et parmi des groupements alkyles inférieurs ayant de 1 à 4 atomes de carbone, alcoxy inférieurs ayant de 1 à 4 atomes de carbone ou nitro,

$R_1$ et $R_2$, qui sont identiques ou différents, représentent chacun l'hydrogène, le radical méthyle ou éthyle ou un halogène,

$R_3$ représente un radical alkyle ayant de 1 à 8 atomes de carbone, linéaire ou ramifié, ou un radical de formule :

$$-Alk-R_5$$

dans laquelle Alk représente une liaison simple ou un radical alkylène, linéaire ou ramifié, ayant de 1 à 5 atomes de carbone et $R_5$ représente un radical pyridyle, phényle, méthylènedioxy-2,3 phényle, méthylènedioxy-3,4 phényle ou un groupement phényle substitué par un ou plusieurs substituants identiques ou différents sélectionnés parmi des atomes d'halogène, des groupements alkyles inférieurs ayant de 1 à 4 atomes de carbone ou des groupements alcoxy inférieurs ayant de 1 à 4 atomes de carbone,

$R_4$ représente l'hydrogène ou un radical alkyle ayant de 1 à 8 atomes de carbone

ou $R_3$ et $R_4$, lorsqu'ils sont pris ensemble, représentent un radical tétraméthylène-1,4 ; pentaméthylène-1,5 ; oxo-3 pentaméthylène-1,5 ; aza-3 pentaméthylène-1,5 ; méthylaza-3 pentaméthylène-1,5 ; phényl-aza-3 pentaméthylène-1,5 ou –CH = CH-N = CH– de sorte que $R_3$ et $R_4$ pris avec l'atome d'azote auquel ils sont attachés peuvent représenter notamment un radical pyrrolidinyle, pipéridinyle, morpholinyle, pipérazinyle, méthyl-4 pipérazinyle, phényl-4 pipérazinyle ou 1H-imidazolyle, ainsi que les sels pharmaceutiquement acceptables de ces dérivés, caractérisé en ce que l'on condense, en présence d'un accepteur d'acide dans un solvant polaire ou non polaire et à une température comprise entre la température ambiante et la température de reflux, un dérivé d'(alcoxy-4 benzènesulfonyl)-1 indolizine de formule générale :

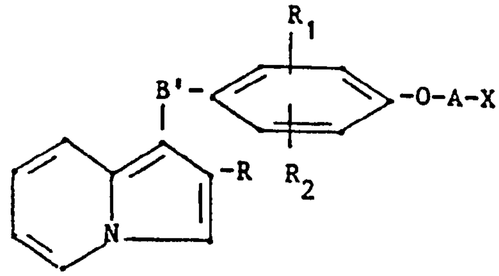

II

dans laquelle B′ représente un groupement –S– ou $-SO_2-$, R, $R_1$ et $R_2$ ont la signification que précédemment, A a la même signification que précédemment et X représente un atome d'halogène, ou un groupement alkylsulfonyloxy ayant de 1 à 4 atomes de carbone ou arylsulfonyloxy ayant de 6 à 10 atomes de carbone, avec

une amine de formule générale :

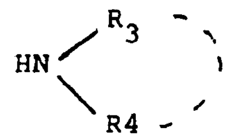

III

dans laquelle $R_3$ et $R_4$ ont la même signification que précédemment pour former le dérivé désiré d'indolizine que l'on fait réagir, si on le désire, avec un acide organique ou inorganique pour former un sel pharmaceutiquement acceptable de ce dérivé.

2. Procédé de préparation de dérivés d'indolizine correspondant à la formule générale :

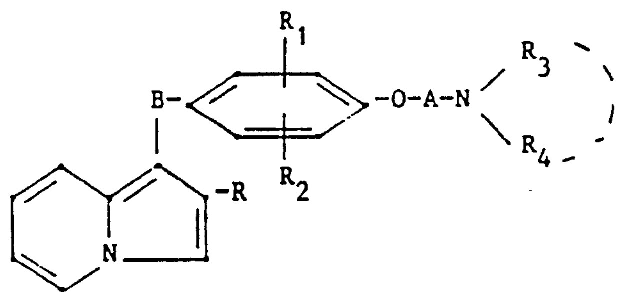

I

dans laquelle

A représente un radical alkylène ayant de 2 à 5 atomes de carbone,

B représente un groupement $-S-$ ou $-SO_2-$,

R représente l'hydrogène, un radical alkyle ayant de 1 à 8 atomes de carbone, linéaire ou ramifié, un radical cycloalkyle comportant au maximum 6 atomes de carbone ou un groupement phényle non substitué ou substitué par un ou plusieurs substituants, identiques ou différents, sélectionnés parmi des atomes d'halogène et parmi des groupements alkyles inférieurs ayant de 1 à 4 atomes de carbone, alcoxy inférieurs ayant de 1 à 4 atomes de carbone ou nitro,

$R_1$ et $R_2$, qui sont identiques ou différents, représentent chacun l'hydrogène, le radical méthyle ou éthyle ou un halogène,

$R_3$ représente un radical alkyle ayant de 1 à 8 atomes de carbone, linéaire ou ramifié, ou un radical de formule :

$$-Alk-R_5$$

dans laquelle Alk représente une liaison simple ou un radical alkylène, linéaire ou ramifié, ayant de 1 à 5 atomes de carbone et $R_5$ représente un radical pyridyle, phényle, méthylènedioxy-2,3 phényle, méthylènedioxy-3,4 phényle ou un groupement phényle substitué par un ou plusieurs substituants identiques ou différents sélectionnés parmi des atomes d'halogène, des groupements alkyles inférieurs ayant de 1 à 4 atomes de carbone ou des groupements alcoxy inférieurs ayant de 1 à 4 atomes de carbone,

$R_4$ représente l'hydrogène ou un radical alkyle ayant de 1 à 8 atomes de carbone

ou $R_3$ et $R_4$, lorsqu'ils sont pris ensemble, représentent un radical tétraméthylène-1,4 ; pentaméthylène-1,5 ; oxo-3 pentaméthylène-1,5 ; aza-3 pentaméthylène-1,5 ; méthylaza-3 pentaméthylène-1,5 ; phényl-aza-3 pentaméthylène-1,5 ou $-CH=CH-N=CH-$ de sorte que $R_3$ et $R_4$ pris avec l'atome d'azote auquel ils sont attachés peuvent représenter notamment un radical pyrrolidinyle, pipéridinyle, morpholinyle, pipérazinyle, méthyl-4 pipérazinyle, phényl-4 pipérazinyle ou 1H-imidazolyle, ainsi que les sels pharmaceutiquement acceptables de ces dérivés, caractérisé en ce que l'on fait réagir, en présence d'un agent basique, une (hydroxy-4 benzènesulfonyl)-1 indolizine de formule générale :

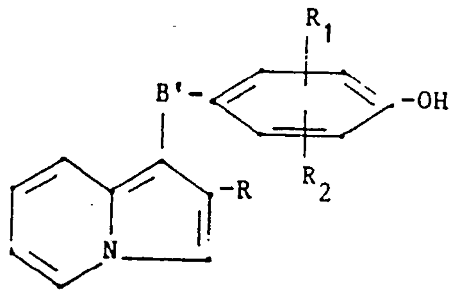

IV

dans laquelle B′ représente un groupement –S– ou –$SO_2$– et R, $R_1$ et $R_2$ ont la même signification que précédemment, avec un composé de formule générale :

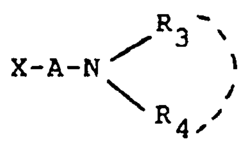

dans laquelle X représente un atome d'halogène ou un groupement alkylsulfonyle ayant de 1 à 4 atomes de carbone ou arylsulfonyle ayant de 6 à 10 atomes, A représente un radical alkylène ayant de 2 à 5 atoms de carbone et $R_3$ et $R_4$ ont la même valeur que précédemment, la réaction ayant lieu au reflux et dans un solvant approprié pour obtenir le dérivé désiré d'indolizine que l'on peut, si on le désire, faire réagir avec un acide organique ou inorganique approprié pour former un sel pharmaceutiquement acceptable.

3. Procédé de préparation de dérivés d'indolizine correspondant à la formule générale :

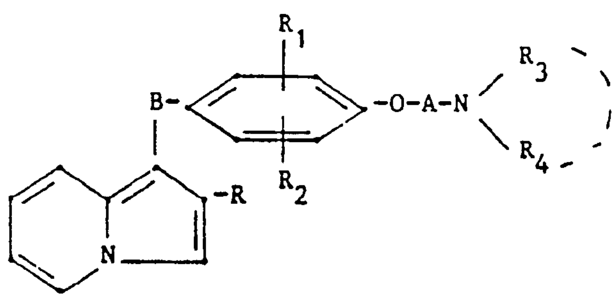

I

dans laquelle

A représente le radical hydroxy-2 propylène,

B représente un groupement –S– ou –$SO_2$–,

R représente l'hydrogène, un radical alkyle ayant de 1 à 8 atomes de carbone, linéaire ou ramifié, un radical cycloalkyle comportant au maximum 6 atomes de carbone ou un groupement phényle non substitué ou substitué par un ou plusieurs substituants, identiques ou différents, sélectionnés parmi des atomes d'halogène et parmi des groupements alkyles inférieurs ayant de 1 à 4 atomes de carbone, alcoxy inférieurs ayant de 1 à 4 atomes de carbone ou nitro,

$R_1$ et $R_2$, qui sont identiques ou différents, représentent chacun l'hydrogène, le radical méthyle ou éthyle ou un halogène,

$R_3$ représente un radical alkyle ayant de 1 à 8 atomes de carbone, linéaire ou ramifié, ou un radical de formule :

$$-Alk-R_5$$

dans laquelle Alk représente une liaison simple ou un radical alkylène, linéaire ou ramifié, ayant de 1 à 5 atomes de carbone et $R_5$ représente un radical pyridyle, phényle, méthylènedioxy-2,3 phényle, méthylènedioxy-3,4 phényle ou un groupement phényle substitué par un ou plusieurs substituants identiques ou différents sélectionnés parmi des atomes d'halogène, des groupements alkyles inférieurs ayant de 1 à 4 atomes de carbone ou des groupements alcoxy inférieurs ayant de 1 à 4 atomes de carbone,

$R_4$ représente l'hydrogène ou un radical alkyle ayant de 1 à 8 atomes de carbone

ou $R_3$ et $R_4$, lorsqu'ils sont pris ensemble, représentent un radical tétraméthylène-1,4 ; pentaméthylène-1,5 ; oxo-3 pentaméthylène-1,5 ; aza-3 pentaméthylène-1,5 ; méthylaza-3 pentaméthylène-1,5 ; phényl-aza-3 pentaméthylène-1,5 ou $-CH = CH-N =CH-$ de sorte que $R_3$ et $R_4$ pris avec l'atome d'azote auquel ils sont attachés peuvent représenter notamment un radical pyrrolidinyle, pipéridinyle, morpholinyle, pipérazinyle, méthyl-4 pipérazinyle, phényl-4 pipérazinyle ou 1H-imidazolyle, ainsi que les sels pharmaceutiquement acceptables de ces dérivés, caractérisé en ce que l'on traite au reflux un dérivé oxyranylméthoxy de formule générale :

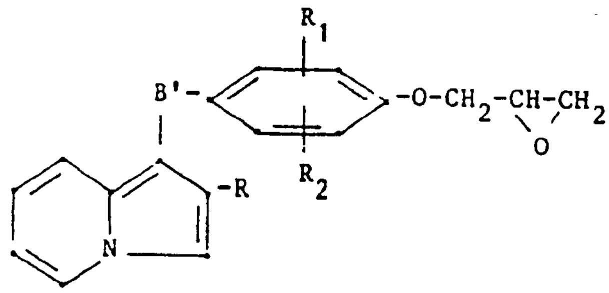

XIV

dans laquelle B' représente un groupement $-S-$ ou $-SO_2-$ et R, $R_1$ et $R_2$ ont la même signification que précédemment avec une amine de formule générale :

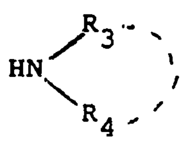

III

dans laquelle $R_3$ et $R_4$ ont la même signification que précédemment et ce, dans un solvant polaire ou dans un excès de ladite amine pour donner le dérivé désiré d'indolizine que l'on fait réagir, si on le désire, avec un acide organique ou inorganique pour former un sel pharmaceutiquement acceptable de ce dérivé.

4. Procédé selon l'une quelconque des revendications précédentes, caractérisé en ce que l'on prépare des dérivés d'indolizine correspondant à la formule générale :

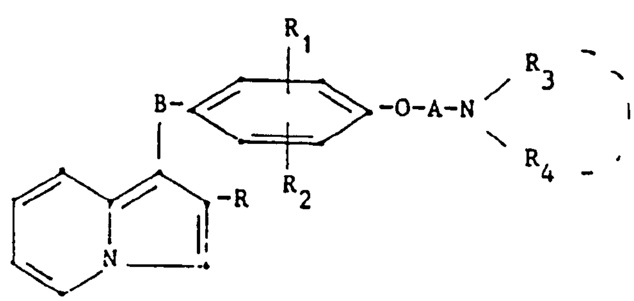

dans laquelle

B représente un groupement $-S-$ ou $-SO_2-$,

R représente l'hydrogène, un radical méthyle, éthyle, n-propyle, isopropyle, n-butyle, isobutyle, tertiobutyle, méthyl-1 propyle, n-pentyle, néopentyle, phényle, mono-fluoro-, mono-chloro- ou mono-bromophényle, di-fluoro-, di-chloro ou di-bromo-phényle, mono-méthyl ou di-méthyl-phényle, mono-méthoxy- ou di-méthoxy-phényle, nitro-phényle ou méthyl-phényle substitué par un atome d'halogène,

$R_1$ et $R_2$, qui sont identiques ou différents, représentent chacun l'hydrogène, le chlore, le brome ou l'iode ou le radical méthyle,

A représente un radical éthylène-1,2 ; propylène-1,3 ; méthyl-2 propylène-1,3 ; tétraméthylène-1,4 ; pentaméthylène-1,5 ou hydroxy-2 propylène,

$R_3$ représente un radical méthyle, éthyle, n-propyle, isopropyle, n-butyle, isobutyle, tertiobutyle, méthyl-1 propyle, n-pentyle, n-hexyle, n-heptyle, n-octyle, phényle, benzyle, phénéthyle, méthoxyphénéthyle, diméthoxyphénéthyle, diméthylphénéthyle, diméthoxybenzyle, pyridyléthyle ou un radical phénéthyle substitué

dans la partie aromatique par des radicaux méthyle et méthoxy,

$R_4$ représente l'hydrogène ou un radical méthyle, éthyle, n-propyle, isopropyle, n-butyle, isobutyle, tertiobutylle, n-pentyle, néopentyle, n-hexyle, n-heptyle ou n-octyle,

$R_3$ et $R_4$ pris ensemble représentent un radical tétraméthylène-1,4 ; pentaméthylène-1,5 ; oxa-3 pentaméthylène-1,5 ; aza-3 pentaméthylène-1,5 ; méthylaza-3 pentaméthylène-1,5 ; phénylaza-3 pentaméthylène-1,5 ou –CH = CH-N = CH- ainsi que les sels pharmaceutiquement acceptables de ces dérivés.

5. Procédé selon l'une quelconque des revendications 1 à 4, caractérisé en ce que l'on prépare des dérivés d'indolizine dans lesquels B représente un groupement $-SO_2-$.

6. Procédé selon l'une quelconque des revendications 1 à 4, caractérisé en ce que l'on prépare des dérivés d'indolizine dans lesquels $R_3$ représente un radical diméthoxy-3,4 phényle, diméthoxy-3,4 benzyle ou diméthoxy-3,4 phénéthyle.

7 - Procédé selon la revendication 1 ou 2, caractérisé en ce que l'on prépare l'isopropyl-2 [{[N-méthyl N-(diméthoxy-3,4 β-phénéthyl)amino]-3 propyl} oxy-4 benzènesulfonyl]-1 indolizine et ses sels pharmaceutiquement acceptables.

8. Procédé selon la revendication 1 ou 2, caractérisé en ce que l'on prépare l'isopropyl-2 [(di-n-butylamino-3 propyl)oxy-4 benzènesulfonyl]-1 indolizine et ses sels pharmaceutiquement acceptables.

9. Procédé selon la revendication 1 ou 2, caractérisé en ce que l'on prépare l'éthyl-2 [{[N-méthyl -(diméthoxy-3,4 β-phénéthylamino]-3 propyl} oxy-4 benzènesulfonyl]-1 indolizine et ses sels pharmaceutiquement acceptables.

10. Procédé selon la revendication 1 ou 2, caractérisé en ce que l'on prépare l'éthyl-2 [(di-n-butylamino-3 propyl)oxy-4 benzènesulfonyl]-1 indolizine et ses sels pharmaceutiquement acceptables.

11. Procédé selon l'une quelconque des revendications précédentes, caractérisé en ce que l'on prépare des dérivés d'indolizine dans lesquels le sel pharmaceutiquement acceptable est le chlorhydrate.

12. Procédé de préparation de compositions pharmaceutiques ou vétérinaires, caractérisé en ce que l'on associe au moins un dérivé d'indolizine préparé selon l'une des revendications précédentes avec un véhicule pharmaceutique ou un excipient approprié.

13. Procédé de préparation de compositions pharmaceutiques ou vétérinaires selon la revendication 12 pour le traitement de syndromes pathologiques du système cardiovasculaire contenant de 50 mg à 500 mg de principe actif.

14. Procédé de préparation de dérivés d'indolizine de formule générale :

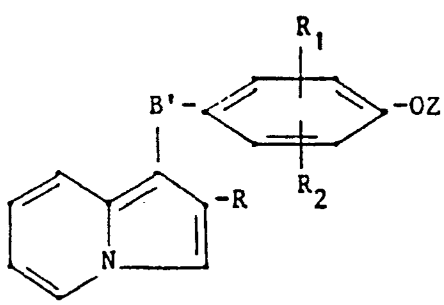

XV

dans laquelle

B' représente un groupement –S– ou $-SO_2-$,

R, $R_1$ et $R_2$ sont tels que définis à la revendication 1,

Z représente l'hydrogène, un radical oxyranylméthyle, alkylsulfonyle ayant de 1 à 4 atomes de carbone, arylsulfonyle ayant de 6 à 10 atomes de carbone ou un radical de formule :

$$-A\text{-}Z_1$$

dans laquelle A est tel que défini à la revendication 1 et $Z_1$ représente un atome d'halogène le radical hydroxy ou un radical alkylsulfonyloxy ayant de 1 à 4 atomes de carbone ou arylsulfonyloxy ayant de 6 à 10 atomes de carbone, caractérisé en ce que :

a) l'on cyclise au reflux, dans un solvant et en présence d'un agent basique, une picolylsulfone ou un picolylsulfure de formule générale :

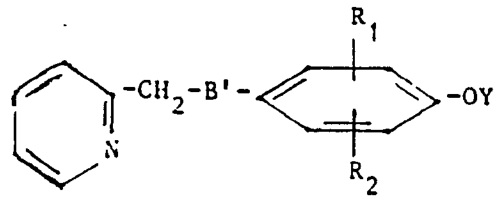

dans laquelle B′, $R_1$ et $R_2$ ont la même signification que précédemment et Y représente un radical alkylsulfonyle ayant de 1 à 4 atomes de carbone ou un radical arylsulfonyle ayant de 6 à 10 atomes de carbone, avec une α-halogénocétone de formule générale :

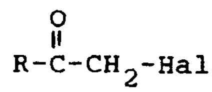

dans laquelle R a la même signification que précédemment et Hal représente un atome d'halogène, pour obtenir les composés de formule XV dans laquelle Z représente un radical alkylsulfonyle ayant de 1 à 4 atomes de carbone ou arylsulfonyle ayant de 6 à 10 atomes de carbone,
b) l'on fait réagir un picolylsulfure de formule générale :

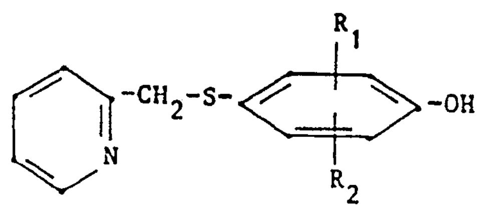

dans laquelle $R_1$ et $R_2$ ont la même signification que précédemment avec une α-halogénocétone de formule générale :

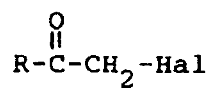

dans laquelle R et Hal ont la même signification que précédemment, pour obtenir un sel de pyridinium que l'on cyclise au reflux, dans un solvant et en présence d'un agent basique pour obtenir les composés de formule XV dans laquelle Z représente l'hydrogène et B′ représente un groupement –S–,
c) l'on hydrolyse, au reflux et en milieu basique, un dérivé d'indolizine de formule XV dans laquelle Z représente un radical alkylsulfonyle ayant de 1 à 4 atomes de carbone ou arylsulfonyle ayant de 6 à 10 atomes de carbone pour obtenir les composés de formule XV dans laquelle Z représente l'hydrogène,
d) l'on condense, au reflux, dans un solvant et en présence d'un agent basique, un dérivé d'indolizine de formule XV dans laquelle Z représente l'hydrogène, avec un dihalogénoalcane de formule générale :

Hal-A-Hal

dans laquelle A a la même signification que précédemment et Hal a la même signification que précédemment, pour obtenir les composés de formule XV dans laquelle Z représente un radical de formule $-A-Z_1$ dans laquelle $Z_1$ représente un atome d'halogène,
e) l'on condense dans un solvant et en présence d'un agent basique, un dérivé d'indolizine de formule XV dans laquelle Z représente l'hydrogène, avec un alcool halogéné de formule générale :

Hal-A-Oh

dans laquelle A et Hal ont la même signification que précédemment pour obtenir les composés de formule

XV dans laquelle Z représente un radical de formule $-A-Z_1$ dans laquelle $Z_1$ représente le groupement hydroxy,

f) l'on condense dans un solvant et en présence d'un accepteur d'acide, un dérivé d'indolizine de formule XVdans laquelle Z représente un radical de formule $-A-Z_1$ dans laquelle $Z_1$ représente le groupement hydroxy, avec un halogénure de formule générale :

Hal-Y

dans laquelle Hal et Y ont la même signification que précédemment pour obtenir un dérivé d'indolizine de formule XV dans laquelle Z représente un radical de formule $-A-Z_1$ dans laquelle $Z_1$ représente un groupement alkylsulfonyloxy ayant de 1 à 4 atomes de carbone ou arylsulfonyloxy, ayant de 6 à 10 atomes de carbone,

g) l'on fait réagir au reflux, dans un solvant polaire et en présence d'un agent basique, un dérivé d'indolizine de formule XV dans laquelle Z représente l'hydrogène avec une épihalohydrine, pour obtenir les composés de formule XV dans laquelle Z représente un radical oxyranylméthyle.

15. Procédé selon l'une des revendications 2 ou 16, caractérisé en ce que l'agent basique est un carbonate de métal alcalin, un hydroxyde de métal alcalin, un hydrure de métal alcalin ou un alcoolate de métal alcalin.

16. Procédé selon la revendication 14, caractérisé en ce que l'on prépare des dérivés d'indolizine dans lesquels Z représente un radical méthanesulfonyloxy, benzènesulfonyloxy, p-toluènesulfonyloxy et $Z_1$ représente un atome de brome, un radical méthanesulfonyloxy, benzènesulfonyloxy ou p-toluènesulfonyloxy.

17. Procédé selon la revendication 14, caractérisé en ce que l'on prépare l'éthyl-2 [(bromo-3 propyl)oxy-4 benzènesulfonyl]-1 indolizine.

18. Procédé selon la revendication 14, caractérisé en ce que l'on prépare l'isopropyl-2 [(bromo-3 propyl)oxy-4 benzènesulfonyl]-1 indolizine.

19. Procédé selon la revendication 14, caractérisé en ce que l'on prépare l'éthyl-2 (tosyloxy-4 benzènesulfonyl)-1 indolizine.

20. Procédé selon la revendication 14, caractérisé en ce que l'on prépare l'isopropyl-2 (tosyloxy-4 benzènesulfonyl)-1 indolizine.

21. Procédé selon la revendication 14, caractérisé en ce que l'on prépare l'éthyl-2 (hydroxy-4 benzènesulfonyl)-1 indolizine.

22. Procédé selon la revendication 14, caractérisé en ce que l'on prépare l'isopropyl-2 (hydroxy-4 benzènesulfonyl)-1 indolizine.

## Ansprüche

### Patentansprüche für die Vertragsstaaten CH, DE, FR, GB, IT, LI, LU, NL, SE

1. Indolizinderivate der allgemeinen Formel I

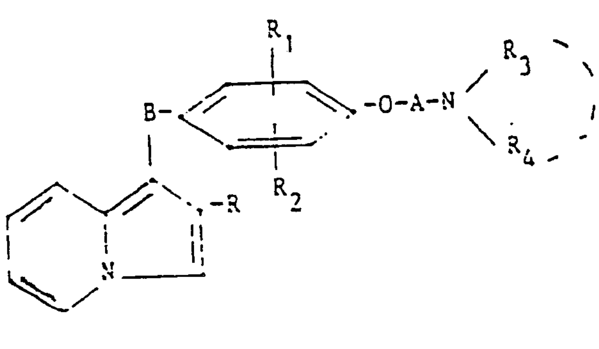

(I),

in der bedeuten :

B eine Gruppe –S– oder $-SO_2-$,

R Wasserstoff, eine geradkettige oder verzweigte Alkylgruppe mit 1 bis 8 Kohlenstoffatomen, eine Cycloalkylgruppe mit maximal 6 Kohlenstoffatomen oder eine Phenylgruppe, die gegebenenfalls mit einem oder mehreren gleichen oder verschiedenen Substituenten substituiert ist, die ausgewählt sind unter Halogenatomen, niederen Alkylgruppen mit 1 bis 4 Kohlenstoffatomen, niederen Alkoxygruppen mit 1 bis 4 Kohlenstoff-

atomen und Nitrogruppen,

$R_1$ und $R_2$, die gleich oder verschieden sind, jeweils Wasserstoff, Methyl oder Ethyl oder ein Halogen,

A eine geradkettige oder verzweigte Alkylengruppe mit 2 bis 5 Kohlenstoffatomen oder die Gruppe 2-Hydroxypropylen,

$R_3$ eine geradkettige oder verzweigte Alkylgruppe mit 1 bis 8 Kohlenstoffatomen oder eine Gruppe der Formel

$$-Alk-R_5,$$

in der Alk eine Einfachbindung oder eine geradkettige oder verzweigte Alkylengruppe mit 1 bis 5 Kohlenstoffatomen und $R_5$ Pyridyl, Phenyl, 2,3-Methylendioxyphenyl, 3,4-Methylendioxyphenyl oder eine Phenylgruppe bedeuten, die mit einem oder mehreren gleichen oder verschiedenen, unter Halogenatomen, niederen Alkylgruppen mit 1 bis 4 Kohlenstoffatomen oder niederen Alkoxygruppen mit 1 bis 4 Kohlenstoffatomen ausgewählten Substituenten substituiert ist, und

$R_4$ Wasserstoff oder eine Alkylgruppe mit 1 bis 8 Kohlenstoffatomen

oder $R_3$ und $R_4$ zusammen 1,4-Tetramethylen, 1,5-Pentamethylen, 3-Oxo-1,5-pentamethylen, 3-Aza-1,5-pentamethylen, 3-Methylaza-1,5-pentamethylen, 3-Phenylaza-1,5-pentamethylen oder $-CH = CH-N = CH-$, derart, daß $R_3$ und $R_4$ zusammen mit dem Stickstoffatom, an dem sie gebunden sind, insbesondere Pyrrolidinyl, Piperidinyl, Morpholinyl, Piperazinyl, 4-Methylpiperazinyl, 4-Phenylpiperazinyl oder 1H-Imidazolyl bedeuten können, sowie die pharmazeutisch akzeptablen Salze dieser Derivate.

2. Indolizinderivate der allgemeinen Formel

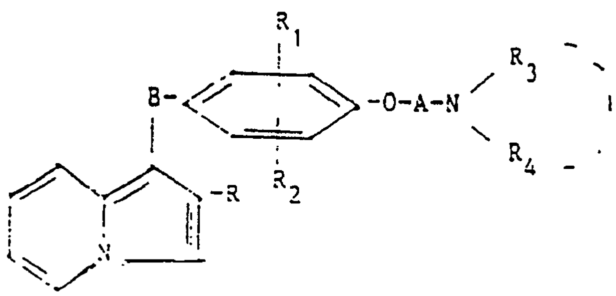

in der bedeuten :

B eine Gruppe –S– oder –SO–,

R Wasserstoff, Methyl, Ethyl, n-Propyl, Isopropyl, n-Butyl, Isobutyl, tert.-Butyl, 1- Methylpropyl, n-Pentyl, Neopentyl, Phernyl, Monofluor-, Monochlor- oder Monobromphenyl, Difluor-, Dichlor- oder Dibromphenyl, Monomethyl- oder Dimethylphenyl, Monomethoxy- oder Dimethoxyphenyl, Nitrophenyl oder mit einem Halogenatom substituiertes Methylphenyl,

$R_1$ und $R_2$, die gleich oder verschieden sind, jeweils Wasserstoff, Chlor, Brom oder Jod oder Methyl,

A 1,2-Ethylen, 1,3-Propylen, 2-Methyl-1,3-propylen, 1,4-Tetramethylen, 1,5-Pentamethylen oder 2-Hydroxypropylen,

$R_3$ Methyl, Ethyl, n-Propyl, Isopropyl, n-Butyl, Isobutyl, tert.-Butyl, 1-Methylpropyl, n-Pentyl, n-Hexyl, n-Heptyl, n-Octyl, Phenyl, Benzyl, Phenethyl, Methoxyphenethyl, Dimethoxyphenethyl, Dimethylphenethyl, Dimethoxybenzyl, Pyridylethyl oder eine im aromatischen Teil mit Methyl und Methoxy substituierte Phenethylgruppe und

$R_4$ Wasserstoff oder Methyl, Ethyl, n-Propyl, Isopropyl, n-Butyl, Isobutyl, tert.-Butyl, n-Pentyl, Neopentyl, n-Hexyl-, n-Heptyl oder n-Octyl oder

$R_3$ und $R_4$ zusammen 1,4-Tetramethylen, 1,5-Pentamethylen, 3-Oxa-1,5-pentamethylen, 3-Aza-1,5-pentamethylen, 3-Methylaza-1,5-pentamethylen, 3-Phenylaza-1,5-pentarmethylen oder $-CH = CH-N = CH-$, sowie die pharmazeutisch akzeptablen Salze dieser Derivate.

3. Indolizinderivate nach Anspruch 1 oder 2, dadurch gekennzeichnet, daß B eine Gruppe $-SO_2-$ bedeutet.

4. Indolizinderivate nach Anspruch 1 oder 2, dadurch gekennzeichnet, daß $R_3$ 3,4-Dimethoxy, Phenyl, 3,4-Dimethoxybenzyl oder 3,4-Dimethoxyphenethyl bedeutet.

5. 2-Isopropyl-1-[4-(3-(N-methyl-N-(3,4-dimethoxy-β-phenethyl)-amino)-propyl)-oxy-benzolsulfonyl]-indolizin und seine pharmazeutisch akzeptablen Salze.

6. 2-Isopropyl-1-[4-(3-di-n-butylaminopropyl)-oxy-benzolsulfonyl]-indolizin und seine pharmazeutisch akzeptablen Salze.

7. 2-Ethyl-1- [4-(3-(N-methyl-N-(3,4-dimethoxy-β-phenethyl)-amino)-propyl)-oxy-benzolsulfonyl]-indolizin und seine pharmazeutisch akzeptablen Salze.

8. 2-Ethyl-1-[4-(3-(di-n-butylaminopropyl)-oxy)-benzolsulfonyl]-indolizin und seine pharmazeutisch akzeptablen Salze.

9. Indolizinderivate nach einem der Ansprüche 1 bis 8, dadurch gekennzeichnet, daß das pharmazeutisch akzeptable Salz das Hydrochlorid ist.

10. Verfahren zur Herstellung der Indolizinderivate nach Anspruch 1, in der A eine Alkylengruppe mit 2 bis 5 Kohlenstoffatomen und B eine Gruppe $-S-$ oder $-SO_2-$ bedeuten, gekennzeichnet durch Kondensation eines 1-(4-Alkoxybenzolsulfonyl)-indolizinderivats der allgemeinen Formel II

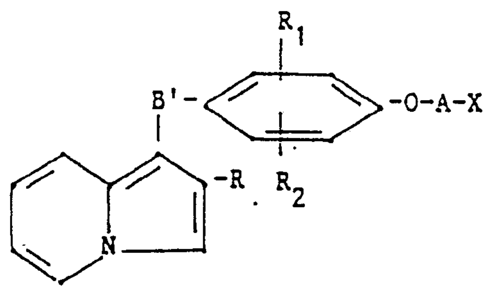

(II),

in der bedeuten :

B' eine Gruppe $-S-$ oder $-SO_2-$,

R, $R_1$ und $R_2$ dasselbe wie in Anspruch 1,

A dasselbe wie oben und

X ein Halogenatom oder eine Alkylsulfonyloxygruppe mit 1 bis 4 Kohlenstoffatomen oder eine Arylsulfonyloxygruppe mit 6 bis 10 Kohlenstoffatomen, mit einem Amin der allgemeinen Formel III

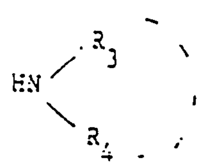

(III),

in der $R_3$ und $R_4$ die gleiche Bedeutung wie in Anspruch 1 haben, in Gegenwart eines Säureakzeptors in einem polaren oder unpolaren Lösungsmittel bei einer Temperatur zwischen Umgebungstemperatur und der Rückflußtemperatur zum gewünschten Indolizinderivat, das gewünschtenfalls mit einer organischen oder anorganischen Säure zu einem pharmazeutisch akzeptablen Salz dieses Derivats umgesetzt wird.

11. Verfahren zur Herstellung der Indolizinderivate nach Anspruch 1, wobei A eine Alkylengruppe mit 2 bis 5 Kohlenstoffatomen und B eine Gruppe $-S-$ oder $-SO_2-$ bedeuten, gekennzeichnet durch Umsetzung eines 1-(4-Hydroxybenzolsulfonyl)-indolizins der allgemeinen Formel IV

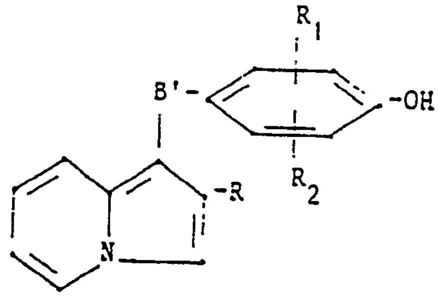

(IV),

in der bedeuten :

B' eine Gruppe $-S-$ oder $-SO_2-$ und

R, $R_1$ und $R_2$ dasselbe wie in Anspruch 1, mit einer Verbindung der allgemeinen Formel

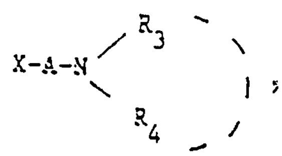

in der bedeuten :

X ein Halogenatom oder eine Alkylsulfonylgruppe mit 1 bis 4 Kohlenstoffatomen oder eine Arylsulfonylgruppe mit 6 bis 10 Kohlenstoffatomen,

A eine Alkylengruppe mit 2 bis 5 Kohlenstoffatomen und $R_3$ und $R_4$ dasselbe wie in Anspruch 1, in Gegenwart einer Base am Rückfluß und in einem geeigneten Lösungsmittel zum gewünschten Indolizinderivat, das gewünschtenfalls mit einer geeigneten organischen oder anorganischen Säure zu einem pharmazeutisch akzeptablen Salz umgesetzt wird.

12. Verfahren zur Herstellung der Indolizinderivate nach Anspruch 1, wobei A die Gruppe 2-Hydroxypropylen und B eine Gruppe –S– oder $-SO_2-$ bedeuten, gekennzeichnet durch Umsetzung eines Oxyranylmethoxyderivats der allgemeinen Formel XIV

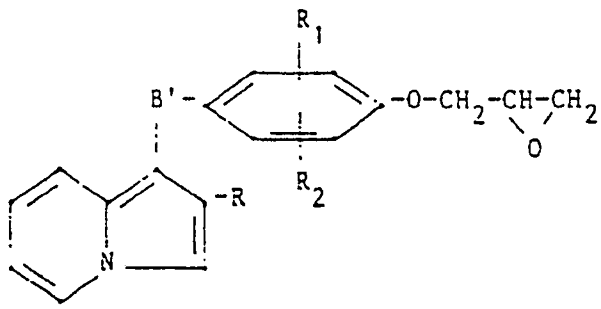

(XIV),

in der bedeuten :

B' eine Gruppe –S– oder $-SO_2-$ und

R, $R_1$ und $R_2$ dasselbe wie in Anspruch 1, mit einem Amin der allgemeinen Formel III

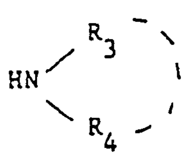

(III),

in der $R_3$ und $R_4$ dasselbe wie in Anspruch 1 bedeuten, am Rückfluß in einem polaren Lösungsmittel oder in einem Überschuß des Amins zum gewünschten Indolizinderivat, das, gewünschtenfalls, mit einer organischen oder anorganischen Säure, zu einem pharmazeutisch akzeptablen Salz des Derivats umgesetzt wird.

13. Pharmazeutische oder veterinärmedizinische Zusammensetzungen, die als Wirkstoff mindestens ein Indolizinderivat nach einem der Ansprüche 1, 2 oder 9 in Verbindung mit einem pharmazeutischen Träger oder einem geeigneten Excipiens enthalten.

14. Pharmazeutische oder veterinärmedizinische Zusammensetzungen, die als Wirkstoff mindestens ein Indolizinderivat nach eineem der Ansprüche 3 bis 8 in Verbindung mit einem pharmazeutischen Träger oder einem geeigneten Excipiens enthalten.

15. Pharmazeutische oder veterinärmedizinische Zusammensetzungen nach Anspruch 13 oder 14 zur Behandlung pathologischer Syndrome des cardiovasculären Systems, die 50 bis 500 mg Wirkstoff enthalten.

16. Indolizinderivate der allgemeinen Formel XV

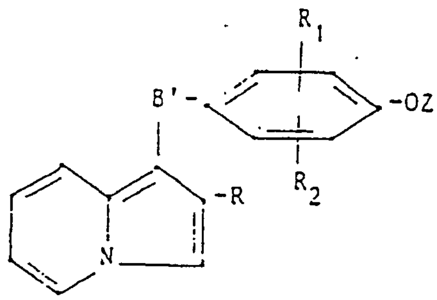

(XV) ,

in der bedeuten :

B′ eine Gruppe –S– oder $-SO_2-$,

R, $R_1$ und $R_2$ dasselbe wie in Anspruch 1 und

Z Wasserstoff, eine Oxyranylmethylgruppe, eine Alkylsulfonylgruppe mit 1 bis 4 Kohlenstoffatomen, eine Arylsulfonylgruppe mit 6 bis 10 Kohlenstoffatomen oder eine Gruppe der Formel

$$-A-Z_1,$$

in der A dasselbe wie in Anspruch 1 und

$Z_1$ ein Halogenatom, eine Hydroxygruppe, eine Alkylsulfonylgruppe mit 1 bis 4 Kohlenstoffatomen oder eine Arylsulfonyloxygruppe mit 6 bis 10 Kohlenstoffatomen bedeuten.

17. Indolizinderivate nach Anspruch 16, dadurch gekennzeichnet, daß Z Methansulfonyloxy, Benzolsulfonyloxy oder p-Toluolsulfonyloxy und $Z_1$ ein Bromatom, Methansulfonyloxy, Benzolsulfonyloxy oder p-Toluolsulfonyloxy bedeuten.

18. 2-Ethyl-1-[4-(3-brompropyl)-oxy-benzolsulfonyl]-indolizin.

19. 2-Isopropyl-1-[4-(3-brompropyl)-oxy-benzolsulfonyl]-indolizin.

20. 2-Ethyl-1-(4-tosyloxybenzolsulfonyl)-indolizin.

21. 2-Isopropyl-1-(4-tosyloxybenzolsulfonyl)-indolizin.

22. 2-Ethyl-1-(4-hydroxybenzolfulfonyl)-indolizin.

23. 2-Isopropyl-1-(4-hydroxybenzolsulfonyl)-indolizin.

24. Verfahren zur Herstellung von Indolizinderivaten nach Anspruch 16, gekennzeichnet durch

a) Cyclisierung eines Picolylsulfons oder eines Picolylsulfids der allgemeinen Formel

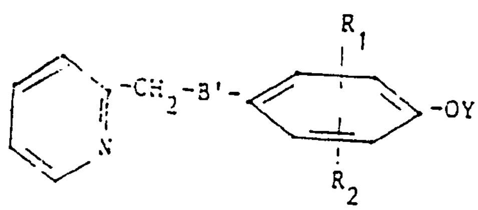

,

in der B′, $R_1$ und $R_2$ die gleiche Bedeutung wie in Anspruch 16 haben und Y eine Alkylsulfonylgruppe mit 1 bis 4 Kohlenstoffatomen oder eine Arylsulfonylgruppe mit 6 bis 10 Kohlenstoffatomen bedeutet, am Rückfluß in einem Lösungsmittel und in Gegenwart eines basischen Mittels mit einem α -Halogenketon der allgemeinen formel

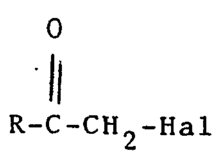

,

in der R die gleiche Bedeutung wie in Anspruch 16 hat und Hal ein Halogenatom bedeutet, zur Verbindung der Formel XV, in der Z eine Alkylsufonylgruppe mit 1 bis 4 Kohlenstoffatomen oder eine Arylsulfonylgruppe mit 6 bis 10 Kohlenstoffatomen bedeuten ;

b) Umsetzung eines Picolylsulfids der allgemeinen Formel

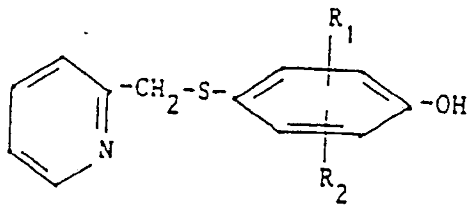

,

in der $R_1$ und $R_2$ die gleiche Bedeutung wie in Anspruch 16 haben, mit einem α-Halogenketon der allgemeinen Formel

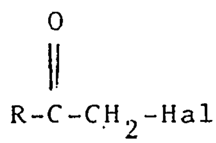

,

in der R und Hal dasselbe wie oben bedeuten, zum Pyridiniumsalz, das am Rückfluß in einem Lösungsmittel und in Gegenwart eines basischen Mittels zur Verbindung der Formel XV cyclisiert wird, in der Z Wasserstoff und B′ eine Gruppe –S– bedeuten ;

c) Hydrolyse eines Indolizinderivats der Formel XV, in der Z eine Alkylsulfonylgruppe mit 1 bis 4 Kohlenstoffatomen oder eine Arylsulfonylgrüppe mit 6 bis 10 Kohlenstoffatomen bedeutet, am Rückfluß und in einem basischen Medium zur Verbindung der Formel XV, in der Z Wasserstoff bedeutet ;

d) Kondensation eines Indolizinderivats der Formel XV, in der Z Wasserstoff bedeutet, mit einem Dihalogenalkan der allgemeinen Formel

Hal-A-Hal,

in der A dasselbe wie in Anspruch 16 und Hal dasselbe wie oben bedeuten, am Rückfluß in einem Lösungsmittel und in Gegenwart eines basischen Mittels zur Verbindung der Formel XV, in der Z eine Gruppe der Formel $-A-Z_1$ bedeutet, in der $Z_1$ ein Halogenatom darstellt ;

e) Kondensation eines Indolizinderivats der Formel XV, in der Z Wasserstoff bedeutet, mit einem halogenierten Alkohol der allgemeinen Formel

Hal-A-OH,

in der A und Hal dasselbe wie oben bedeuten, in einem Lösungsmittel in Gegenwart eines basischen Mittels zur Verbindung der Formel XV, in der Z eine Gruppe der Formel $-A-Z_1$ bedeutet, in der $Z_1$ eine Hydroxygruppe darstellt ;

f) Kondensation eines Indolizinderivats der Formel XV, in der Z eine Gruppe der Formel $-A-Z_1$ bedeutet, in der $Z_1$ eine Hydroxygruppe darstellt, mit einem Halogenid der allgemeinen Formel

Hal-Y,

in der Hal und Y dasselbe wie ober bedeuten, in einem Lösungsmittel und in Gegenwart eines Säureakzeptors zum Indolizinderivat der Formel $-A-Z_1$, in der $Z_1$ eine Alkylsulfonyloxygruppe mit 1 bis 4 Kohlenstoffatomen oder eine Arylsulfonyloxygruppe mit 6 bis 10 Kohlenstoffatomen bedeutet ;

g) Umsetzung eines Indolizinderivats der Formel XV, in der Z Wasserstoff bedeutet, mit einem Epihalohydrin am Rückfluß in einem polaren Lösungsmittel und in Gegenwart eines basischen Mittels zur Verbindung der Formel XV, in der Z eine Oxyranylmethylgruppe bedeutet.

25. Verfahren nach Anspruch 11 oder 24, dadurch gekennzeichnet, daß das basische Mittel ein Alkalimetallcarbonat, ein Alkalimetallhydroxid, ein Alkalimetallhydrid oder ein Alkalimetallalkoholat ist.

26. Verwendung der Indolizinderivate nach Anspruch 16 zur Herstellung der Indolizinderivate nach einem der Ansprüche 1 bis 8.

**Patentansprüche für die Vertragsstaaten AT, ES, GR**

1. Verfahren zur Herstellung von Indolizinderivaten der allgemeinen Formel I

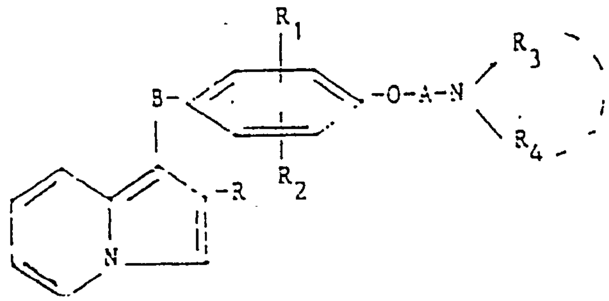

(I),

in der bedeuten :

A eine Alkylengruppe mit 2 bis 5 Kohlenstoffatomen,

B eine Gruppe –S– der –$SO_2$–,

R Wasserstoff, eine geradkettige oder verzweigte Alkylgruppe mit 1 bis 8 Kohlenstoffatomen, eine Cycloalkylgruppe mit maximal 6 Kohlenstoffatomen oder eine Phenylgruppe, die gegebenenfalls mit einem oder mehreren gleichen oder verschiedenen Substituenten substituiert ist, die ausgewählt sind unter Halogenatomen, niederen Alkylgruppen mit 1 bis 4 Kohienstoffatomen, niederen Alkoxygruppen mit 1 bis 4 Kohlenstoffatomen und Nitrogruppen,

$R_1$ und $R_2$, die gleich oder verschieden sind, jeweils Wasserstoff, Methyl oder Ethyl oder eine Halogen,

$R_3$ eine geradkettige oder verzweigte Alkylgruppe mit 1 bis 8 Koholenstoffatomen oder eine Gruppe der Formel

–Alk-$R_5$,

in der Alk eine Einfachbindung oder eine geradkettige oder verzweigte Alkylengruppe mit 1 bis 5 Kohlenstoffatomen und $R_5$ Pyridyl, Phenyl, 2,3-Methylendioxyphenyl, 3,4-Methylendioxyphenyl oder eine Phenylgruppe bedeuten, die mit einem oder mehreren gleichen oder verschiedenen, unter Halogenatomen, niederen Alkylgruppen mit 1 bis 4 Kohlenstoffatomen oder niederen Alkoxygruppen mit 1 bis 4 Kohlenstoffantomen ausgewählten Substituenten substituiert ist.

$R_4$ Wasserstoff oder eine Alkylgruppe mit 1 bis 8 Kohlenstoffatomen,

oder $R_3$ und $R_4$ zusammen 1,4-Tetramethylen, 1,5-Pentamethylen, 3-Oxo-1,5-pentamethylen, 3-Aza-1,5-pentamethylen, 3-Methylaza-1,5-pentamethylen, 3-Phenylaza-1,5-pentamethylen oder –CH = CH-N = CH–, derart, daß $R_3$ und $R_4$ zusammen mit dem Stickstoffatom, an dem sie gebunden sind, insbesondere Pyrrolidinyl, Piperidinyl, Morpholinyl, Piperazinyl, 4-Methylpiperazinyl, 4-Phenylpiperazinyl oder 1H-Imidazolyl bedeuten könen, sowie der pharmazeutisch akzeptablen Salze dieser Derivate, gekennzeichnet durch Kondensation eines 1-(4-Alkoxybenzolsulfonyl)-indolizinderivats der allgemeinen Formel II

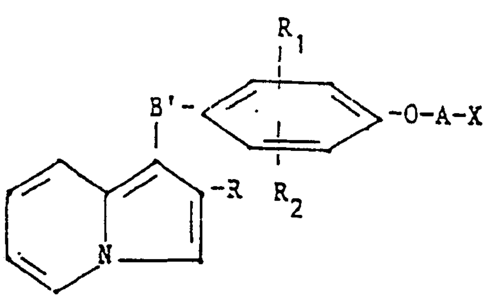

(II),

in der bedeuten :

B′ eine Gruppe –S– oder –$SO_2$–,

R, $R_1$, $R_2$ und A dasselbe wie oben und

X ein Halogenatom oder eine Alkylsulfonyloxygruppe mit 1 bis 4 Kohlenstoffatomen oder eine Arylsulfonyloxygruppe mit 6 bis 10 Kohlenstoffatomen, mit einem Amin der allgemeinen Formel III

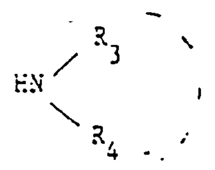

(III),

in der $R_3$ und $R_4$ die gleiche Bedeutung wie oben haben, in Gegenwart eines Säureakzeptors in einem polaren oder unpolaren Lösungsmittel bei einer Temperatur zwischen Umgebungstemperatur und der Rückflußtemperatur zum gewünschten Indolizinderivat, das gewünschtenfalls mit einer organischen oder anorganischen Säure zu einem pharmazeutisch akzeptablen Salz dieses Dreivats umgesetzt wird.

2. Verfahren zur Herstellung von Indolizinderivaten der allgemeinen Formel I

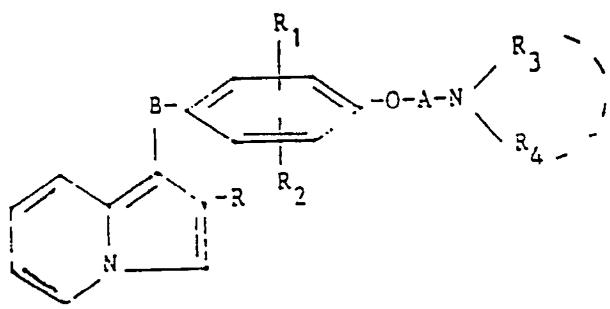

(I) :

in der bedeuten :

A eine Alkylengruppe mit 2 bis 5 Kohlenstoffatomen,

B eine Gruppe $-S-$ oder $-SO_2-$,

R Wasserstoff, eine geradkettige oder verzweigte Alkylgruppe mit 1 bis 8 Kohlenstoffatomen, eine Cycloalkylgruppe mit maximal 6 Kohlenstoffatomen oder eine Phenylgruppe, die gegebenenfalls mit einem oder mehreren gleichen oder verschiedenen Substituenten substituiert ist, die ausgewählt sind unter Halogenatomen, niederen Alkylgruppen mit 1 bis 4 Kohlenstoffatomen, niederen Alkoxygruppen mit 1 bis 4 Kohlenstoffatomen und Nitrogruppen,

$R_1$ und $R_2$ die gleich oder verschieden sind, jeweils Wasserstoff, Methyl oder Ethyl oder ein Halogen,

$R_3$ eine geradkettige oder verzweigte Alkylgruppe mit 1 bis 8 Kohlenstoffatomen oder eine Gruppe der Formel

$$-Alk-R_5,$$

in der Alk eine Einfachbindung oder eine geradkettige oder verzweigte Alkylengruppe mit 1 bis 5 Kohlenstoffatomen und $R_5$ Pyridyl, Phenyl, 2,3-Methylendioxyphenyl, 3,4-Methylendioxyphenyl oder eine phenylgruppe bedeuten, die mit einem oder mehreren gleichen oder verschiedenen, unter Halogenatomen, niederen Alkylgruppen mit 1 bis 4 Kohlenstoffatomen oder niederen Alkoxygruppen mit 1 bis 4 Kohlenstoffatomen ausgewählten Substituenten substituiert ist,

und

$R_4$ Wasserstoff oder eine Alkylgruppe mit 1 bis 8 Kohlenstoffatomen

oder $R_3$ une $R_4$ zusammen 1,4-Tetramethylen, 1,5-Pentamethylen, 3-Oxo-1,5-pentamethylen, 3-Aza-1,5-pentamethylen, 3-Methylaza-1,5-pentamethylen, 3-Phenyl- aza-1,5-pentamethylen oder $-CH=CH-N=CH-$, daß $R_3$ und $R_4$ zusammen mit dem Stickstoffatom, an dem sie gebunden sind, insbesondere Pyrrolidinyl, Piperidinyl, Morpholinyl, Piperazinyl, 4-Methylpiperazinyl, 4-Phenylpiperazinyl oder 1H-Imidazolyl bedeuten können, sowie der pharmazeutisch akzeptablen Salze dieser Derivate, gekennzeichnet durch Umsetzung eines 1-(4-Hydroxybenzolsulfonyl)-indolizins der allgemeinen Formel IV

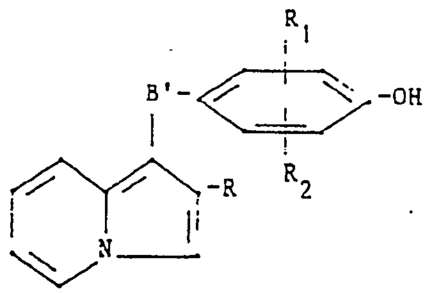

(IV),

in der bedeuten :

B′ eine Gruppe –S– oder –$SO_2$– und

R, $R_1$ und $R_2$ dasselbe wie oben, mit einer Verbindung der allgemeinen Formel

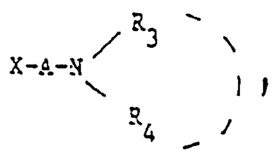

in der bedeuten :

X eine Halogenatom oder eine Alkylsulfonylgruppe mit 1 bis 4 Kohlenstoffatomen oder eine Arylsulfonylgruppe mit 6 bis 10 Kohlenstoffatomen,

A eine Alkylengruppe mit 2 bis 5 Kohlenstoffatomen und

$R_3$ und $R_4$ dasselbe wie oben, in Gegenwart einer Base am Rückfluß und in einem geeigneten Lösungsmittel zum gewünschten Indolizinderivat, das gewünschtenfalls mit einer geeigneten organischen oder anorganischen Säure zu einem pharmazeutisch akzeptablen Salz umgesetzt wird.

3. Verfahren zur Herstellung von Indolizinderivaten der Formel I, in der bedeuten :

A die Gruppe 2-Hydroxypropylen,

B eine Gruppe –S– oder –$SO_2$–,

R Wasserstoff, eine geradkettige oder verzweigte Alkylgruppe mit 1 bis 8 Kohlenstoffatomen, eine Cycloalkylgruppe mit maximal 6 Kohlenstoffatomen oder eine Phenylgruppe, die gegebenenfalls mit einem oder mehreren gleichen oder verschiedenen Substituenten substituiert ist, die ausgewählt sind unter Halogenatomen, niederen Alkylgruppen mit 1 bis 4 Kohlenstoffatomen, niederen Alkoxygruppen mit 1 bis 4 Kohlenstoffatomen und Nitrogruppen,

$R_1$ und $R_2$, die gleich oder verschieden sind, jeweils Wasserstoff, Methyl oder Ethyl oder eine Halogen,

$R_3$ eine geradkettige oder verzweigte Alkylgruppe mit 1 bis 8 Kohlenstoffatomen oder eine Gruppe der Formel

$$-Alk-R_5,$$

in der Alk eine Einfachbindung oder eine geradkettige oder verzweigte Alkylengruppe mit 1 bis 5 Kohlenstoffatomen und $R_5$ Pyridyl, Phenyl, 2,3-Methylendioxyphenyl, 3,4-Methylendioxyphenyl oder eine Phenylgruppe bedeuten, die mit einem oder mehreren gleichen oder verschiedenen, unter Halogenatomen, niederen Alkylgruppen mit 1 bis 4 Kohlenstoffatomen oder niederen Alkoxygruppen mit 1 bis 4 Kohlenstoffatomen ausgewählten Substituenten substituiert ist, und

$R_4$ Wasserstoff oder eine Alkylgruppe mit 1 bis 8 Kohlenstoffatomen

oder $R_3$ und $R_4$ zusammen 1,4-Tetramethylen, 1,5-Pentamethylen, 3-Oxo-1,5-pentametylen, 3-Aza-1,5-pentamethylen, 3-Methylaza-1,5-pentamethylen, 3-Phenyl- aza-1,5-pentamethylen oder –CH = CH-N = CH–, derart, daß $R_3$ und $R_4$ zusammen mit dem Stickstoffatom, an dem sie gebunden sind, insbesondere Pyrrolidinyl, Piperidinyl, Morpholinyl, Piperazinyl, 4-Methylpiperazinyl, 4-Phenylpiperazinyl oder 1H-Imidazolyl Bedeuten können, sowie der pharmazeutisch akzeptablen Salze dieser Derivate, gekennzeichnet durch Umsetzung eines Oxyranylmethoxyderivats der allgemeinen Formel XIV

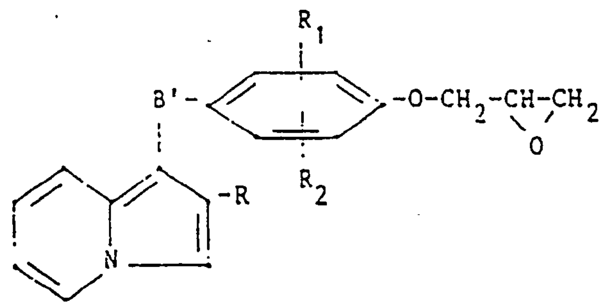

(XIV),

in der bedeuten :

B′ eine Gruppe –S– oder –$SO_2$– und

R, $R_1$ und $R_2$ dasselbe wie oben, mit einem Amin der allgemeinen Formel III

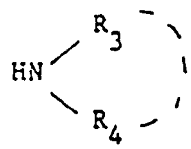

(III),

in der $R_3$ und $R_4$ dasselbe wie oben bedeuten, am Rückfluß in einem polaren Lösungsmitterl oder in einem Überschuß des Amins zum gewünschten Indolizinderivat, das gewünschtenfalls mit einer organischen oder anorganischen Säure zu einem pharmazeutisch akzeptablen Salz des Derivats umgesetzt wird.

4. Verfahren nach einem der vorhergehenden Ansprüche, gekennzeichnet durch Herstellung von Indolizinderivaten der allgemeinen Formel

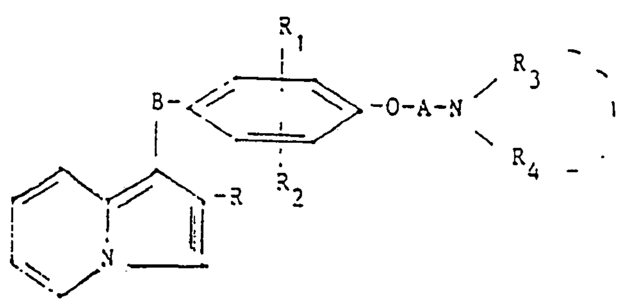

,

in der bedeuten :

B eine Gruppe –S– oder –$SO_2$–,

R Wasserstoff, Methyl, Ethyl, n-Propyl, Isopropyl, n-Butyl, Isobutyl, tert.-Butyl, 1-Methylpropyl, n-Pentyl, Neopentyl, Phenyl, Monofluor-, Monochlor- oder Monobromphenyl, Difluor-, Dichlor- oder Dibromphenyl, Monomethyl- oder Dimethylphenyl, Monomethoxy- oder Dimethoxyphenyl, Nitrophenyl oder mit einem Halogenatom substituiertes Methylphenyl,

$R_1$ und $R_2$, die gleich oder verschieden sind, jeweils Wasserstoff, Chlor, Brom oder Jod oder Methyl,

A 1,2-Ethylen, 1,3-Propylen, 2-Methyl-1,3-propylen, 1,4-Tetramethylen, 1,5-Pentamethylen oder 2-Hydroxypropylen,

$R_3$ Methyl, Ethyl, n-Propyl, Isopropyl, n-Butyl, Isobutyl, tert.-Butyl, 1-Methylpropyl, n-Pentyl, n-Hexyl, n-Heptyl, n-Octyl, Phenyl, Benzyl, Phenethyl, Methoxyphenethyl, Dimethoxyphenethyl, Dimethylphen- ethyl, Dimethoxybenzyl, Pyridylethyl oder eine im aromatischen Teil mit Methyl und Methoxy substituierte Phenethylgruppe, und

$R_4$ Wasserstoff oder Methyl, Ethyl, n-Propyl, Isopropyl, n-Butyl, Isobutyl, tert.-Butyl, n-Pentyl, Neopentyl, n-Hexyl-, n-Heptyl oder n-Octyl oder

$R_3$ und $R_4$ zusammen 1,4-Tetramethylen, 1,5-Pentamethylen, 3-Oxa-1,5-pentamethylen, 3-Aza-1,5-pentamethylen, 3-Methylaza-1,5-pentamethylen, 3-Phenylaza-1,5-pentamethylen oder –CH = CH-N = CH–, sowie der pharmazeutisch akzeptablen Salze dieser Derivate.

5. Verfahren nach Anspruch 1 bis 4, gekennzeichnet durch Herstellung von Indolizinderivaten, in deren

Formel B eine Gruppe $-SO_2-$ bedeutet.

6. Verfahren nach Anspruch 1 bis 4, gekennzeichnet durch Herstellung von Indolizinderivaten, in deren Formel $R_3$ 3,4-Dimethoxy, Phenyl, 3,4-Dimethoxybenzyl oder 3,4-Dimethoxyphenethyl bedeutet.

7. Verfahren nach Anspruch 1 oder 2, gekennzeichnet durch Herstellung von 2-Isopropyl-1- [4-(3-(N-methyl-N-(3,4-dimethoxy-β-phenethyl)-amino)-propyl)-oxy-benzolsulfonyl]-indolizin und seiner pharmazeutisch akzeptablen Salze.

8. Verfahren nach Anspruch 1 oder 2, gekennzeichnet durch Herstellung von 2-Isopropyl-1-[4-(3-di-n-butylamino-propyl)-oxy-benzolsulfonyl]-indolizin und seiner pharmazeutisch akzeptablen Salze.

9. Verfahren nach Anspruch 1 oder 2, gekennzeichnet durch Herstellung von 2-Ethyl-1-[4-(3-(N-methyl-N-(3,4-di-methoxy-β-phenethyl)-amino)-propyl)-oxy-benzolsulfonyl]-indolizin und seiner pharmazeutisch akzeptablen Salze.

10. Verfahren nach Anspruch 1 oder 2, gekennzeichnet durch Herstellung von 2-Ethyl-1-[4-(3-(di-n-butylamino-propyl)-oxy)-benzolsulfonyl]-indolizin und seiner pharmazeutisch akzeptablen Salze.

11. Verfahren nach einem der vorhergehenden Ansprüche, gekennzeichnet durch Herstellung von Indolizinderivaten, deren pharmazeutisch akzeptables Salz das Hydrochlorid ist.

12. Verfahren zur Herstellung pharmazeutischer oder veterinärmedizinischer Zusammensetzungen, dadurch gekennzeichnet, daß mindestens ein nach einem der vorhergehenden Ansprüche hergestelltes Indolizinderivat zusammen mit einem pharmazeutischen Träger oder einem geeigneten Excipiens formuliert wird.

13. Verfahren zur HErstellung pharmazeutischer oder veterinärmedizinischer Zusammensetzungen nach Anspruch 12 zur Behandlung pathologischer Syndrome des cardiovasculären Systems, die 50 bis 500 mg Wirkstoff enthalten.

14. Verfahren zur Herstellung von Indolizinderivaten der allgemeinen Formel XV

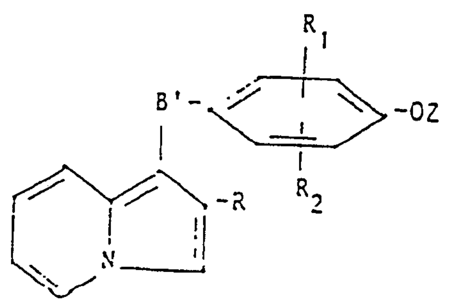

(XV),

in der bedeuten :

B' eine Gruppe -S- oder $-SO_2-$,

R, $R_1$ und $R_2$ dasselbe wie in Anspruch 1 und

Z Wasserstoff, eine Oxyranylmethylgruppe, eine Alkylsulfonylgruppe mit 1 bis 4 Kohlenstoffatomen, eine Arylsulfonylgruppe mit 6 bis 10 Kohlenstoffantomen oder eine Gruppe der Formel

$$-A-Z_1,$$

in der A dasselbe wie in Anspruch 1 und

$Z_1$ ein Halogenatom, eine Hydroxygruppe, eine Alkylsulfonylgruppe mit 1 bis 4 Kohlenstoffatomen oder eine Arylsulfonyloxygruppe mit 6 bis 10 Kohlenstoffatomen bedeuten, gekennzeichnet durch

a) Cyclisierung eines Picolylsulfons oder eines Picolylsulfids der allgemeinen Formel

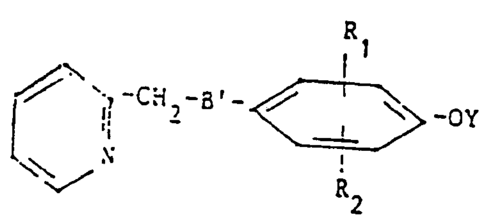

,

in der B', $R_1$und $R_2$ die gleiche Bedeutung wie oben haben und Y eine Alkylsulfonylgruppe mit 1 bis 4 Kohlenstoffatomen oder eine Arylsulfonylgruppe mit 6 bis 10 Kohlenstoffatomen bedeuten, am Rückfluß in einem Lösungsmittel und in Gegenwart eines basischen Mittels mit einem α-Halogenketon der allgemeinen

Formel

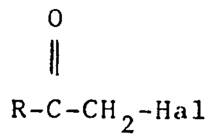
,

in der R die gleiche Bedeutung wie oben hat und Hal ein Halogenatom bedeutet, zur Verbindung der Formel XV, in der Z eine Alkylsulfonylgruppe mit 1 bis 4 Kohlenstoffatomen oder eine Arylsulfonylgruppe mit 6 bis 10 Kohlenstoffatomen bedeuten ;

b) Umsetzung eines Picolylsulfids der allgemeinen Formel

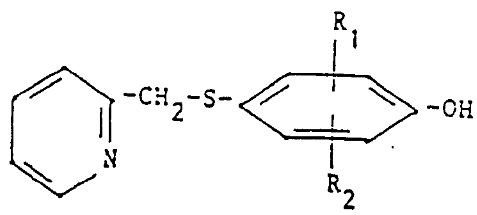
,

in der $R_1$ und $R_2$ die gleiche Bedeutung wie oben haben, mit einem α-Halogenketon der allgemeinen Formel

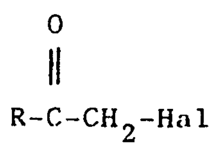
,

in der R und Hal dasselbe wie oben bedeuten, zum Pyridiniumsalz, das am Rückfluß in einem Lösungsmittel und in Gegenwart eines basischen Mittels zur Verbindung der Formel XV cyclisiert wird, in der Z Wasserstoff und B' eine Gruppe –S– bedeuten ;

c) Hydrolyse eines Indolizinderivats der Formel XV, in der Z eine Alkylsulfonylgruppe mit 1 bis 4 Kohlenstoffatomen oder eine Arylsulfonylgruppe mit 6 bis 10 Kohlenstoffatomen bedeutet, am Rückfluß und in einem basischen Medium zur Verbindung der Formel XV, in der Z Wasserstoff bedeutet ;

d) Kondensation eines Indolizinderivats der Formel XV, in der Z Wasserstoff bedeutet, mit einem Dihalogenalkan der allgemeinen Formel

Hal-A-Hal,

in der A und Hal dasselbe wie oben bedeuten, am Rückfluß in einem Lösungsmittel und in Gegenwart eines basischen Mittels zur Verbindung der Formel XV, in der Z eine Gruppe der Formel $-A-Z_1$ bedeutet, in der $Z_1$ eine Halogenatom darstellt ;

e) Kondensation eines Indolizinderivats der Formel XV, in der Z Wasserstoff bedeutet, mit einem halogenierten Alkohol der allgemeinen Formel

Hal-A-OH,

in der A und Hal dasselbe wie oben bedeuten, in einem Lösungsmittel in Gegenwaart eines basischen Mittels zur Verbindung der Formel XV, in der Z eine Gruppe der Formel $-A-Z_1$ bedeutet, in der $Z_1$ eine Hydroxygruppe darstellt ;

f) Kondensation eines Indolizinderivats der Formel XV, in der Z eine Gruppe der Formel $-A-Z_1$ bedeutet, in der $Z_1$ eine Hydroxygruppe darstellt, mit einem Halogenid der allgemeinen Formel

Hal-Y,

in der Hal und Y dasselbe wie oben bedeuten, in einem Lösungsmittel und in Gegenwart eines Säureakzeptors zum Indolizinderivat der Formel $-A-Z_1$, in der $Z_1$ eine Alkylsulfonyloxygruppe mit 1 bis 4 Kohlenstoffatomen oder eine Arylsulfonyloxygruppe mit 6 bis 10 Kohlenstoffatomen bedeutet ;

g) Umsetzung eines Indolizinderivats der Formel XV, in der Z Wasserstoff bedeutet, mit einem Epihalohydrin am Rückfluß in einem polaren Lösungsmittel und im Gegenwart eines basischen Mittels zur Verbindung der Formel XV, in der Z eine Oxyranylmethylgruppe bedeutet.

15. Verfahren nach Anspruch 2 oder 14, dadurch gekennzeichnet, daß das basische Mittel eine Alkalimetallcarbonat, ein Alkalimetallhydroxid, ein Alkalimetallhydrid oder eine Alkalimetallalkoholat ist.

16. Verfahren nach Anspruch 14, gekennzeichnet durch herstellung von Indolizinderivaten, wobei Z Methansulfonyloxy, Benzolsulfonyloxy oder p-Toluolsulfonyloxy und $Z_1$ ein Bromatom, Methansulfonyloxy, Benzolsulfonyloxy oder p-Toluolsulfonyloxy bedeuten.

17. Verfahren nach Anspruch 14, gekennzeichnet durch Herstellung von 2-Ethyl-1-[4-(3-brompropyl)-oxy-benzolsulfonyl]-indolizin.

18. Verfahren nach Anspruch 14, gekennzeichnet durch Herstellung von 2-Isopropyl-1-[4-(3-brompropyl)-oxy-benzolsulfonyl]-indolizin.

19. Verfahren nach Anspruch 14, gekennzeichnet durch Herstellung von 2-Ethyl-1-(4-tosyloxybenzolsulfonyl)-indolizin.

20. Verfahren nach Anspruch 14, gekennzeichnet durch Herstellung von 2-Isopropyl-1-(4-tosyloxybenzolsulfonyl)-indolizin.

21. Verfahren nach Anspruch 14, gekennzeichnet durch Herstellung von 2-Ethyl-1-(4-hydroxybenzolsulfonyl)-indolizin.

22. Verfahren nach Anspruch 14, gekennzeichnet durch Herstellung von 2-Isopropyl-1-(4-hydroxybenzolsulfonyl)-indolizin.

## Claims

### CLAIMS for the contracting states : CH, DE, FR, GB, IT, LI, LU, NL, SE

1. Indolizine derivatives corresponding to the general formula :

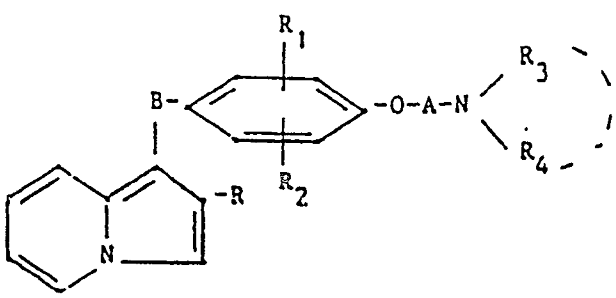

I

in which :

B represents an –S– or $-SO_2-$ group,

R represents hydrogen, a linear or branched alkyl radical having from 1 to 8 carbon atoms, a cycloalkyl radical having 6 carbon atoms at the maximum or a phenyl group which is non-substituted or substituted by one or more identical or different substituents, selected from halogen atoms and from lower alkyl groups having from 1 to 4 carbon atoms, lower alkoxy groups having from 1 to 4 carbon atoms or nitro groups,

$R_1$ and $R_2$, which are identical or different, each represents hydrogen, the methyl or ethyl radical or a halogen,

A represents a linear or branched alkylene radical having from 2 to 5 carbon atoms or the 2-hydroxypropylene radical,

$R_3$ represents a linear or branched alkyl radical having from 1 to 8 carbon atoms or a radical of formula :

$$-Alk-R_5$$

in which Alk represents a simple bond or a linear or branched alkylene radical having from 1 to 5 carbon atoms and $R_5$ represents a pyridyl, phenyl, 2,3-methylenedioxyphenyl or 3,4-methylenedioxyphenyl radical or a phenyl group substituted by one or more identical or different substituents selected from halogen atoms, lower alkyl groups having from 1 to 4 carbon atoms or lower alkoxy groups having from 1 to 4 carbon atoms,

$R_4$ represents hydrogen or an alkyl radical having from 1 to 8 carbon atoms,

or $R_3$ and $R_4$, when taken together, represent a 1,4-tetramethylene, 1,5-pentamethylene, 3-oxo-1,5-pentamethylene, 3-aza-1,5-pentamethylene, 3-methylaza-1,5-pentamethylene, 3-phenylaza-1,5-pentamethylene or $-CH=CH-N=CH-$ group in such a way that $R_3$ and $R_4$ taken with the nitrogen atom to which they are attached can represent in particular a pyrrolidinyl, piperidinyl, morpholinyl, piperazinyl, 4-methylpiperazinyl, 4-phenylpiperazinyl or 1H-imidazolyl radical as well as the pharmaceutically acceptable salts of these derivatives.

2. Indolizine derivatives corresponding to the general formula :

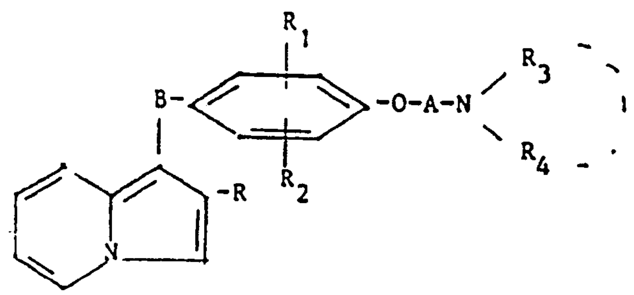

in which :

B represents an $-S-$ or $-SO_2-$ group,

R represents hydrogen or a methyl, ethyl, n-propyl, isopropyl, n-butyl, isobutyl, t-butyl, 1-methylpropyl, n-pentyl, neopentyl, phenyl, monofluoro-, monochloro- or monobromophenyl, difluoro-, dichloro- or dibromophenyl, monomethyl- or dimethylphenyl, monomethoxy- or dimethoxyphenyl, or nitrophenyl radical or a methylphenyl radical substituted by a halogen atom,

$R_1$ and $R_2$, which are identical or different, each represents hydrogen, chlorine, bromine or iodine or the methyl radical,

A represents a 1,2-ethylene, 1,3-propylene, 2-methyl-1,3-propylene, 1,4-tetramethylene, 1,5-pentamethylene or 2-hydroxypropylene radical,

$R_3$ represents a methyl, ethyl, n-propyl, isopropyl, n-butyl, isobutyl, t-butyl, 1-methylpropyl, n-pentyl, n-hexyl, n-heptyl, n-octyl, phenyl, benzyl, phenethyl, methoxyphenethyl, dimethoxyphenethyl, dimethylphenethyl, dimethoxybenzyl or pyridylethyl radical or a phenethyl radical substituted in its aromatic part by methyl and methoxy radicals,

$R_4$ represents hydrogen or a methyl, ethyl, n-propyl, isopropyl, n-butyl, isobutyl, t-butyl, n-pentyl, neopentyl, n-hexyl, n-heptyl or n-octyl radical,

$R_3$ and $R_4$ taken together represent a 1,4-tetramethylene, 1,5-pentamethylene, 3-oxa-1,5-pentamethylene, 3-aza-1,5-pentamethylene, 3-methylaza-1,5-pentamethylene, 3-phenylaza-1,5-pentamethylene or $-CH=CH-N=CH-$ radical, as well as the pharmaceutically acceptable salts of these derivatives.

3. Indolizine derivatives according to one of Claims 1 or 2, characterized in that B represents an $-SO_2-$ group.

4. Indolizine derivatives according to one of Claims 1 or 2, characterized in that $R_3$ represents a 3,4-dimethoxyphenyl, 3,4-dimethoxybenzyl or 3,4-dimethoxyphenethyl radical.

5. 2-isopropyl-1{{{3-[N-methyl-N-(3,4-dimethoxy-β-phenethyl)amino]propyl}-4-oxybenzenesulphonyl}}indoziline and its pharmaceutically acceptable salts.

6. 2-isopropyl-1-[(3-di-n-butylaminopropyl)-4-oxybenzenesulphonyl]indolizine and its pharmaceutically acceptable salts.

7. 2-ethyl-1{{{3-[N-methyl-N-(3,4-dimethoxy-β-phenethyl)amino]propyl}-4-oxybenzenesulphonyl}}indoziline and its pharmaceutically acceptable salts.

8. 2-ethyl-1-[(3-di-n-butylaminopropyl)-4-oxybenzenesulphonyl]indolizine and its pharmaceutically acceptable salts.

9. Indolizine derivatives according to one of Claims 1 to 8, characterized in that the pharmaceutically acceptable salt is the hydrochloride.

10. Process for preparation of indolizine derivatives according to Claim 1, in which A represents an alkylene radical having from 2 to 5 carbon atoms and B represents an $-S-$ or $-SO_2-$ group, characterized in that a 1-(4-alkoxybenzenesulphonyl)indolizine derivative of general formula

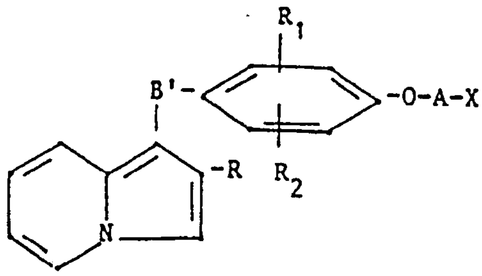

II

in which B′ represents an –S– or –$SO_2$ group, R, $R_1$ and $R_2$ have the same meaning as in Claim 1, A has the same meaning as before and X represents a halogen atom or an alkylsulphonyloxy group having from 1 to 4 carbon atoms or arylsulphonyloxy having from 6 to 10 carbon atoms is condensed, in presence of an acid acceptor in a polar or nonpolar solvent and at a temperature of between ambient temperature and the reflux temperature, with an amine of general formula :

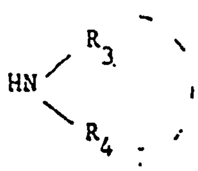

III

in which $R_3$ and $R_4$ have the same meaning as in Claim 1, to form the desired indolizine derivative, which is reacted if desired with an organic or inorganic acid in order to form a pharmaceutically acceptable salt of this derivative.

11. Process for preparation of indolizine derivatives according to Claim 1 in which A represents an alkylene radical having from 2 to 5 carbon atoms and B represents an –S– or –$SO_2$– group, characterized in that a 1-(4-hydroxybenzenesulphonyl)indolizine of general formula

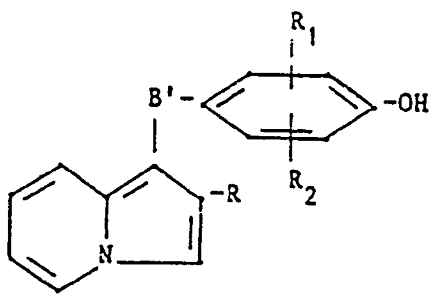

IV

in which B′ represents an –S– or –$SO_2$– group and R, $R_1$ and $R_2$ have the same meaning as in Claim 1, is reacted in presence of a base with a compound of general formula

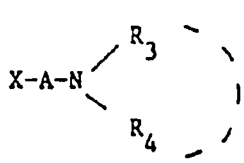

in which X represents a halogen atom or an alkylsulphonyl group having from 1 to 4 carbon atoms or an arylsulphonyl group having from 6 to 10 carbon atoms, A represents an alkylene radical having from 2 to 5 carbon atoms and $R_3$ and $R_4$ have the same value as in Claim 1, the reaction taking place under reflux and in an appropriate solvent to obtain the desired indolizine derivative which can, if desired, be reacted with an appropriate organic or inorganic acid to form a pharmaceutically acceptable salt.

12. Process for preparation of indolizine derivatives according to Claim 1 in which A represents the 2-hydroxypropylene radical and B represents an –S– or –$SO_2$– group, characterized in that an oxiranylmethoxy deri-

vative of general formula :

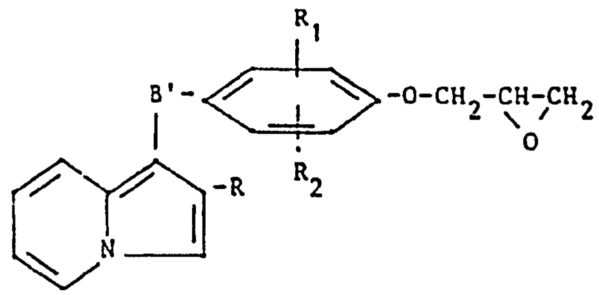

XIV

in which B′ represents an –S– or –$SO_2$– group and R, $R_1$ and $R_2$ have the same meaning as in Claim 1 is refluxed with an amine of general formula :

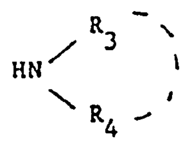

III

in which $R_3$ and $R_4$ have the same meaning as in Claim 1 and that, in a polar solvent or in an excess of the said amine to give the desired indolizine derivative, reaction is carried out, if desired, with an organic or inorganic acid to form a pharmaceutically acceptable salt of this derivative.

13. Pharmaceutical or veterinary compositions containing, as active principle, at least one indolizine derivative according to one of Claims 1, 2 or 9, in association with a pharmaceutical vehicle or an appropriate excipient.

14. Pharmaceutical or veterinary compositions containing, as active principle, at least one indolizine derivative according to one of Claims 3 to 8, in association with a pharmaceutical vehicle or an appropriate excipient.

15. Pharmaceutical or veterinary compositions according to one of Claims 13 or 14 for the treatment of pathological syndromes of the cardiovascular system containing from 50 mg to 500 mg of active principle.

16. Indolizine derivatives of general formula :

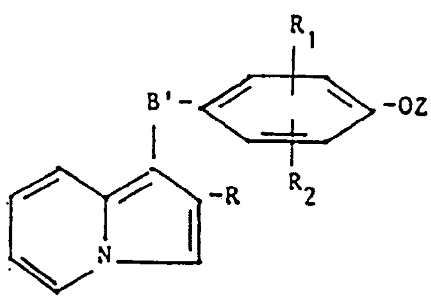

XV

in which

B′ represents an –S– or –$SO_2$– group,

Z represents hydrogen, an oxiranylmethyl radical, an alkylsulphonyl radical having from 1 to 4 carbon atoms, an arylsulphonyl radical having from 6 to 10 carbon atoms or a radical of formula :

$$-A-Z_1$$

in which A is as defined in Claim 1 and $Z_1$ represents a halogen atom, the hydroxy radical, an alkylsulphonyloxy radical having from 1 to 4 carbon atoms or an arylsulphonyloxy radical having from 6 to 10 carbon atoms.

17. Indolizine derivatives according to Claim 16, characterized in that Z represents a methanesulphonyloxy, benzenesulphonyloxy or p-toluenesulphonyloxy radical and $Z_1$ represents a bromine atom or a methanesulphonyloxy, benzenesulphonyloxy or p-toluenesulphonyloxy radical.

18. 2-Ethyl-1-[(3-bromopropyl)-4-oxybenzenesulphonyl]indolizine.

19. 2-Isopropyl-1-[(3-bromopropyl)-4-oxybenzenesulphonyl]indolizine.
20. 2-Ethyl-1-(4-tosyloxybenzenesulphonyl)indolizine.
21. 2-Isopropyl-1-(4-tosyloxybenzenesulphonyl)indolizine.
22. 2-Ethyl-1-(4-hydroxybenzenesulphonyl)indolizine.
23. 2-Isopropyl-1-(4-hydroxybenzenesulphonyl)indolizine.
24. Process for preparation of indolizine derivatives according to Claim 16, characterized in that :
(a) a picolyl sulphone or a picolyl sulphide of general formula :

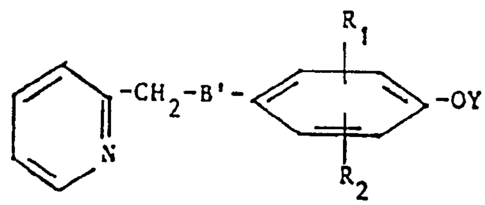

in which B′, $R_1$1 and $R_2$ have the same meaning as in Claim 16 and Y represents an alkylsulphonyl radical having from 1 to 4 carbon atoms or an arylsulphonyl radical having from 6 to 10 carbon atoms, is cyclized under reflux in a solvent and in presence of a base with an α-halogenoketone of general formula :

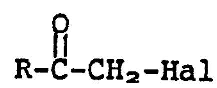

in which R has the same meaning as in Claim 16 and Hal represents a halogen atom, in order to obtain the compounds of formula XV in which Z represents an alkylsulphonyl radical having from 1 to 4 carbon atoms or arylsulphonyl radical having from 6 to 10 carbon atoms,
(b) a picolyl sulphide of general formula :

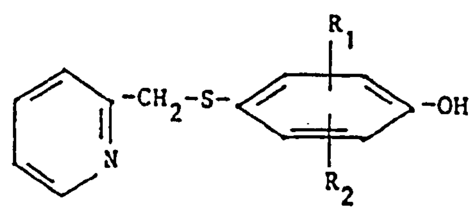

in which $R_1$ and $R_2$ have the same meaning as in Claim 16 is reacted with an α-halogenoketone of general formula :

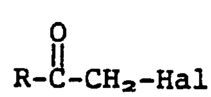

in which R and Hal have the same meaning as before, in order to obtain a pyridinium salt which is cyclized under reflux in a solvent and in presence of a base in order to obtain the compounds of formula XV in which Z represents hydrogen and B′ represents an –S– group,
(c) an indoziline derivative of formula XV, in which Z represents an alkylsulphonyl radical having from 1 to 4 carbon atoms or arylsulphonyl radical having from 6 to 10 carbon atoms, is hydrolyzed under reflux and in basic medium in order to obtain the compounds of formula XV in which Z represents hydrogen,
(d) an indolizine derivative of formula XV in which Z represents hydrogen is condensed under reflux, in a solvent and in presence of a base, with a dihalogenoalkane of general formula :

Hal-A-Hal

in which A has the same meaning as in Claim 16 and Hal has the same meaning as before, in order to

obtain the compounds of formula XV in which Z represents a radical of formula $-A-Z_1$ in which $Z_1$ represents a halogen atom,

e) an indolizine derivative of formula XV in which Z represents hydrogen is condensed in a solvent and in presence of a base with a halogenated alcohol of general formula :

Hal-A-OH

in which A and Hal have the same meaning as before to obtain the compounds of formula XV in which Z represents a radical of formula $-A-Z_1$ in which $Z_1$ represents the hydroxy group,

f) an indolizine derivative of formula XV in which Z represents a radical of formula $-A-Z_1$ in which $Z_1$ represents the hydroxy group is condensed in a solvent and in presence of an acid acceptor with a halide of general formula

Hal-Y

in which Hal and Y have the same meaning as before in order to obtain an indolizine derivative of formula XV in which Z represents a radical of formula $-A-Z_1$ in which $Z_1$ represents an alkylsulphonyloxy group having from 1 to 4 carbon atoms or arylsulphonyloxy group having from 6 to 10 carbon atoms,

g) an indolizine derivative of formula XV in which Z represents hydrogen is reacted under reflux, in a polar solvent and in presence of a base, with an epihalohydrin, in order to obtain the compounds of formula XV in which Z represents an oxiranylmethyl radical.

25. Process according to one of Claims 11 or 24, characterized in that the base is an alkali metal carbonate, an alkali metal hydroxide, an alkali metal hydride or an alkali metal alcoholate.

26. Use of the indolizine derivatives according to Claim 16 for the preparation of indolizine derivatives according to one of Claims 1 to 8.

**Claims for the contracting states : AT, ES, GR**

1. Process for the production of indolizine derivatives corresponding to the general formula :

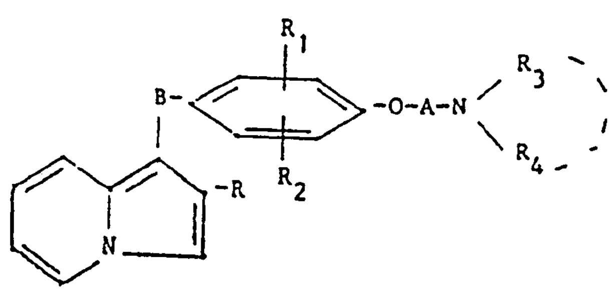

I

in which :

A represents an alkylene radical having from 2 to 5 carbon atoms,

B represents an $-S-$ or $-SO_2-$ group,

R represents hydrogen, a linear or branched alkyl radical having from 1 to 8 carbon atoms, a cycloalkyl radical having 6 carbon atoms at the maximum or a phenyl group which is non-substituted or substituted by one or more identical or different substituents, selected from halogen atoms and from lower alkyl groups having from 1 to 4 carbon atoms, lower alkoxy groups having from 1 to 4 carbon atoms or nitro groups,

$R_1$ and $R_2$, which are identical or different, each represents hydrogen, the methyl or ethyl radical or a halogen,

$R_3$ represents an alkyl radical having from 1 to 8 carbon atoms or a radical of formula :

$-Alk-R_5$

in which Alk represents a simple bond or a linear or branched alkylene radical having from 1 to 5 carbon atoms and $R_5$ represents a pyridyl, phenyl, 2,3-methylenedioxyphenyl or 3,4-methylenedioxyphenyl radical or a phenyl group substituted by one or more identical or different substituents selected from halogen atoms, lower alkyl groups having from 1 to 4 carbon atoms or lower alkoxy groups having from 1 to 4 carbon atoms,

$R_4$ represents hydrogen or an alkyl radical having from 1 to 8 carbon atoms,

or $R_3$ and $R_4$, when taken together, represent a 1,4-tetramethylene, 1,5-pentamethylene, 3-oxo-1,5-pentamethylene, 3-aza-1,5-pentamethylene, 3-methylaza-1,5-pentamethylene or 3-phenylaza-1,5-pentamethylene or $-CH = CH\text{-}N = CH-$ group in such a way that $R_3$ and $R_4$ taken with the nitrogen atom to which they are attached can represent in particular a pyrrolidinyl, piperidinyl, morpholinyl, piperazinyl, 4-methylpiperazinyl, 4-phenylpiperazinyl or 1H-imidazolyl radical, as well as the pharmaceutically acceptable salts of these derivatives, characterized in that a 1-(4-alkoxybenzenesulphonyl)indolizine derivative of general formula

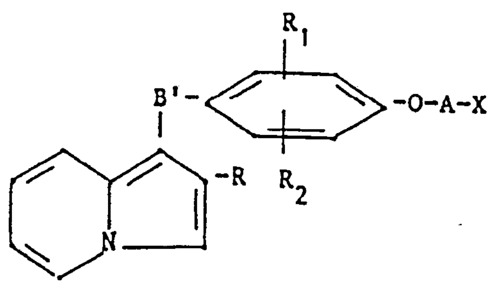

II

in which B′ represents an –S– or $-SO_2-$ group, R, $R_1$ and $R_2$ have the same meaning as before, A has the same meaning as before and X represents a halogen atom or an alkylsulphonyloxy group having from 1 to 4 carbon atoms or arylsulphonyloxy group having from 6 to 10 carbon atoms is condensed, in presence of an acid acceptor in a polar or nonpolar solvent and at a temperature of between ambient temperature and the reflux temperature, with an amine of general formula :

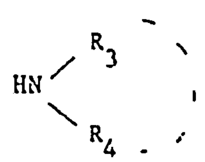

III

in which $R_3$ and $R_4$ have the same meaning as before to form the desired indolizine derivative, which is reacted if desired with an organic or inorganic acid in order to form a pharmaceutically acceptable salt of this derivative.

2. Process for preparation of indolizine derivatives corresponding to the general formula :

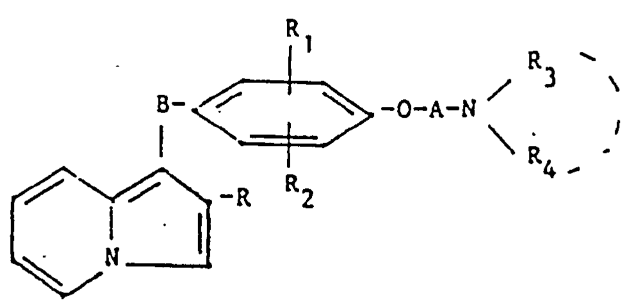

I

in which

A represents an alkylene radical having from 2 to 5 carbon atoms,

B represents an –S– or $-SO_2-$ group,

R represents hydrogen, a linear or branched alkyl radical having from 1 to 8 carbon atoms, a cycloalkyl radical comprising a maximum of 6 carbon atoms or a phenyl group which is non-substituted or substituted by one or more identical or different substituents, selected from halogen atoms and from lower alkyl groups having from 1 to 4 carbon atoms, lower alkoxy groups having from 1 to 4 carbon atoms or nitro groups,

$R_1$ and $R_2$, which are identical or different, each represents hydrogen, the methyl or ethyl radical or a halogen,

$R_3$ represents a linear or branched alkyl radical having from 1 to 8 carbon atoms or a radical of formula :

$-Alk-R_5$

in which Alk represents a simple bond or a linear or ranched alkylene radical having from 1 to 5 carbon atoms and $R_5$ represents a pyridyl, phenyl, 2,3-methylenedioxyphenyl or 3,4-methylenedioxyphenyl radical or a phenyl group substituted by one or more identical or different substituents selected from halogen atoms, lower alkyl groups having from 1 to 4 carbon atoms or lower alkoxy groups having from 1 to 4 carbon atoms,

$R_4$ represents hydrogen or an alkyl radical having from 1 to 8 carbon atoms,

or $R_3$ and $R_4$, when taken together, represent a 1,4-tetramethylene, 1,5-pentamethylene, 3-oxo-1,5-pentamethylene, 3-aza-1,5-pentamethylene, 3-methylaza-1,5-pentamethylene or 3-phenylaza-1,5-pentamethylene or $-CH=CH-N=CH-$ group in such a way that $R_3$ and $R_4$ taken with the nitrogen atom to which they are attached can represent in particular.a pyrrolidinyl, piperidinyl, morpholinyl, piperazinyl, 4-methylpiperazinyl, 4-phenylpiperazinyl or 1H-imidazolyl radical, as well as the pharmaceutically acceptable salts of these derivatives, characterized in that a 1-(4-hydroxybenzenesulphonyl)indolizine of general formula

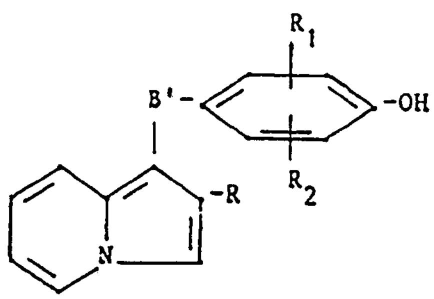

IV

in which

B′ represents an $-S-$ or $-SO_2-$ group and

R, $R_1$ and $R_2$ have the same meaning as before, is reacted in presence of a base with a compound of general formula :

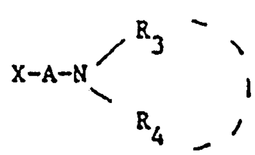

in which

X represents a halogen atom or an alkylsulphonyl group having from 1 to 4 carbon atoms or an arylsulphonyl group having from 6 to 10 carbon atoms,

A represents an alkylene radical having from 2 to 5 carbon atoms and

$R_3$ and $R_4$ have the same value as before, the reaction taking place under reflux and in an appropriate solvent to obtain the desired indolizine derivative which can, if desired, be reacted with an appropriate organic or inorganic acid to form a pharmaceutically acceptable salt.

3. Process for preparation of indolizine derivatives corresponding to the general formula :

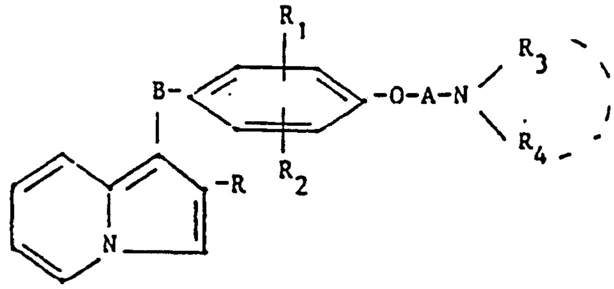

I

in which

A represents the 2-hydroxypropylene radical,

B represents an –S– or –$SO_2$– group,

R represents hydrogen, ‖ a linear or branched alkyl radical having from 1 to 8 carbon atoms, a cycloalkyl radical having a maximum of 6 carbon atoms or a phenyl group which is non-substituted or substituted by one or more identical or different substituents, selected from halogen atoms and from lower alkyl groups having from 1 to 4 carbon atoms, lower alkoxy groups having from 1 to 4 carbon atoms or nitro groups,

$R_1$ and $R_2$, which are identical or different, each represents hydrogen, the methyl or ethyl radical or a halogen,

$R_3$ represents a linear or branched alkyl radical having from 1 to 8 carbon atoms or a radical of formula :

$$-Alk-R_5$$

in which Alk represents a simple bond or a linear or branched alkylene radical having from 1 to 5 carbon atoms and $R_5$ represents a pyridyl, phenyl, 2,3-methylenedioxyphenyl or 3,4-methylenedioxyphenyl radical or a phenyl group substituted by one or more identical or different substituents selected from halogen atoms, lower alkyl groups having from 1 to 4 carbon atoms or lower alkoxy groups having from 1 to 4 carbon atoms,

$R_4$ represents hydrogen or an alkyl radical having from 1 to 8 carbon atoms,

or $R_3$ and $R_4$, when taken together, represent a 1,4-tetramethylene, 1,5-pentamethylene, 3-oxo-1,5-pentamethylene, 3-aza-1,5-pentamethylene, 3-methylaza-1,5-pentamethylene, 3-phenylaza-1,5-pentamethylene or –CH = CH-N = CH– group in such a way that $R_3$ and $R_4$ taken with the nitrogen atom to which they are attached can represent in particular a pyrrolidinyl, piperidinyl, morpholinyl, piperazinyl, 4-methylpiperazinyl, 4-phenylpiperazinyl or 1H-imidazolyl radical as well as the pharmaceutically acceptable salts of these derivatives, characterized in that an oxiranylmethoxy derivative of general formula :

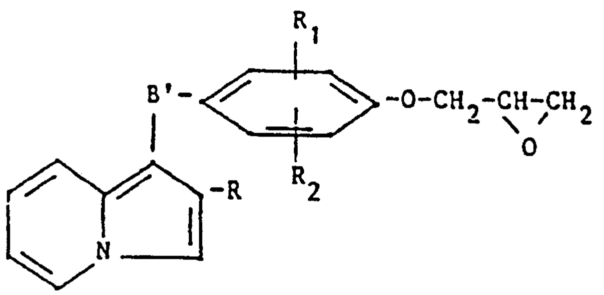

XIV

in which B′ represents an –S– or –$SO_2$– group and R, $R_1$ and $R_2$ have the same meaning as before is treated under reflux with an amine of general formula :

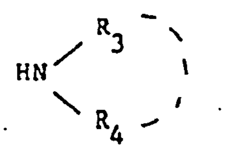

III

in which $R_3$ and $R_4$ have the same meaning as before and that, in a polar solvent or in an excess of the said amine to give the desired indolizine derivative, reaction is carried out if desired with an organic or inorganic acid to form a pharmaceutically acceptable salt of this derivative.

4. Process according to any one of the preceding claims, characterized in that indoline derivatives according to the general formula :

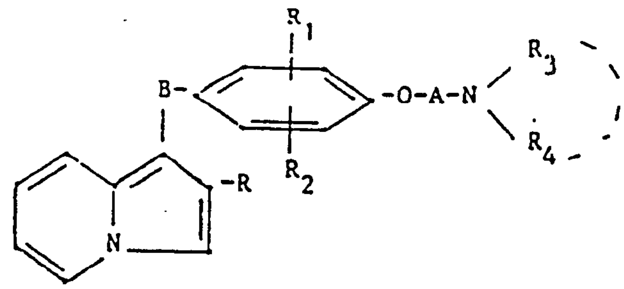

in which :

B represents an –S– or $-SO_2-$ group,

R represents hydrogen or one of the radicals methyl, ethyl, n-propyl, isopropyl, n-butyl, isobutyl, t-butyl, 1-methylpropyl, n-pentyl, neopentyl, phenyl, monofluoro-, monochloro- or monobromophenyl, difluoro-, dichloro- or dibromophenyl, monomethyl-or dimethylphenyl, monomethoxy- or dimethoxyphenyl, nitrophenyl or methylphenyl substituted by a halogen atom,

$R_1$ and $R_2$, which are identical or different, each represents hydrogen, chlorine, bromine or iodine or the methyl radical,

A represents a 1,2-ethylene, 1,3-propylene, 2-methyl-1,3-propylene, 1,4-tetramethylene, 1,5-pentamethylene or 2-hydroxypropylene radical,

$R_3$ represents a methyl, ethyl, n-propyl, isopropyl, n-butyl, isobutyl, t-butyl, 1-methylpropyl, n-pentyl, n-hetyl, n-heptyl, n-octyl, phenyl, benzyl, phenethyl, methoxyphenethyl, dimethoxyphenethyl, dimethylphenethyl, dimethoxybenzyl or pyridylethyl radical or a phenethyl radical substituted in its aromatic part by methyl and methoxy radicals,

$R_4$ represents hydrogen or a methyl, ethyl, n-propyl, isopropyl, n-butyl, isobutyl, t-butyl, n-pentyl, neopentyl, n-hexyl, n-heptyl or n-octyl radical,

$R_3$ and $R_4$ taken together represent a 1,4-tetramethylene, 1,5-pentamethylene, 3-oxa-1,5-pentamethylene, 3-aza-1,5-pentamethylene, 3-methylaza-1,5-pentamethylene, 3-phenylaza-1,5-pentamethylene or –CH = CH–N = CH–radical,

as well as the pharmaceutically acceptable salts of these derivatives are prepared.

5. Process according to any one of Claims 1 to 4, characterized in that indolizine derivatives are prepared in which B represents an $-SO_2-$ group.

6. Process according to any one of Claims 1 to 4, characterized in that indolizine derivatives are prepared in which $R_3$ represents a 3,4-dimethoxyphenyl, 3,4-dimethoxybenzyl or 3,4-dimethoxyphenethyl radical.

7. Process according to Claim 1 or 2, characterized in that 2-isopropyl-1-[{3-[N-methyl-N-(3,4-dimethoxy-β-phenethyl)amino]-propyl}-4-oxybenzenesulphonyl]indolizine and its pharmaceutically acceptable salts are prepared.

8. Process according to Claim 1 or 2, characterized in that 2-isopropyl-1-[(3-di-n-butylaminopropyl)-4-oxybenzenesulphonyl]indolizine and its pharmaceutically acceptable salts are prepared.

9. Process according to Claim 1 or 2, characterized in that 2-ethyl-1-[{3-[N-methyl-N-(3,4-dimethoxy-β-phenethyl)amino]propyl}-4-oxybenzenesulphonyl]indoziline and its pharmaceutically acceptable salts are prepared.

10. Process according to Claim 1 or 2, characterized in that 2-ethyl-1-[(3-di-n-butylaminopropyl)-4-oxybenzenesulphonyl]indolizine and its pharmaceutically acceptable salts are prepared.

11. Process according to any one of the preceding claims, characterized in that indolizine derivatives are prepared in which the pharmaceutically acceptable salt is the hydrochloride.

12. Process for preparation of pharmaceutical or veterinary compositions, characterized in that at least one indolizine derivative prepared according to one of the preceding claims is combined with a pharmaceutical vehicle or appropriate excipient.

13. Process for preparation of pharmaceutical or veterinary compositions according to Claim 12 for the treatment of pathological syndromes of the cardiovascular system containing from 50 mg to 500 mg of active principle.

14. Process for preparation of indolizine derivatives of general formula

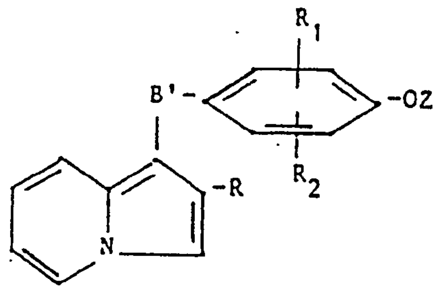

XV

in which

B′ represents an –S– or –$SO_2$– group,

R, $R_1$ and $R_2$ are as defined in Claim 1,

Z represents hydrogen, an oxiranylmethyl radical, an alkylsulphonyl radical having from 1 to 4 carbon atoms, an arylsulphonyl radical having from 6 to 10 carbon atoms or a radical of the formula :

$$-A-Z_1$$

in which A is as defined in Claim 1 and $Z_1$ represents a halogen atom, the hydroxy radical, an alkylsulphonyloxy radical having from 1 to 4 carbon atoms or an arylsulphonyloxy radical having from 6 to 10 carbon atoms, characterized in that :

(a) a picolyl sulphone or a picolyl sulphide of general formula :

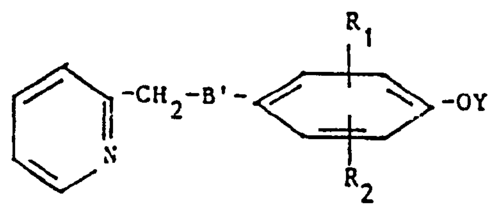

in which B′, $R_1$ and $R_2$ have the same meaning as before and Y represents an alkylsulphonyl radical having from 1 to 4 carbon atoms or an arylsulphonyl radical having from 6 to 10 carbon atoms, is cyclized under reflux in a solvent and in presence of a base with an a-halogenoketone of general formula :

$$R-\overset{\overset{\displaystyle O}{\|}}{C}-CH_2-Hal$$

in which R has the same meaning as before and Hal represents a halogen atom, in order to obtain the compounds of formula XV in which Z represents an alkylsulphonyl radical having from 1 to 4 carbon atoms or arylsulphonyl radical having from 6 to 10 carbon atoms,

(b) a picolyl sulphide of general formula :

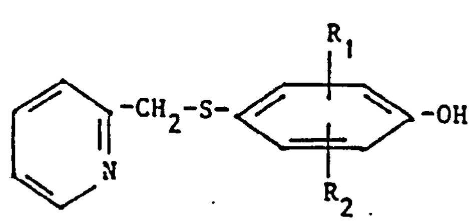

in which $R_1$ and $R_2$ have the same meaning as before is reacted with an a-halogenoketone of general formula :

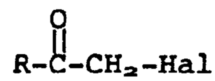

in which R and Hal have the same meaning as before, in order to obtain a pyridinium salt which is cyclized under reflux in a solvent and in presence of a base in order to obtain the compounds of formula XV in which Z represents hydrogen and B′ represents an –S– group,

(c) an indoziline derivative of formula XV, in which Z represents an alkylsulphonyl radical having from 1 to 4 carbon atoms or arylsulphonyl radical having from 6 to 10 carbon atoms, is hydrolyzed under reflux and in basic medium in order to obtain the compounds of formula XV in which Z represents hydrogen,

(d) an indolizine derivative of formula XV in which Z represents hydrogen, is condensed under reflux in a solvent and in presence of a base with a dihalogenoalkane of general formula :

Hal-A-Hal

in which A has the same meaning as before and Hal has the same meaning as before, in order to obtain the compounds of formula XV in which Z represents a radical of formula $-A-Z_1$ in which $Z_1$ represents a halogen atom,

e) an indolizine derivative of formula XV in which Z represents hydrogen is condensed in a solvent and in presence of a base with a halogenated alcohol of general formula :

Hal-A-OH

in which A and Hal have the same meaning as before to obtain the compounds of formula XV in which Z represents a radical of formula $-A-Z_1$ in which $Z_1$ represents the hydroxy group,

f) an indolizine derivative of formula XV in which Z represents a radical of formula $-A-Z_1$ in which $Z_1$ represents the hydroxy group is condensed in a solvent and in the presence of an acid acceptor with a halide of general formula

Hal-Y

in which Hal and Y have the same meaning as before in order to obtain an indolizine derivative of formula XV in which Z represents a radical of formula $-A-Z_1$ in which $Z_1$ represents an alkylsulphonyloxy group having from 1 to 4 carbon atoms or arylsulphonyloxy group having from 6 to 10 carbon atoms,

g) an indolizine derivative of formula XV in which Z represents hydrogen is reacted under reflux, in a polar solvent and in presence of a base, with an epihalohydrin, in order to obtain the compounds of formula XV in which Z represents an oxiranylmethyl radical.

15. Process according to one of Claims 2 or 16, characterized in that the base is an alkali metal carbonate, an alkali metal hydroxide, an alkali metal hydride or an alkali metal alcoholate.

16. Process according to Claim 14, characterized in that indolizine derivatives are prepared in which Z represents a methanesulphonyloxy, benzenesulphonyloxy or p-toluenesulphonyloxy radical and $Z_1$ represents a bromine atom or a methanesulphonyloxy, benzenesulphonyloxy or p-toluenesulphonyloxy radical.

17. Process according to Claim 14, characterized in that 2-ethyl-1-[(3-bromopropyl)-4-oxybenzenesulphonyl]indolizine is prepared.

18. Process according to Claim 14, characterized in that 2-isopropyl-1-[(3-bromopropyl)-4-oxybenzenesulphonyl]indolizine is prepared.

19. Process according to Claim 14, characterized in that 2-ethyl-1-(4-tosyloxybenzenesulphonyl)indolizine is prepared.

20. Process according to Claim 14, characterized in that 2-isopropyl-1-(4-tosyloxybenzenesulphonyl)indolizine is prepared.

21. Process according to Claim 14, characterized in that 2-ethyl-1-(4-hydroxybenzenesulphonyl)indolizine is prepared.

22. Process according to Claim 14, characterized in that 2-isopropyl-1-(4-hydroxybenzenesulphonyl)indolizine is prepared.